# EUROPEAN PATENT SPECIFICATION

(11) **EP 2 467 156 B1**
(45) Date of publication and mention of the grant of the patent: **01.11.2017**
(21) Application number: 10810448.0
(22) Date of filing: 16.08.2010
(51) Int. Cl.: A61K 39/00, C12P 21/08, C07K 16/22, C07K 16/28, C07K 16/30

(54) **COMBINATION THERAPY OF CANCER WITH ANTI-ENDOGLIN ANTIBODIES AND ANTI-VEGF AGENTS**
KOMBINATIONS-KREBSTHERAPIE MIT ANTI-ENDOGLIN-ANTIKÖRPERN UND ANTI-VEGF-MITTELN
POLYTHÉRAPIE DU CANCER AU MOYEN D ANTICORPS ANTI-ENDOGLINE ET D AGENTS ANTI-VEGF

(30) Priority: 17.08.2009 US 234574 P
(43) Date of publication of application: 27.06.2012
(60) Divisional application: 14151471.1
(73) Proprietor: Tracon Pharmaceuticals, Inc., San Diego, CA 92122 (US); Health Research, Inc., Buffalo, NY 14263 (US)
(72) Inventor: THEUER, Charles, P., Carlsbad, CA 92009 (US); SEON, Ben K., Williamsville, NY 14221 (US)
(74) Representative: HGF Limited
(86) International application number: PCT/US2010/045651
(87) International publication number: WO 2011/022339

(56) References cited:
- US-A- 928 641
- US-A1- 2003 129 193
- US-A1- 2004 023 313
- US-A1- 2006 292 643
- US-A1- 2007 072 797
- US-A1- 2009 142 343
- Rosen, L. S.: "EARLY EVIDENCE OF TOLERABILITY AND CLINICAL ACTIVITY FROM A PHASE 1 STUDY OFTRC105 (ANT-CD105 ANTIBODY) IN PATIENTS WITH ADVANCED REFRACTORY CANCER", 2009 ASCO Annual Meeting , 2 June 2009 (2009-06-02), XP002692012, Retrieved from the Internet: URL:http://www.traconpharma.com/pdfs/105ST 101_TRC105_EORTC_NCI_AACR_Poster.pdf [retrieved on 2013-02-11]
- Tracon Pharmaceuticals: "TRACON Pharmaceuticals Announces Dosing of Initial Three Cancer Patients in a Phase 1 Clinical Trial with TRC105, a Human Chimeric AntibodyTRACON Pharmaceuticals Announces Dosing of Initial Three Cancer Patients in a Phase 1 Clinical Trial with TRC105, a Human Chimeric Antibody", Company News , 8 January 2008 (2008-01-08), XP002692013, Retrieved from the Internet: URL:http://www.traconpharma.com/content/pr _01_9_08.html [retrieved on 2013-02-12]
- SHIMA UNEDA ET AL: 'Anti-endoglin monoclonal antibodies are effective for suppressing metastasis and the primary tumors by targeting tumor vasculature' INTERNATIONAL JOURNAL OF CANCER vol. 125, no. 6, 15 September 2009, pages 1446 - 1453, XP055208365 DOI: 10.1002/ijc.24482 ISSN: 0020-7136

## Description

### BACKGROUND OF THE INVENTION

Cancer is the second leading cause of human death next to coronary disease. Worldwide, millions of people die from cancer every year. In the United States alone, cancer causes the death of well over a half-million people each year, with some 1.4 million new cases diagnosed per year. While deaths from heart disease have been declining significantly, those resulting from cancer generally are on the rise. In the early part of the next century, cancer is predicted to become the leading cause of death.

Moreover, even for those cancer patients that initially survive their primary cancers, common experience has shown that their lives are dramatically altered. Many cancer patients experience strong anxieties driven by the awareness of the potential for recurrence or treatment failure. Many cancer patients experience significant physical debilitations following treatment.

Generally speaking, the fundamental problem in the management of the deadliest cancers is the lack of effective and non-toxic systemic therapies. Cancer is a complex disease characterized by genetic mutations that lead to uncontrolled cell growth. Cancerous cells are present in all organisms and under normal circumstances their excessive growth is tightly regulated by various physiological factors.

Angiogenesis is the physiological process by which new blood vessels develop from pre-existing vessels. Angiogenesis has been suggested to play a role in both normal and pathological processes. For example, angiogenic processes are involved in the development of the vascular systems of animal organs and tissues.

In certain pathological conditions, angiogenesis is stimulated as a means to provide adequate blood and nutrient supply to the cells within affected tissue. Many of these pathological conditions involve aberrant cell proliferation and/or regulation. Solid cancers and exudative macular degeneration depend upon the recruitment of a new blood supply for continued growth as well as metastasis.

### SUMMARY OF THE INVENTION

The present invention provides a method of inhibiting VEGF induced sprouting by contacting cells in vitro with a composition comprising a chimeric anti-endoglin antibody and a composition comprising an antagonistic anti-VEGF antibody; said chimeric anti-endoglin antibody comprising a light chain variable region (VL) having an amino acid sequence set forth as SEQ ID NO: 1; a light chain constant region (CL) having an amino acid sequence set forth as SEQ ID NO: 2; a heavy chain variable region (VH) having an amino acid sequence set forth as SEQ ID NO: 3; and a constant region (Fc) having an amino acid sequence set forth as SEQ ID NO: 4.

The present invention also provides a composition comprising a chimeric anti-endoglin antibody and a composition comprising an antagonistic anti-VEGF antibody for use in treating an angiogenesis-related disease in a subject; said chimeric anti-endoglin antibody comprising a light chain variable region (VL) having an amino acid sequence set forth as SEQ ID NO: 1; a light chain constant region (CL) having an amino acid sequence set forth as SEQ ID NO: 2; a heavy chain variable region (VH) having an amino acid sequence set forth as SEQ ID NO: 3; and a constant region (Fc) having an amino acid sequence set forth as SEQ ID NO: 4; wherein the treatment in said angiogenesis-related disease is characterized by the inhibition of VEGF-induced angiogenesis.

The composition of the invention may further comprise an acceptable carrier or excipient.

The angiogenesis-related disease may be a cancer, or a metastasis thereof. Thus, the composition may inhibit cancer growth. The cancer may be a solid tumor, preferably, the cancer is an epithelial based tumor. The cancer may be selected from a lung cancer, a gynecologic malignancy, a melanoma, a breast cancer, a pancreatic cancer, an ovarian cancer, a uterine cancer, a colorectal cancer, a prostate cancer, a head cancer, a liver cancer (hepatocellular cancer), a neck cancer, a kidney cancer (renal cell cancer), a sarcoma, a myeloma, and a lymphoma.

Alternatively, the angiogenesis-related disease may be an ocular disease characterized by angiogenesis/neovascularization. The ocular disease may be macular degeneration or diabetic retinopathy. The macular degeneration may be age-related macular degeneration (AMD).

The chimeric anti-endoglin antibody may be present in the composition of the invention in an amount of 0.01 mg/kg, 0.05 mg/kg, 0.1 mg/kg, 0.5 mg/kg, 1 mg/kg, 5 mg/kg, 10 mg/kg, 20 mg/kg, 30 mg/kg. The anti-VEGF antibody may be present in the composition of the invention in an amount of 2.5 mg/kg, 5 mg/kg, 7.5 mg/kg, 10 mg/kg or 15 mg/kg.

The chimeric anti-endoglin antibody and the anti-VEGF antibody may be administered sequentially or concurrently. The chimeric anti-endoglin antibody and the anti-VEGF antibody may be administered at the same site, or they may be administered at different sites.

The anti-VEGF antibody may be bevacizumab.

The anti-VEGF antibody may be ranibizumab.

The compositions described herein can also shrink blood vessels, inhibit endothelial cell proliferation associated with disease, and/or prevent leakage of blood vessels.

The compositions described herein can be used to treat or prevent macular degeneration, CNV, diabetic retinopathy, or proliferative vitreoretinopathy. The compositions described herein can also shrink blood vessels, inhibit endothelial cell proliferation associated with ocular disease, clear symptoms of bleeding, treat cloudy vision, provide stasis of vision loss, and/or prevent leakage of blood vessels.

Provided herein is a chimeric anti-endoglin antibody having a heavy chain variable region (V_{H}) having an amino acid sequence set forth as SEQ ID NO: 3; a heavy chain constant region (Fc) having an amino acid sequence set forth as SEQ ID NO: 4; a light chain variable region (V_{L}) having an amino acid sequence set forth as SEQ ID NO: 1; and a light chain constant region (C_{L}) having an amino acid sequence set forth as SEQ ID NO: 2.

The antibodies described herein can be modified to alter a pharmacokinetic property of the compound such as, for example, in vivo stability, solubility, bioavailability or half-life. Such modifications include, but are not limited to, PEGylation and/or glycosylation.

The antibodies described herein can be formulated for rapid or extended delivery using conventional means. Rapid delivery is, for example, by intravenous injection. Extended delivery is, for example, by subcutaneous deposition.

Described herein are compositions of antibodies and antigen-binding fragments described herein and an acceptable carrier or excipient.

Antibodies and antigen-binding fragments thereof as described herein can be used to treat various diseases and conditions associated with angiogenesis, e.g., various forms of cancer, solid tumors, and metastases. Additionally, these antibodies and antigen-binding fragments thereof described herein can be used in the formulation of a medicament for the prophylaxis, treatment, or diagnosis of diseases and conditions associated with various forms of cancer, solid tumors, and metastases.

Antibodies and antigen-binding fragments thereof as described herein can be used to treat various forms of ocular or non-ocular diseases characterized by angiogenesis/neovascularization (e.g., macular degeneration, diabetic retinopathy). Additionally, these antibodies and antigen-binding fragments thereof described herein can be used in the formulation of a medicament for the prophylaxis, treatment, or diagnosis of diseases and conditions associated with angiogenesis, e.g., various forms of ocular or non-ocular diseases characterized by angiogenesis/neovascularization (e.g., macular degeneration, diabetic retinopathy).

Described herein is a method for inducing a host immune response in a patient, by administering to the patient a first composition contains a chimeric anti-endoglin antibody and a second composition contains an anti-VEGF antibody or antigen-binding fragment thereof, whereby an effective host immune response is induced.

Described herein is a method for inducing a host immune response in a patient, by administering to the patient a composition containing a chimeric anti-endoglin antibody and an anti-VEGF antibody or antigen-binding fragment thereof, whereby an effective host immune response is induced.

The host immune response can be a humoral immune response or a cell-mediated immune response. The immune response can be a response that inhibits angiogenesis, an angiogenesis-dependent disease or an angiogenesis-dependent disorder. Immune responses also include induction or blockage of cell signaling pathways (e.g. Smad signaling).

Described herein is a method of affecting cell signaling pathways associated with angiogenesis. Angiogenic cells can be contacted (*in vitro, in vivo* or *ex vivo*) with one or more compositions described herein in an amount sufficient to alter cell signaling pathwaysIn one example, in response to chimeric anti-endoglin antibody binding, Smad 1, 5 and/or 8 signaling is inhibited by about 1.5 fold or more in angiogenic cells. In another example, Smad 3 levels increase by about 1.5 fold or more, indicating that cells are returning to a quiescent state. A composition containing an anti-VEGF antibody or antigen-binding fragment thereof is also administered in connection with a composition containing a chimeric anti-endoglin antibody.

In one embodiment, inhibiting angiogenesis or an angiogenesis-dependent disease or disorder alleviates symptoms associated with the disease or disorder. In another embodiment, inhibiting angiogenesis or an angiogenesis-dependent disease or disorder results in decreased tumor size, prevention of tumor progression, decreased cell proliferation, increased apoptosis, or increased survival of a subject.

Administration of the compositions of the invention can prolong life of the subject being treated. A cancer/tumor to be treated includes a solid tumor; a tumor can be a primary tumor or a metastatic tumor. Exemplary solid tumors are of a tissue or organ selected from among skin, melanoma, lung, pancreas, breast, ovary, colon, rectum, stomach, thyroid, laryngeal, ovarian, prostate, colorectal, head, neck, eye, mouth, throat, esophagus, chest, bone, testicular, lymphoid, marrow, bone, sarcoma, renal, sweat gland, liver, kidney, brain, e.g. glioblastoma multiforme and the like tissues. In one non-limiting example a solid tumor is a colon tumor, a breast tumor, a kidney tumor, a lung tumor, a prostate tumor, an ovarian tumor, or metastasis of any of such tumors.

An ocular tissue to be treated is, for example, a retinal tissue of a patient with diabetic retinopathy, macular degeneration or neovascular glaucoma and the angiogenesis to be inhibited is retinal tissue angiogenesis where there is neovascularization of retinal tissue.

The described treatments can further include surgical removal of a tumor and/or administration of one or more anti-cancer agents. An additional anti-cancer agent can be administered prior to, concomitant with, or subsequent to, administration of compositions described herein. An additional anti-cancer agent can be administered within a week before the compositions, within a week after the compositions, or the additional anti-cancer agent can be administered on the same day as the compositions. If an additional anti-cancer agent is administered on the same day as the compositions, administration can be concomitant.

Described herein are methods of inhibiting angiogenesis by contacting a cell or tissue with a therapeutically effective amount of one or more compositions as described herein sufficient to inhibit angiogenesis. Described herein are methods of inhibiting growth of tumor cells by contacting tumor cells with or administering to a subject a therapeutically effective amount of one or more compositions as described herein sufficient to inhibit growth of tumor cells.

Desceibed herein is a method, comprising contacting a tissue with one or more compositions as described herein, wherein contacting inhibits angiogenesis, cell growth, proliferation, cell division, etc. The tissue can be a cultured tissue biopsy sample or can be present in a subject.

Desceibed herein is a method of preventing or treating a cell proliferative disorder by administering to a subject having or at risk of having a cell proliferative disorder an effective amount of one or more compositions provided herein effective to treat the cell proliferative disorder. The cell proliferative disorder can be, for example a benign or malignant solid or non-solid tumor and the tumor can be metastatic or non-metastatic. The treatment can result in improving the subject's condition and can be assessed by determining if one or more of the following factors has occurred: decreased cell proliferation, decreased numbers of cells, increased apoptosis, or decreased survival of at least a portion of the cells comprising the cell proliferative disorder. One or more of these occurrences may, in some cases, result in partial or total elimination of a tumor or metastases and prolongation of survival of the patient.

Provided herein are compositions for use in treating diabetic retinopathy, macular degeneration, choroidal neovascularization or neovascular glaucoma in a patient. The treatment can result in improving the subject's condition and can be assessed by determining if one or more of the following factors has occurred: decreased macular edema, decreased areas of CNV, or increased visual acuity.

In the uses provided herein, a subject to be treated can be a human or a non-human subject. Compositions and the anti-cancer agent or treatments provided herein can be administered once or multiple times depending on the health of the patient, the progression of the disease or condition, and the efficacy of the treatment. Adjustments to therapy and treatments can be made throughout the course of treatment (e.g., the dosage of an antibody in a composition).

Compositions can be administered locally, regionally or systemically, such as, for example, administration by subcutaneous, subcutaneous, intravitreal, intradermal, intravenous, intra-arterial, intraperitoneal or intramuscular injection.

Additionally, chimeric anti-endoglin antibodies can also be used in combination with other known therapies and/or anti-VEGF antibodies that inhibit the VEGF pathway. Examples of such compounds include, but are not limited to, ranibizumab (LUCENTIS®), aflibercept (VEGF-Trap), sunitinib (SUTENT®), sorafenib (NEXAVAR®), axitinib, pegaptanib and pazopanib.

Additionally, chimeric anti-endoglin antibodies and anti-VEGF antibodies or antigen-binding fragments thereof described herein can also be used in combination with other known therapies and/or compounds for the treatment of a cancer. Examples of such compounds include, but are not limited to, ranibizumab (LUCENTIS®), aflibercept (VEGF-Trap), sunitinib (SUTENT®), sorafenib (NEXAVAR®), axitinib, pegaptanib and pazopanib. Examples of other therapies include, but are not limited to, irradiation, surgery or administration of one or more chemotherapeutic agents.

Provided herein is a method of monitoring the efficacy of one or more of any of the methods provided herein. Increased levels of soluble endoglin have been correlated with decreased survival in cancer patients. Thus, levels of soluble endoglin can be monitored prior to and during therapy. A decrease in the levels of soluble endoglin can, therefore, be one indication that a therapeutic regimen is effective in treating the patient.

One embodiment of the present application contemplates the use of any of the compositions described herein to formulate a medicament for treating a disorder described herein. Medicaments can be formulated based on the physical characteristics of the patient/subject needing treatment, and can be formulated in single or multiple formulations based on the stage of, for example, cancerous tissue. Medicaments can be packaged in a suitable package with appropriate labels for the distribution to hospitals and clinics wherein the label is for the indication of treating a disorder as described herein in a subject. Medicaments can be packaged as a single or multiple units. Instructions for the dosage and administration of the compositions described herein can be included with the packages.

### BRIEF DESCRIPTION OF THE DRAWINGS

**Figure 1** provides diagram of the TGF-β/ALK5 signaling pathway. The TGF-β/ALK5 pathway (A) leads to inhibition of cell proliferation. The TGF-β/ALK1 pathway (B) induces endothelial cell proliferation and requires CD105 (endoglin) for ALK1 signaling. The dotted lines indicate inactive or blocked pathways. The bolded arrow indicates stimulation of a signaling pathway.

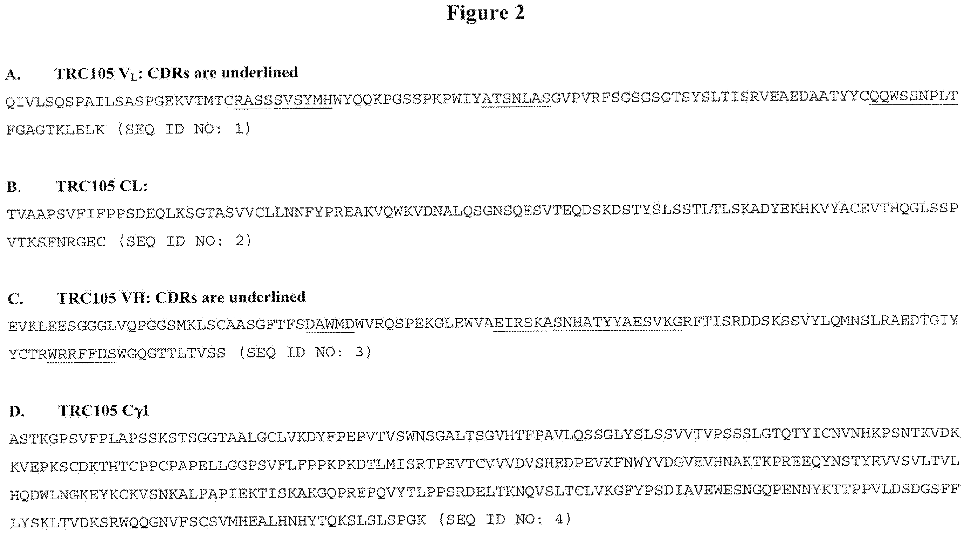

**Figure 2** provides amino acid sequences of a chimeric anti-endoglin antibody (TRC105) described herein. Figure 2A is a representative variable light chain of a chimeric anti-endoglin antibody (SEQ ID NO: 1); Figure 2B is a representative constant light chain of a chimeric anti-endoglin antibody (SEQ ID NO: 2); Figure 2C is a representative variable heavy chain of a chimeric anti-endoglin antibody (SEQ ID NO: 3); and Figure 2D is a representative constant gamma1 heavy chain of a chimeric anti-endoglin antibody (SEQ ID NO: 4).
**Figure 3** illustrates that using HUVEC spheroid seeded in collagen, TRC105 inhibits VEGF induced sprouting in a dose dependent manner (N=3).
**Figure 4** illustrates that while TRC105 blocks VEGF induced sprouting (diagonal hatching), it does not inhibit bFGF induced sprouting (diamond hatching) of HUVEC spheroids (N=2).
**Figure 5** illustrates the inhibitory effect of TRC105 on VEGF induced sprouting (diagonal hatching), is enhanced when combined with the VEGF inhibitor Bevacizumab (diamond hatching), such that HUVEC sprouting is completely inhibited.
**Figure 6** illustrates that the inhibitory effect of TRC105 on VEGF induced sprouting (diagonal hatching), is not enhanced when combined with an inhibitor of the VEGF receptor kinase (diamond hatching).

### DETAILED DESCRIPTION OF THE INVENTION

It is to be understood that this application is not limited to particular formulations or process parameters, as these may, of course, vary. It is also to be understood that the terminology used herein is for the purpose of describing particular embodiments only, and is not intended to be limiting. Further, it is understood that a number of methods and materials similar or equivalent to those described herein can be used in the practice of the present inventions.

In accordance with the present application, there may be employed conventional molecular biology, microbiology, and recombinant DNA techniques as explained fully in the art.

Chimeric anti-endoglin antibodies can be used in combination with anti-VEGF agents to treat or prevent various forms of cancer, solid tumors, and metastases and the like. Described herein are methods of treating or various forms of cancer, solid tumors, and metastases and the like via the administration of the compositions described herein. The compositions described herein can also shrink blood vessels, inhibit endothelial cell proliferation associated with disease, and/or prevent leakage of blood vessels.

### ANTIBODY TERMINOLOGY

As used herein, the term "antibody" refers to an immunoglobulin (Ig) whether natural or partly or wholly synthetically produced. The term also covers any polypeptide or protein having a binding domain which is, or is homologous to, an antigen-binding domain. The term further includes "antigen-binding fragments" and other interchangeable terms for similar binding fragments such as described below. Chimeric antibodies are also contemplated by this term.

Native antibodies and native immunoglobulins are usually heterotetrameric glycoproteins of about 150,000 Daltons, composed of two identical light (L) chains and two identical heavy (H) chains. Each light chain is typically linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ("V_{H}" or "VH") followed by a number of constant domains ("C_{H}" or "CH"). Each light chain has a variable domain at one end ("V_{L}" or "VL") and a constant domain ("C_{L}" or "CL") at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The terms "synthetic polynucleotide," "synthetic gene" or "synthetic polypeptide," as used herein, mean that the corresponding polynucleotide sequence or portion thereof, or amino acid sequence or portion thereof, is derived, from a sequence that has been designed, or synthesized *de novo,* or modified, compared to an equivalent naturally-occurring sequence. Synthetic polynucleotides (antibodies or antigen binding fragments) or synthetic genes can be prepared by methods known in the art, including but not limited to, the chemical synthesis of nucleic acid or amino acid sequences. Synthetic genes are typically different from naturally-occurring genes, either at the amino acid, or polynucleotide level, (or both) and are typically located within the context of synthetic expression control sequences. Synthetic gene polynucleotide sequences, may not necessarily encode proteins with different amino acids, compared to the natural gene; for example, they can also encompass synthetic polynucleotide sequences that incorporate different codons but which encode the same amino acid (i.e., the nucleotide changes represent silent mutations at the amino acid level).

With respect to antibodies, the term "variable domain" refers to the variable domains of antibodies that are used in the binding and specificity of each particular antibody for its particular antigen. However, the variability is not evenly distributed throughout the variable domains of antibodies. Rather, it is concentrated in three segments called hypervariable regions (also known as CDRs) in both the light chain and the heavy chain variable domains. More highly conserved portions of variable domains are called the "framework regions" or "FRs." The variable domains of unmodified heavy and light chains each contain four FRs (FR1, FR2, FR3 and FR4), largely adopting a β-sheet configuration interspersed with three CDRs which form loops connecting and, in some cases, part of the β-sheet structure. The CDRs in each chain are held together in close proximity by the FRs and, with the CDRs from the other chain, contribute to the formation of the antigen-binding site of antibodies (see Kabat et al., Sequences of Proteins of Immunological Interest, 5th Ed. Public Health Service, National Institutes of Health, Bethesda, Md. (1991), pages 647-669).

The terms "hypervariable region" and "CDR" when used herein, refer to the amino acid residues of an antibody which are responsible for antigen-binding. The CDRs comprise amino acid residues from three sequence regions which bind in a complementary manner to an antigen and are known as CDR1, CDR2, and CDR3 for each of the V_{H} and V_{L} chains. In the light chain variable domain, the CDRs typically correspond to approximately residues 24-34 (CDRL1), 50-56 (CDRL2) and 89-97 (CDRL3), and in the heavy chain variable domain the CDRs typically correspond to approximately residues 31-35 (CDRH1), 50-65 (CDRH2) and 95-102 (CDRH3) according to Kabat et al., Sequences of Proteins of Immunological Interest, 5th Ed. Public Health Service, National Institutes of Health, Bethesda, Md. (1991)). It is understood that the CDRs of different antibodies may contain insertions, thus the amino acid numbering may differ. The Kabat numbering system accounts for such insertions with a numbering scheme that utilizes letters attached to specific residues (e.g., 27A, 27B, 27C, 27D, 27E, and 27F of CDRL1 in the light chain) to reflect any insertions in the numberings between different antibodies. Alternatively, in the light chain variable domain, the CDRs typically correspond to approximately residues 26-32 (CDRL1), 50-52 (CDRL2) and 91-96 (CDRL3), and in the heavy chain variable domain, the CDRs typically correspond to approximately residues 26-32 (CDRH1), 53-55 (CDRH2) and 96-101 (CDRH3) according to Chothia and Lesk, J. Mol. Biol., 196: 901-917 (1987)).

As used herein, "framework region" or "FR" refers to framework amino acid residues that form a part of the antigen binding pocket or groove. In some embodiments, the framework residues form a loop that is a part of the antigen binding pocket or groove and the amino acids residues in the loop may or may not contact the antigen. Framework regions generally comprise the regions between the CDRs. In the light chain variable domain, the FRs typically correspond to approximately residues 0-23 (FRL1), 35-49 (FRL2), 57-88 (FRL3), and 98-109 and in the heavy chain variable domain the FRs typically correspond to approximately residues 0-30 (FRH1), 36-49 (FRH2), 66-94 (FRH3), and 103-133 according to Kabat et al., Sequences of Proteins of Immunological Interest, 5th Ed. Public Health Service, National Institutes of Health, Bethesda, Md. (1991)). As discussed above with the Kabat numbering for the light chain, the heavy chain too accounts for insertions in a similar manner (e.g., 35A, 35B of CDRH1 in the heavy chain). Alternatively, in the light chain variable domain, the FRs typically correspond to approximately residues 0-25 (FRL1), 33-49 (FRL2) 53-90 (FRL3), and 97-109 (FRL4), and in the heavy chain variable domain, the FRs typically correspond to approximately residues 0-25 (FRH1), 33-52 (FRH2), 56-95 (FRH3), and 102-113 (FRH4) according to Chothia and Lesk, J. Mol. Biol., 196: 901-917 (1987)).

Constant domains (Fc) of antibodies are not involved directly in binding an antibody to an antigen but, rather, exhibit various effector functions, such as participation of the antibody in antibody-dependent cellular toxicity via interactions with, for example, Fc receptors (FcR). Fc domains can also increase bioavailability of an antibody in circulation following administration to a patient. Substitution of a murine Fc domain for a human Fc domain can also reduce side HAMA reactions.

Depending on the amino acid sequence of the constant domain of their heavy chains, immunoglobulins can be assigned to different classes. There are five major classes of immunoglobulins: IgA, IgD, IgE, IgG, and IgM, and several of these can be further divided into subclasses (isotypes), e.g., IgG1, IgG2, IgG3, IgG4, IgA1, and IgA2. The heavy-chain constant domains (Fc) that correspond to the different classes of immunoglobulins are called α, δ, ε, γ, and µ, respectively. The subunit structures and three-dimensional configurations of different classes of immunoglobulins are well known.

The "light chains" of antibodies from any vertebrate species can be assigned to one of two clearly distinct types, called kappa or ("κ") and lambda or ("λ"), based on the amino acid sequences of their constant domains.

The terms "antigen-binding portion of an antibody," "antigen-binding fragment," "antigen-binding domain," "antibody fragment" or a "functional fragment of an antibody" are used interchangeably herein to refer to one or more fragments of an antibody that retain the ability to specifically bind to an antigen. Non-limiting examples of antibody fragments included within such terms include, but are not limited to, (i) a Fab fragment, a monovalent fragment consisting of the V_{L}, V_{H}, C_{L} and C_{H1} domains; (ii) a F(ab')₂ fragment, a bivalent fragment containing two Fab fragments linked by a disulfide bridge at the hinge region; (iii) a Fd fragment consisting of the V_{H} and C_{H1} domains; (iv) a Fv fragment containing the V_{L} and V_{H} domains of a single arm of an antibody, (v) a dAb fragment (Ward et al., (1989) Nature 341:544 546), which containing a V_{H} domain; and (vi) an isolated CDR. Additionally included in this definition are "one-half" antibodies comprising a single heavy chain and a single light chain. Other forms of single chain antibodies, such as diabodies are also encompassed herein.

"F(ab')₂" and "Fab"' moieties can be produced by treating an Ig with a protease such as pepsin and papain, and include antibody fragments generated by digesting immunoglobulin near the disulfide bonds existing between the hinge regions in each of the two heavy chains. For example, papain cleaves IgG upstream of the disulfide bonds existing between the hinge regions in each of the two heavy chains to generate two homologous antibody fragments in which an light chain composed of V_{L} and C_{L} (light chain constant region), and a heavy chain fragment composed of V_{H} and C_{Hγ1} (γ1) region in the constant region of the heavy chain) are connected at their C terminal regions through a disulfide bond. Each of these two homologous antibody fragments is called Fab'. Pepsin also cleaves IgG downstream of the disulfide bonds existing between the hinge regions in each of the two heavy chains to generate an antibody fragment slightly larger than the fragment in which the two above-mentioned Fab' are connected at the hinge region. This antibody fragment is called F(ab')₂.

The Fab fragment also contains the constant domain of the light chain and the first constant domain (C_{H}1) of the heavy chain. Fab' fragments differ from Fab fragments by the addition of a few residues at the carboxyl terminus of the heavy chain C_{H}1 domain including one or more cysteine(s) from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

"Fv" refers to an antibody fragment which contains a complete antigen-recognition and antigen-binding site. This region consists of a dimer of one heavy chain and one light chain variable domain in tight, non-covalent or covalent association (disulfide linked Fv's have been described in the art, Reiter et al. (1996) Nature Biotechnology 14:1239-1245). It is in this configuration that the three CDRs of each variable domain interact to define an antigen-binding site on the surface of the V_{H}-V_{L} dimer. Collectively, a combination of one or more of the CDRs from each of the V_{H} and V_{L} chains confer antigen-binding specificity to the antibody. For example, it would be understood that, for example, the CDRH3 and CDRL3 could be sufficient to confer antigen-binding specificity to an antibody when transferred to V_{H} and V_{L} chains of a recipient antibody or antigen-binding fragment thereof and this combination of CDRs can be tested for binding, affinity, etc. using any of the techniques described herein. Even a single variable domain (or half of an Fv comprising only three CDRs specific for an antigen) has the ability to recognize and bind antigen, although likely at a lower affinity than when combined with a second variable domain. Furthermore, although the two domains of a Fv fragment (V_{L} and V_{H}), are coded for by separate genes, they can be joined using recombinant methods by a synthetic linker that enables them to be made as a single protein chain in which the V_{L} and V_{H} regions pair to form monovalent molecules (known as single chain Fv (scFv); Bird et al. (1988) Science 242:423-426; Huston et al. (1988) Proc. Natl. Acad. Sci. USA 85:5879-5883; and Osbourn et al. (1998) Nat. Biotechnol. 16:778). Such scFvs are also intended to be encompassed within the term "antigen-binding portion" of an antibody. Any V_{H} and V_{L} sequences of specific scFv can be linked to an Fc region cDNA or genomic sequences, in order to generate expression vectors encoding complete Ig (e.g., IgG) molecules or other isotypes. V_{H} and V_{L} can also be used in the generation of Fab, Fv or other fragments of Igs using either protein chemistry or recombinant DNA technology.

"Single-chain Fv" or "sFv" antibody fragments comprise the V_{H} and V_{L} domains of an antibody, wherein these domains are present in a single polypeptide chain. In some embodiments, the Fv polypeptide further comprises a polypeptide linker between the V_{H} and V_{L} domains which enables the sFv to form the desired structure for antigen binding. For a review of sFvs see, e.g., Pluckthun in The Pharmacology of Monoclonal Antibodies, Vol. 113, Rosenburg and Moore eds. Springer-Verlag, New York, pp. 269-315 (1994).

The term "AVIMER™" refers to a class of therapeutic proteins of human origin, which are unrelated to antibodies and antibody fragments, and are composed of several modular and reusable binding domains, referred to as A-domains (also referred to as class A module, complement type repeat, or LDL-receptor class A domain). They were developed from human extracellular receptor domains by *in vitro* exon shuffling and phage display (Silverman et al., 2005, Nat. Biotechnol. 23:1493-1494; Silverman et al., 2006, Nat. Biotechnol. 24:220). The resulting proteins can contain multiple independent binding domains that can exhibit improved affinity (in some cases, sub-nanomolar) and specificity compared with single-epitope binding proteins. See, for example, U.S. Patent Application Publ. Nos. 2005/0221384, 2005/0164301, 2005/0053973 and 2005/0089932, 2005/0048512, and 2004/0175756.

Each of the known 217 human A-domains comprises ∼35 amino acids (∼4 kDa); and these domains are separated by linkers that average five amino acids in length. Native A-domains fold quickly and efficiently to a uniform, stable structure mediated primarily by calcium binding and disulfide formation. A conserved scaffold motif of only 12 amino acids is required for this common structure. The end result is a single protein chain containing multiple domains, each of which represents a separate function. Each domain of the proteins binds independently and the energetic contributions of each domain are additive. These proteins were called "AVIMERs™" from avidity multimers.

The term "diabodies" refers to small antibody fragments with two antigen-binding sites, which fragments comprise a heavy chain variable domain (VH) connected to a light chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA 90:6444 6448 (1993).

Antigen-binding polypeptides also include heavy chain dimers such as, for example, antibodies from camelids and sharks. Camelid and shark antibodies comprise a homodimeric pair of two chains of V-like and C-like domains (neither has a light chain). Since the V_{H} region of a heavy chain dimer IgG in a camelid does not have to make hydrophobic interactions with a light chain, the region in the heavy chain that normally contacts a light chain is changed to hydrophilic amino acid residues in a camelid. V_{H} domains of heavy-chain dimer IgGs are called V_{HH} domains. Shark Ig-NARs comprise a homodimer of one variable domain (termed a V-NAR domain) and five C-like constant domains (C-NAR domains). In camelids, the diversity of antibody repertoire is determined by the CDRs 1, 2, and 3 in the V_{H} or V_{HH} regions. The CDR3 in the camel V_{HH} region is characterized by its relatively long length, averaging 16 amino acids (Muyldermans et al., 1994, Protein Engineering 7(9): 1129). This is in contrast to CDR3 regions of antibodies of many other species. For example, the CDR3 of mouse V_{H} has an average of 9 amino acids. Libraries of camelid-derived antibody variable regions, which maintain the *in vivo* diversity of the variable regions of a camelid, can be made by, for example, the methods disclosed in U.S. Patent Application Ser. No. 20050037421.

"Chimeric" forms of non-human (e.g., murine) antibodies include chimeric antibodies which contain minimal sequence derived from a non-human Ig. For the most part, chimeric antibodies are murine antibodies in which at least a portion of an immunoglobulin constant region (Fc), typically that of a human immunoglobulin are inserted in place of the murine Fc. For details, see Jones et al., Nature 321: 522-525 (1986); Reichmann et al., Nature 332: 323-329 (1988); and Presta, Curr. Op. Struct. Biol., 2: 593-596 (1992).

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations, which can include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, monoclonal antibodies can be made by the hybridoma method first described by Kohler et al., Nature 256:495 (1975), or can be made by recombinant DNA methods (see, e.g., U.S. Pat. No. 4,816,567). In certain embodiments, the monoclonal antibodies can be isolated from phage antibody libraries using the techniques described in Clackson et al., Nature 352:624-628 (1991) and Marks et al., J. Mol. Biol. 222:581-597 (1991), for example.

Antibodies can be isolated and purified from the culture supernatant or ascites mentioned above by saturated ammonium sulfate precipitation, euglobulin precipitation method, caproic acid method, caprylic acid method, ion exchange chromatography (DEAE or DE52), or affinity chromatography using anti-Ig column or a protein A, G or L column such as described in more detail below.

When constructing an immunoglobulin molecule, variable regions or portions thereof may be fused to, connected to, or otherwise joined to one or more constant regions or portions thereof to produce any of the antibodies described herein. This may be accomplished in a variety of ways known in the art, including but not limited to, molecular cloning techniques or direct synthesis of the nucleic acids encoding the molecules.

As used herein, "immunoreactive" refers to binding agents, antibodies or fragments thereof that are specific to a sequence of amino acid residues ("binding site" or "epitopes"), yet if are cross-reactive to other peptides/proteins, are not toxic at the levels at which they are formulated for administration to human use. The term "binding" refers to a direct association between two molecules, due to, for example, covalent, electrostatic, hydrophobic, and ionic and/or hydrogen-bond interactions under physiological conditions, and including interactions such as salt bridges and water bridges and any other conventional binding means. The term "preferentially binds" means that the binding agent binds to the binding site with greater affinity than it binds unrelated amino acid sequences. Affinity can be at least 1-fold greater, at least 2-fold greater, at least 3-fold greater, at least 4-fold greater, at least 5-fold greater, at least 6-fold greater, at least 7-fold greater, at least 8-fold greater, at least 9-fold greater, 10-fold greater, at least 20-fold greater, at least 30-fold greater, at least 40-fold greater, at least 50-fold greater, at least 60-fold greater, at least 70-fold greater, at least 80-fold greater, at least 90-fold greater, at least 100-fold greater, or at least 1000-fold greater than the affinity of the binding agent for unrelated amino acid sequences. The terms "immunoreactive" and "preferentially binds" are used interchangeably herein.

As used herein, the term "affinity" refers to the equilibrium constant for the reversible binding of two agents and is expressed as Kd. Affinity of a binding protein to a ligand such as affinity of an antibody for an epitope can be, for example, from about 100 nanomolar (nM) to about 0.1 nM, from about 100 nM to about 1 picomolar (pM), or from about 100 nM to about 1 femtomolar (fM). As used herein, the term "avidity" refers to the resistance of a complex of two or more agents to dissociation after dilution. Apparent affinities can be determined by methods such as an enzyme linked immunosorbent assay (ELISA) or any other technique familiar to one of skill in the art. Avidities can be determined by methods such as a Scatchard analysis or any other technique familiar to one of skill in the art.

"Epitope" refers to that portion of an antigen or other macromolecule capable of forming a binding interaction with the variable region binding pocket of an antibody. Such binding interactions can be manifested as an intermolecular contact with one or more amino acid residues of one or more CDRs. Antigen binding can involve, for example, a CDR3 or a CDR3 pair or, in some cases, interactions of up to all six CDRs of the V_{H} and V_{L} chains. An epitope can be a linear peptide sequence (i.e., "continuous") or can be composed of noncontiguous amino acid sequences (i.e., "conformational" or "discontinuous"). An antibody can recognize one or more amino acid sequences; therefore an epitope can define more than one distinct amino acid sequence. Epitopes recognized by antibodies can be determined by peptide mapping and sequence analysis techniques well known to one of skill in the art. Binding interactions are manifested as intermolecular contacts with one or more amino acid residues of a CDR. TRC105 is a murine antibody which is the same amino acid sequence as murine antibody described as Y4-2F1 or SN6j in US patent numbers 5,928,641; 6,200,566; 6,190,660; and 7,097,836. Epitopes recognized by Y4-2F1 and SN6j, and thus TRC105, have been previously identified.

The term "specific" refers to a situation in which an antibody will not show any significant binding to molecules other than the antigen containing the epitope recognized by the antibody. The term is also applicable where for example, an antigen binding domain is specific for a particular epitope which is carried by a number of antigens, in which case the antibody will be able to bind to the various antigens carrying the epitope. The terms "preferentially binds" or "specifically binds" mean that the antibodies bind to an epitope with greater affinity than it binds unrelated amino acid sequences, and, if cross-reactive to other polypeptides containing the epitope, are not toxic at the levels at which they are formulated for administration to human use. In one aspect, such affinity is at least 1-fold greater, at least 2-fold greater, at least 3-fold greater, at least 4-fold greater, at least 5-fold greater, at least 6-fold greater, at least 7-fold greater, at least 8-fold greater, at least 9-fold greater, 10-fold greater, at least 20-fold greater, at least 30-fold greater, at least 40-fold greater, at least 50-fold greater, at least 60-fold greater, at least 70-fold greater, at least 80-fold greater, at least 90-fold greater, at least 100-fold greater, or at least 1000-fold greater than the affinity of the antibody for unrelated amino acid sequences. The terms "immunoreactive," "binds," "preferentially binds" and "specifically binds" are used interchangeably herein. The term "binding" refers to a direct association between two molecules, due to, for example, covalent, electrostatic, hydrophobic, and ionic and/or hydrogen-bond interactions under physiological conditions, and includes interactions such as salt bridges and water bridges, as well as any other conventional means of binding.

"Isolated" (used interchangeably with "substantially pure") when applied to polypeptides means a polypeptide or a portion thereof which, by virtue of its origin or manipulation: (i) is present in a host cell as the expression product of a portion of an expression vector; or (ii) is linked to a protein or other chemical moiety other than that to which it is linked in nature; or (iii) does not occur in nature, for example, a protein that is chemically manipulated by appending, or adding at least one hydrophobic moiety to the protein so that the protein is in a form not found in nature. By "isolated" it is further meant a protein that is: (i) synthesized chemically; or (ii) expressed in a host cell and purified away from associated and contaminating proteins. The term generally means a polypeptide that has been separated from other proteins and nucleic acids with which it naturally occurs. Typically, the polypeptide is also separated from substances such as antibodies or gel matrices (polyacrylamide) which are used to purify it.

### ANGIOGENESIS TERMINOLOGY

As used herein, the terms "angiogenesis inhibitory," "angiogenesis inhibiting" or "anti-angiogenic" include inhibition of vasculogenesis, and are intended to mean affecting a decrease in the extent, amount, or rate of neovascularization. Effecting a decrease in the extent, amount, or rate of endothelial cell proliferation or migration in the tissue is a specific example of inhibiting angiogenesis.

The term "angiogenesis inhibitory composition" refers to a composition which inhibits angiogenesis-mediated processes such as endothelial cell migration, proliferation, tube formation and subsequently leading to the inhibition of the generation of new blood vessels from existing ones, and consequently affects angiogenesis-dependent conditions.

As used herein, the term "angiogenesis-dependent condition" is intended to mean a condition where the process of angiogenesis or vasculogenesis sustains or augments a pathological condition or beneficially influences normal physiological processes. Therefore, treatment of an angiogenesis-dependent condition in which angiogenesis sustains a pathological condition could result in mitigation of disease, while treatment of an angiogenesis-dependent condition in which angiogenesis beneficially influences normal physiological processes could result in, e.g., enhancement of a normal process.

Angiogenesis is the formation of new blood vessels from pre-existing capillaries or post-capillary venules. Vasculogenesis results from the formation of new blood vessels arising from angioblasts which are endothelial cell precursors. Both processes result in new blood vessel formation and are included in the meaning of the term angiogenesis-dependent conditions. The term "angiogenesis" as used herein is intended to include de novo formation of vessels such as that arising from vasculogenesis as well as those arising from branching and sprouting of existing vessels, capillaries and venules. Angiogenesis can also be inclusive of induction of ALK1 signaling and related Smad 1/5/8 phosphorylation and/or signaling. CD105 is also known to be involved in the ALK1 signaling pathway and is thus also included within the meaning of angiogenesis.

Tumor-initiating CD105⁺ cell populations have been found in human renal carcinomas. The CD105⁺ cells presented the characteristic of tumor stem cells previously described for cancer stem cells present in other tumor types. The CD105+ cells observed were clonogenic, expressed stem cell markers and lacked differentiative markers, could differentiate in vitro into epithelial and endothelial cell types and could generate in vivo serially transplantable tumors. The tumors, despite being derived from clones expressing mesenchymal markers, are epithelial carcinomas as the tumor of origin and are characterized by the maintenance of a CD105⁺ tumorigenic population and by the presence of a non-tumorigenic differentiated CD105- population.

"Inducing a host immune response" means that a patient experiences alleviation or reduction of signs or symptoms of illness, and specifically includes, without limitation, prolongation of survival.

As used herein, the terms "proliferative disorder" and "proliferative condition" mean any pathological or non-pathological physiological condition characterized by aberrant or undesirable proliferation. The terms "cell proliferative disorder" and "cell proliferative condition" mean any pathological or non-pathological physiological condition characterized by aberrant or undesirable cell proliferation, as well as including conditions characterized by undesirable or unwanted cell proliferation or cell survival (e.g., due to deficient apoptosis), conditions characterized by deficient or aberrant or deficient apoptosis, as well as conditions characterized by aberrant or undesirable or unwanted cell survival. The term "differentiative disorder" means any pathological or non-pathological physiological condition characterized by aberrant or deficient differentiation.

Proliferative or differentiative disorders amenable to treatment include diseases conditions, benign and neoplastic, characterized by abnormal or undesirable cell numbers, cell growth or cell survival. Such disorders or conditions may therefore constitute a disease state and include all types of cancerous growths or oncogenic processes, metastatic tissues or malignantly transformed cells, tissues, or organs.

Cells comprising the proliferative or differentiative disorder may be aggregated in a cell mass or be dispersed. A "non-solid tumor" refers to neoplasias of the hematopoietic system, such as lymphomas, myelomas and leukemias, or neoplasias that are diffuse in nature, as they do not typically form a solid mass. Particular examples of leukemias include for example, acute and chronic lymphoblastic, myeloblastic and multiple myeloma.

The term "solid tumor" refers to neoplasias or metastases that typically aggregate together and form a mass. Such disorders include neoplasms or cancers, which can affect virtually any cell or tissue type, e.g., carcinoma, sarcoma, melanoma, metastatic disorders or hematopoietic neoplastic disorders. A metastatic tumor can arise from a multitude of primary tumor types, including but not limited to breast, lung, thyroid, head and neck, brain, lymphoid, gastrointestinal (mouth, esophagus, stomach, small intestine, colon, rectum), genitourinary tract (uterus, ovary, cervix, bladder, testicle, penis, prostate), kidney, pancreas, liver, bone, muscle, skin, etc.

Carcinomas refer to malignancies of epithelial or endocrine tissue, and include respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from the cervix, lung, prostate, breast, head and neck, colon, liver and ovary. The term also includes carcinosarcomas, e.g., which include malignant tumors composed of carcinomatous and sarcomatous tissues. Adenocarcinoma includes a carcinoma of a glandular tissue, or in which the tumor forms a gland like structure.

A cancerous tissue to be treated is, for example, an endothelial tissue expressing an abnormal level of endoglin. As used herein, the term "transformed cells" refers to cells that have spontaneously converted to a state of unrestrained growth, i.e., they have acquired the ability to grow through an indefinite number of divisions in culture. Transformed cells may be characterized by such terms as neoplastic, anaplastic and/or hyperplastic, with respect to their loss of growth control. For purposes of this invention, the terms "transformed phenotype of malignant mammalian cells" and "transformed phenotype" are intended to encompass, but not be limited to, any of the following phenotypic traits associated with cellular transformation of mammalian cells: immortalization, morphological or growth transformation, and tumorigenicity, as detected by prolonged growth in cell culture, growth in semi-solid media, or tumorigenic growth in immuno-incompetent or syngeneic animals.

The term "tumor cell antigen" is defined herein as an antigen that is present in higher quantities on a tumor cell or in body fluids than unrelated tumor cells, normal cells, or in normal body fluid. The antigen presence may be tested by any number of assays known to those skilled in the art and include without limitation negative and/or positive selection with antibodies, such as an ELISA assay, a radioimmunoassay, or by Western Blot.

The terms "apoptosis" or "programmed cell death," refers to the physiological process by which unwanted or useless cells are eliminated during development and other normal biological processes. Apoptosis is a mode of cell death that occurs under normal physiological conditions and the cell is an active participant in its own demise ("cellular suicide"). It is most often found during normal cell turnover and tissue homeostasis, embryogenesis, induction and maintenance of immune tolerance, development of the nervous system and endocrine-dependent tissue atrophy. Cells undergoing apoptosis show characteristic morphological and biochemical features. These features include chromatin aggregation, nuclear and cytoplasmic condensation, partition of cytoplasm and nucleus into membrane bound vesicles (apoptotic bodies), which contain ribosomes, morphologically intact mitochondria and nuclear material. *In vivo,* these apoptotic bodies are rapidly recognized and phagocytized by macrophages, dendritic cells or adjacent epithelial cells. Due to this efficient mechanism for the removal of apoptotic cells *in vivo* no inflammatory response is elicited. *In vitro,* the apoptotic bodies as well as the remaining cell fragments ultimately swell and finally lyse. This terminal phase of in vitro cell death has been termed "secondary necrosis." Apoptosis can be measured by methods known to those skilled in the art like DNA fragmentation, exposure of Annexin V, activation of caspases, release of cytochrome c, etc. A cell that has been induced to die is termed herein as an "apoptotic cell."

"Apoptosis inducing agent" is defined herein to induce apoptosis/programmed cell death, and include, for example, anti-endoglin antibodies, anti-VEGF antibodies, irradiation, chemotherapeutic agents or receptor ligation agents, wherein cells, for example, tumor cells or endothelial cells are induced to undergo programmed cell death. Exemplary apoptosis inducing agents are described in more detail below.

Apoptosis can be tested using a standard Annexin V Apoptosis Assay: NIH:OVCAR-3 cells are grown in 6-well plates (NUNC) and irradiated or treated with an antagonist (or in combination with another anti-cancer drug) for 4-48 hours, washed and stained with Annexin V-FITC (BD-Pharmingen) for 1 hour. Cells are analyzed by flow cytometry (Becton-Dickinson, CellQuest), counterstained with Propidium Iodide and analyzed again in the flow cytometer.

### METHODS OF MAKING AND EXPRESSING ANTIBODIES

Chimeric immunoglobulins have been constructed by means of genetic engineering. Most chimeric immunoglobulins that have been previously described have comprised a V_{H} and V_{L} from a mouse monoclonal antibody and a C_{L} and Fc of a human antibody. Fc regions can be used from any of the isotypes described herein. As described herein, chimeric can also include criteria by which a limited number of amino acids in the framework of the light chain variable region and/or the heavy chain variable chain are modified in order to increase the affinity of an antibody.

Chimeric antibodies generally have several potential advantages over mouse antibodies for use in human therapy. Because the effector portion of an antibody is human, it is believed to interact better with the other parts of the human immune system (e.g., destroy the target cells more efficiently by complement-dependent cytotoxicity (CDC) or antibody-dependent cellular cytotoxicity (ADCC)). Additionally, the human immune system should not recognize the constant region of the chimeric antibody as foreign, and therefore the antibody response against such an injected antibody should, typically, be less than against a totally foreign mouse antibody. Finally, mouse antibodies are known to have a half-life in the human circulation that is much shorter than the half-life of human antibodies. Chimeric antibodies can, presumably, have a half-life more similar to naturally-occurring human antibodies, allowing smaller and less frequent doses to be given.

When increased affinity of a chimeric antibody is desired, residues within the CDRs of an antibody may be additionally substituted with other amino acids. Typically, no more than four amino acid residues in a CDR are changed, and most typically no more than two residues in the CDR will be changed, except for heavy chain CDR2, where as many as 10 residues may be changed. Changes in affinity can be measured by conventional methods such as those described herein (e.g., Biacore).

Chimeric antibodies can be constructed and produced using conventional techniques known in the art. In addition, recombinantly prepared antibodies can often be produced in large quantities, particularly when utilizing high level expression vectors.

For veterinary uses, an antibody can be synthesized for administration to a non-human (e.g., a primate, a cow, a horse, a pig, etc.) by using a non-human Fc.

Art-recognized techniques such as those provided herein, can be used to modify nucleotides encoding amino acid sequences using recombinant techniques in restriction endonuclease sites.

For expression, an expression system is one which utilizes the GS system (Lonza) using a glutamine synthetase gene as the selectable marker. Briefly, a transfection is performed in CHO cells by electroporation (250V) using the GS system (Lonza) using the glutamine synthetase gene as the selectable marker. Wild type CHO cells are grown in DMEM (Sigma) containing 10% dialyzed Fetal Calf Serum (FCS) with 2 mM glutamine. 6x10⁷ CHO cells are transfected with 300 µg of linearized DNA by electroporation. After electroporation the cells are resuspended in DMEM with glutamine and plated out into 36x96-well plates (50 µl/well), and incubated at 37° C. in 5% CO₂. The following day, 150 µl/well of selective medium (DMEM without glutamine) is added. After approximately 3 weeks the colonies are screened by ELISA (see below) using an irrelevant antibody as a negative control. All colonies producing >20 µg/ml are expanded into 24-well plates and then into duplicate T25 flasks.

For high level production, a widely used mammalian expression system is one which utilizes the gene amplification procedure offered by dehydrofolate reductase deficient ("dhfr- ") Chinese hamster ovary cells. The system is based upon the dehydrofolate reductase *"dhfr"* gene, which encodes the DHFR enzyme, which catalyzes conversion of dehydrofolate to tetrahydrofolate. In order to achieve high production, dhfr- CHO cells are transfected with an expression vector containing a functional DHFR gene, together with a gene that encodes a desired protein. In this case, the desired protein is recombinant antibody heavy chain and/or light chain.

By increasing the amount of the competitive DHFR inhibitor methotrexate (MTX), the recombinant cells develop resistance by amplifying the *dhfr* gene. In standard cases, the amplification unit employed is much larger than the size of the *dhfr* gene, and as a result the antibody heavy chain is co-amplified.

When large scale production of the protein, such as the antibody chain, is desired, both the expression level and the stability of the cells being employed are taken into account. In long term culture, recombinant CHO cell populations lose homogeneity with respect to their specific antibody productivity during amplification, even though they derive from a single, parental clone.

The present application provides an isolated polynucleotide (nucleic acid) encoding an antibody or portion thereof as described herein, vectors containing such polynucleotides, and host cells and expression systems for transcribing and translating such polynucleotides into polypeptides.

The present application also provides constructs in the form of plasmids, vectors, transcription or expression cassettes which comprise at least one polynucleotide as above.

The present application also provides a recombinant host cell which comprises one or more constructs as above. A nucleic acid encoding any antibody described herein forms an aspect of the present application, as does a method of production of the antibody, which method comprises expression from encoding nucleic acid therefrom. Expression can conveniently be achieved by culturing under appropriate conditions recombinant host cells containing the nucleic acid. Following production by expression, an antibody or a portion thereof can be isolated and/or purified using any suitable technique, then used as appropriate.

Specific antibodies (or portions thereof) encoding nucleic acid molecules and vectors containing same described herein can be provided isolated and/or purified, e.g., from their natural environment, in substantially pure or homogeneous form. In the case of nucleic acid, free or substantially free of nucleic acid or genes origin other than the sequence encoding a polypeptide with the required function. Nucleic acid sequences can comprise DNA or RNA and can be wholly or partially synthetic. Methods of purification are well known in the art.

Systems for cloning and expression of a polypeptide in a variety of different host cells are well known. Suitable host cells include bacteria, mammalian cells, yeast and baculovirus systems. Mammalian cell lines available in the art for expression of a heterologous polypeptide include, but are not limited to, Chinese hamster ovary cells, HeLa cells, baby hamster kidney cells, NS0 mouse myeloma cells and many others.

A wide variety of unicellular host cells are also useful in expressing the DNA sequences. These hosts include well-known eukaryotic and prokaryotic hosts, such as strains of *E. coli,* Pseudomonas, Bacillus, Streptomyces, fungi such as yeasts, and animal cells, such as CHO, YB/20, NS0, SP2/0, Rl.l, B-W and L-M cells, African Green Monkey kidney cells (e.g., COS 1, COS 7, BSC1, BSC40, and BMT10), insect cells (e.g., Sf9), and human cells and plant cells in tissue culture.

The expression of antibodies or portions thereof in prokaryotic cells such as *E. coli* is well established in the art. For a review, see for example Plückthun, A. Bio/Technology 9: 545-551 (1991).

Expression in eukaryotic cells in culture is also available to those skilled in the art as an option for production of the antibodies described herein, see for recent reviews, for example Raff, M.E. (1993) Curr. Opinion Biotech. 4: 573-576; Trill J.J. et al. (1995) Curr. Opinion Biotech 6: 553-560.

Suitable vectors can be chosen or constructed, containing appropriate regulatory sequences, including promoter sequences, terminator sequences, polyadenylation sequences, enhancer sequences, marker genes and other sequences as appropriate. Vectors can be plasmids, viral e.g. 'phage, or phagemid, as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Short Protocols in Molecular Biology, Second Edition, Ausubel et al. eds., John Wiley & Sons, 1992.

Thus, the application further provides a host cell containing nucleic acid as disclosed herein. The application still further provides a method comprising introducing such nucleic acid into a host cell. The introduction can employ any available technique. For eukaryotic cells, suitable techniques can include, for example, calcium phosphate transfection, DEAE Dextran, electroporation, liposome-mediated transfection and transduction using retrovirus or other virus, e.g., vaccinia or, for insect cells, baculovirus. For bacterial cells, suitable techniques can include, for example, calcium chloride transformation, electroporation and transfection using bacteriophage.

The introduction can be followed by causing or allowing expression from the nucleic acid, e.g. by culturing host cells under conditions for expression of the gene.

In one instance, the nucleic acid is integrated into the genome (e.g. chromosome) of the host cell. Integration can be promoted by inclusion of sequences which promote recombination with the genome, in accordance with standard techniques. Ig enhances can be initialized as needed to maximize expression.

The present application also describes a method which comprises using a construct as stated above in an expression system in order to express the antibodies (or portions thereof) as above.

The present application also relates to isolated nucleic acids, such as recombinant DNA molecules or cloned genes, or degenerate variants thereof, mutants, analogs, or fragments thereof, which encode a chimeric antibody that binds endoglin.

In a further instance, the full DNA sequence of the recombinant DNA molecule or cloned gene of an antibody or portion thereof described herein can be operatively linked to an expression control sequence which can be introduced into an appropriate host. The application accordingly extends to unicellular hosts transformed with the cloned gene or recombinant DNA molecule comprising a DNA sequence encoding the V_{H}, V_{L}, C_{L} and/or Fc, of the antibody.

Another feature is the expression of the DNA sequences disclosed herein. As is well known in the art, DNA sequences can be expressed by operatively linking them to an expression control sequence in an appropriate expression vector and employing that expression vector to transform an appropriate unicellular host.

Such operative linking of a DNA sequence to an expression control sequence, of course, includes, if not already part of the DNA sequence, the provision of an initiation codon, ATG, in the correct reading frame upstream of the DNA sequence.

Polynucleotides and vectors can be provided in an isolated and/or a purified form (e.g., free or substantially free of polynucleotides of origin other than the polynucleotide encoding a polypeptide with the required function). As used herein, "substantially pure," and "substantially free" refer to a solution or suspension containing less than, for example, about 20% or less extraneous material, about 10% or less extraneous material, about 5% or less extraneous material, about 4% or less extraneous material, about 3% or less extraneous material, about 2% or less extraneous material, or about 1% or less extraneous material.

A wide variety of host/expression vector combinations can be employed in expressing the DNA sequences. Useful expression vectors, for example, can consist of segments of chromosomal, non-chromosomal and synthetic DNA sequences. Suitable vectors include, but are not limited to, derivatives of SV40 and known bacterial plasmids, e.g., *E. coli* plasmids col El, Pcr1, Pbr322, Pmb9 and their derivatives, plasmids such as RP4; phage DNAs, e.g., the numerous derivatives of phage λ, e.g., NM989, and other phage DNA, e.g., M13 and filamentous single stranded phage DNA; yeast plasmids such as the 2u plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like.

Also described herein is a recombinant host cell which comprises one or more polynucleotide constructs. A polynucleotide encoding an antibody as provided herein forms an part of the present application, as does a method of production of the antibody which method comprises expression from one or more polynucleotides. Expression can be achieved, for example, by culturing under appropriate conditions recombinant host cells containing the polynucleotide. An antibody can then be isolated and/or purified using any suitable technique, and used as appropriate.

Any of a wide variety of expression control sequences - sequences that control the expression of a DNA sequence operatively linked to it - can be used in these vectors to express the DNA sequences. Such useful expression control sequences include, for example, the early or late promoters of SV40, CMV, vaccinia, polyoma or adenovirus, the lac system, the trp system, the TAC system, the TRC system, the LTR system, the major operator and promoter regions of phage λ, the control regions of fd coat protein, the promoter for 3-phosphoglycerate kinase or other glycolytic enzymes, the promoters of acid phosphatase (e.g., Pho5), the promoters of the yeast -mating factors, and other sequences known to control the expression of genes of prokaryotic or eukaryotic cells or their viruses, and various combinations thereof.

It will be understood that not all vectors, expression control sequences and hosts will function equally well to express the DNA sequences. Neither will all hosts function equally well with the same expression system. However, one skilled in the art will be able to select the proper vectors, expression control sequences, and hosts without undue experimentation to accomplish the desired expression without departing from the scope of this application. For example, in selecting a vector, the host must be considered because the vector must function in it. The vector's copy number, the ability to control that copy number, and the expression of any other proteins encoded by the vector, such as antibiotic markers, will also be considered. One of ordinary skill in the art can select the proper vectors, expression control sequences, and hosts to accomplish the desired expression without departing from the scope of this application. For example, in selecting a vector, the host is considered because the vector functions in it. The vector's copy number, the ability to control that copy number, and the expression of any other proteins encoded by the vector, such as antibiotic markers, can also be considered.

The present application also describes constructs in the form of plasmids, vectors, transcription or expression cassettes as described elsewhere herein which comprise at least one polynucleotide as above. Suitable vectors can be chosen or constructed, containing appropriate regulatory sequences, including promoter sequences, terminator sequences, polyadenylation sequences, enhancer sequences, selectable markers and other sequences as appropriate. Vectors can be plasmids, viral e.g., phage, phagemid, etc., as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Short Protocols in Molecular Biology, Second Edition, Ausubel et al. eds., John Wiley & Sons, 1992.

In selecting an expression control sequence, a variety of factors will normally be considered. These include, for example, the relative strength of the system, its controllability, and its compatibility with the particular DNA sequence or gene to be expressed, particularly as regards potential secondary structures. Suitable unicellular hosts will be selected by consideration of, e.g., their compatibility with the chosen vector, their secretion characteristics, their ability to fold proteins correctly, and their fermentation requirements, as well as the toxicity to the host of the product encoded by the DNA sequences to be expressed, and the ease of purification of the expression products.

Also provided is a host cell containing one or more polynucleotides as disclosed herein. Yet further provided is a method of introducing such one or more polynucleotides into a host cell, any available technique. For eukaryotic cells, suitable techniques can include, for example, calcium phosphate transfection, DEAE Dextran, electroporation, liposome-mediated transfection and transduction using retrovirus or other virus (e.g., vaccinia) or, for insect cells, baculovirus. For bacterial cells, suitable techniques can include, for example calcium chloride transformation, electroporation and transfection using bacteriophages.

The introduction can be followed by causing or allowing expression from the one or more polynucleotides, e.g. by culturing host cells under conditions for expression of one or more polypeptides from one or more polynucleotides. Inducible systems can be used and expression induced by addition of an activator.

In one instance, the polynucleotides can be integrated into the genome (e.g., chromosome) of the host cell. Integration can be promoted by inclusion of sequences which promote recombination with the genome, in accordance with standard techniques. In another embodiment, the nucleic acid is maintained on an episomal vector in the host cell.

Methods are described herein which include using a construct as stated above in an expression system in order to express a specific polypeptide.

Considering these and other factors, a person skilled in the art will be able to construct a variety of vector/expression control sequence/host combinations that will express the DNA sequences on fermentation or in large scale animal culture.

A polynucleotide encoding an antibody or a portion thereof can be prepared recombinantly/synthetically in addition to, or rather than, cloned. The polynucleotide can be designed with the appropriate codons. In general, one will select preferred codons for an intended host if the sequence will be used for expression. The complete polynucleotide can be assembled from overlapping oligonucleotides prepared by standard methods and assembled into a complete coding sequence. See, e.g., Edge, Nature, 292:756 (1981); Nambair et al., Science, 223:1299 (1984); Jay et al., J. Biol. Chem., 259:6311 (1984).

Simultaneous incorporation of the antibody (or portion thereof)-encoding nucleic acids and the selected amino acid position changes can be accomplished by a variety of methods known to those skilled in the art, including for example, recombinant and chemical synthesis.

### ANTI-ENDOGLIN ANTIBODIES

Endoglin (CD105) is expressed on the cell surface as a 180 kDa homodimeric transmembrane protein. The external domain binds TGF-β1 and -3 isoforms with high affinity (50 nM), and the transmembrane and the intracellular domains of CD105 share a 71 % sequence similarity with betaglycan. The human CD105 gene is located on chromosome 9q34, identified using fluorescence *in situ* hybridization, and the coding region contains 14 exons, and two different isoforms (L and S) of CD105 with capacity to bind TGF- β have been characterized. The L-CD105 consists of 633 amino acid residues with 47 amino acid residues in the cytoplasmic tail as opposed to the S-CD105, which consists of 600 amino acid residues with a 14 amino acid cytoplasmic tail. However, L-CD105 is the predominant form. CD105 is constitutively phosphorylated in endothelial cells, mainly on serine and threonine residues, and this phosphorylation is due to the constitutively active TGF- β RII within the cell. TGF- β binding to CD105 results in down-regulation of phosphorylation, similar to effects seen with protein kinase C inhibitors. The human CD105 amino acid sequence contains the tripeptide arginine-glycine-aspartic acid (RGD) located in an exposed region of the extracellular domain. The RGD peptide is a key recognition structure found on ECM proteins such as fibronectin, vitronectin, von Willebrand factor (vWF), type I collagen, and fibrinogen and is recognized by cell surface integrins. Integrin adhesion has been implicated in hemostasis, thrombosis, angiogenesis and inflammation, processes in which the endothelium plays a critical role. (Duff et al., FASEB J., 17:984-992 (2003)).

CD105 is a member of the TGF-β receptor family that is expressed by proliferating endothelial cells. Normal levels of CD105 are needed for endothelial cell proliferation. CD105 expression is increased by cellular hypoxia through the production of hypoxia-inducible factor-1-α (HIF-1-α) and protects hypoxic cells from apoptosis. Several functions of CD105 are associated with TGF-β signaling. TGF-β signals through heterodimeric receptors consisting of serine kinases, receptor I (RI), and receptor II (RII). Binding of TGF-β to the external domains of the receptor unmasks the cytoplasmic RII kinase activity that phosphorylates the TGF-β RI, which can then interact with downstream signalers such as the Smad proteins. CD105 forms part of the TGF-β receptor complex but it can exist independently on the cell surface. In many cells *in vitro,* CD105 suppresses TGF-β signaling.

CD105 also binds other growth factors such as activin A and bone morphogenic proteins (BMP) -10, -9, -7 and -2. Binding of TGF-β or other growth factor ligands to CD105 requires the presence of at least the receptor RII, and it cannot bind ligands by itself. CD105 association with receptors does not alter their affinity for the ligand itself. Upon association, the cytoplasmic domain of CD105 is phosphorylated by TGF-β RI and TGF-β RII; then TGF-β RI, but not TGF-β RII, kinase dissociates from the receptor complex.

CD105 expression inhibits phosphorylation levels of TGF-β RII but increases that of TGF-β RI, resulting in increased phosphorylation of Smad 2 but not Smad 3. Since Smad 2 can interact with a variety of transcription factors, co-activators, and suppressors, phosphorylated Smad 2 may act as an integrator of multiple signals to modulate gene transcription. Thus, CD105 modulates TGF-β functions via interaction with TGF-β RI and TGF-β RII and modifies the phosphorylation of downstream Smad proteins.

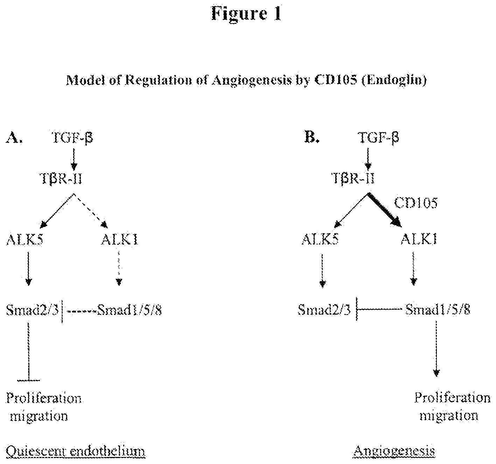

CD105 acts to modulate signaling of multiple kinase receptor complexes of the TGF-β superfamily, including TGF-β receptors (TGF-βR), activin receptor-like kinases (ALK) and activin receptors. In the absence of CD105, activation of TGF-β receptors results in phosphorylation of SMAD proteins (SMAD 2 and 3) that inhibit endothelial cell growth. However, activation of CD105 by TGF-β modulates SMAD protein phosphorylation (including the phosphorylation of SMAD 1, 5 and 8). The end result is release of the growth inhibitory effects of TGF-β receptor activation on endothelial cells (see Figure 1). Not surprisingly, prevention of CD105 activation by anti-CD105 antibody or antisense oligonucleotide acts synergistically with TGF-β to suppress endothelial cell growth.

The CD105 promoter is 2.6 kb in length but does not contain TATA or CAAT transcription initiation boxes. However, it has two GC-rich regions, consensus motifs for Sp1, ets, GATA, AP-2, NGF-β, and Mad, as well as TGF-β response elements. Nonetheless, CD105 has a relatively restricted cellular distribution. The basal level of transcription appears to require an ets site at position -68 and the Sp1 sites, but the relative restriction of expression, for example, to endothelial cells, appears to involve multiple regulatory regions, in particular, one at -1294 to -932 and another very close to the transcription initiation site. CD105 is up-regulated by TGF-β, and this has been shown to require a Sp1 site at-37 to -29, also involving one or more juxtaposed upstream SBE sites binding Smads 3 and/or 4 (which are activated by TGF-β signaling). Hypoxia is a common feature of ischemic tissues and tumors, and is a potent stimulator for CD105 gene expression in vascular endothelial cells (ECs). Such an effect is potentiated in combination with TGF-β 1. The up-regulated CD105 can exert a self-protective role in ECs under hypoxic stress.

Vascular EC are the major source of CD105. Other cell types including vascular smooth muscle cells, fibroblasts, macrophages, leukemic cells of pre-B and myelomonocytic origin, and erythroid precursors express CD105 to a lesser extent.

CD105 is involved in angiogenesis. Antisense experiments have demonstrated that suppression of CD105 expression in HUVEC results in marked inhibition of in vitro angiogenesis in combination with TGF-β1, indicating that CD105 is a proangiogenic component in the endothelial cells. Further evidence of the important role of CD105 in angiogenesis comes from CD105 knockout mice. The CD105 null mice exhibit multiple vascular and cardiac defects leading to death at an early embryonic stage. Severe vascular impairments observed in CD105 null mice indicate that CD105 is required for the formation of mature blood vessels in the extraembryonic vasculature, further confirming the direct role of endoglin in angiogenesis.

Endoglin, also known as, *inter alia,* CD105 or edg-1, is a type I homodimeric membrane glycoprotein which is expressed at high levels in proliferating vascular endothelial cells. Thus, endoglin is primarily a proliferation-associated marker for endothelial cells undergoing active angiogenesis. However, there may be limited expression of endoglin by the vascular endothelium of normal tissues. Human endoglin is known to specifically bind transforming growth factor-β (TGF-β), and the deduced amino acid sequence of endoglin has strong homology to β-glycan, a type of TGF-β receptor.

Endoglin (EDG) has been targeted in antibody-based methods of reducing tumor vasculature, as EDG is a proliferation-associated antigen on endothelial and leukemia cells. Its expression is up-regulated in tumor-associated vascular endothelium, and EDG is essential for angiogenesis. Angiogenesis includes the formation of new capillary blood vessels leading to neovascularization as well as the maintenance of the existing vasculature. It is a complex process which includes a series of sequential steps including endothelial cell-mediated degradation of vascular basement membrane and interstitial matrices, migration of endothelial cells, proliferation of endothelial cells, and formation of capillary loops by endothelial cells.

Provided herein are chimeric antibodies that bind endoglin. Endoglin can be found on cells that comprise and support existing vasculature as well as cells that are promoting the growth of, and become part of, new vasculature. These antibodies can bind endoglin and thereby inhibit angiogenesis, inhibit the existing vasculature or the maintenance of the existing vasculature, and/or inhibit small vessel dilation. In addition to their use for purification of endoglin, these antibodies are useful for purification, detection and diagnostic purposes as well as therapeutic purposes. The antibodies provided herein can be used for the formulation of medicaments for the treatment a variety of conditions and diseases, methods to treat said conditions and diseases and methods of detection or diagnosis. As used herein, angiogenesis is inclusive of the growth and/or development of new blood vessels (also referred to as neovascularization), dilation of the small vessels, excessive or prolonged vascular growth, and maintenance of the existing vasculature. Angiogenic conditions and diseases refer to those diseases and conditions related to, caused by, or associated with angiogenesis. Non-limiting examples of such diseases include, for example, various forms of cancer (primary tumors and metastases).

Murine monoclonal antibodies (mAbs) have been raised against endoglin which modulate endoglin activity and thereby inhibit angiogenesis and/or inhibit vasodilation of small blood vessels. These murine antibodies are described in U.S. patents 5,928,641, 6,200,566, 6,190,660, and 7,097,836. Additionally, the *ex vivo* and *in vivo* efficiency of a number of these antibodies has been demonstrated; monoclonal antibodies that bind endoglin are of interest as endoglin modulating compounds. Therapeutic use of murine antibodies is not feasible, however, as administration of the murine antibodies has a number of limitations, including immunogenicity in, for example, the form of human anti-mouse antibodies (HAMA).

Several anti-endoglin antibodies, in particular anti-endoglin monoclonal antibodies ("mAb"), have been described. MAb SN6 is an antibody generated from immunization of mice with glycoprotein mixtures of cell membranes of human leukemia cells (Haruta and Seon, 1986, Proc. Natl. Acad. Sci. 83:7898-7902). SN6 is a murine mAb that recognizes human endoglin. MAb 44G4 is an antibody generated from immunization of mice with whole cell suspensions of human pre-B leukemia cells (Gougos and Letarte, 1988, J. Immunol. 141:1925-1933; 1990, J. Biol. Chem. 265:8361-8364). 44G4 is also a murine mAb that recognizes human endoglin. MAb MJ7/18 is an antibody generated from immunization of rats with inflamed mouse skins (Ge and Butcher, 1994, supra). MJ7/18 is also a mAb that recognizes murine endoglin. mAb Tec-11 is an antibody generated from immunization of mice with human umbilical vein endothelial cells (Burrows et al., 1995, Clin. Cancer Res. 1:1623-1634). Tec-11 is a murine mAb with reactivity restricted to human endoglin. Uneda et al (2009) Int. J. Cancer: 125, 1446-53 describes a study in which 3 anti-endoglin monoclonal antibodies SN6a, SN6j and SN6k, which define individually distinct epitopes of endoglin of tumour vasculature, are capable of suppressing tumor metastasis in metastasis models. Chimeric antibodies that bind endoglin are described herein that exhibit reduced immunogenicity while maintaining and/or improving their specificity. Additionally, to address problems associated with murine antibodies, chimeric antibodies that bind endoglin and decrease and/or inhibit angiogenesis are described herein that exhibit reduced immunogenicity while maintaining and/or improving their specificity. These chimeric anti-endoglin antibodies are useful for the diagnosis and treatment of various conditions and diseases as well as for purification and detection of endoglin. Antibodies against endoglin represent an important area for the development of therapies for the treatment of a variety of diseases and conditions which involve, are influenced by, or affected by angiogenesis.

Provided herein are chimeric antibodies thereof that bind to endoglin. Also provided are chimeric antibodies thereof that bind endoglin and inhibit (partially or fully) or manage/treat (partially or fully) angiogenesis/neovascularization, dilation of small vessels, inhibition of cell proliferation or inhibition or tumor growth. Similarly, inhibition of endoglin function (e.g., signaling, binding, activation, and the like) is also included within the meaning of inhibiting or binding endoglin. In yet another embodiment, a chimeric antibody inhibits angiogenesis by binding to endoglin. The application also provides cell lines which can be used to produce the chimeric antibodies, methods for producing the cell lines, methods for expressing antibodies and purifying the same.

One can recognize that the antibodies that specifically bind endoglin generated using the methods described herein can be tested using the assays provided herein or known in the art for the ability to bind to endoglin using conventional methods including, but not limited to, ELISA. Affinity of antibodies described herein can also be determined using conventional methods including, but not limited to, Biacore or surface plasmon resonance.

Provided herein are chimeric antibodies that bind endoglin. Also provided herein are chimeric antibodies that bind endoglin and inhibit angiogenesis.

Provided herein is a chimeric antibody comprising a light chain variable region having an amino acid sequence set forth as SEQ ID NO: 1, a light chain constant region having an amino acid sequence set forth as SEQ ID NO: 2, a heavy chain variable region having an amino acid sequence set forth as SEQ ID NO: 3 and a gamma 1 (γ1) constant region (Fc) having an amino acid sequence set forth as SEQ ID NO: 4.

In another aspect, the present application provides a chimeric antibody capable of competing with a chimeric anti-endoglin antibody described herein under conditions in which at least 5% of an antibody having the V_{H} and V_{L} sequences of the antibody is blocked from binding to endoglin by competition with such an antibody in an ELISA assay.

Provided herein are neutralizing chimeric antibodies that bind to endoglin and modulate the activity of endoglin. The neutralizing antibody can for example, inhibit angiogenesis by binding to endoglin.

Percentage of (%) inhibition of angiogenesis, cell proliferation and/or tumor growth by a chimeric anti-endoglin antibody of at least 2-fold, at least 3-fold, at least 4-fold, at least 5-fold, at least 6-fold, at least 7-fold, at least 8-fold, at least 9-fold, at least 10-fold, at least 20-fold, at least 30-fold, at least 40-fold, at least 50-fold, at least 60-fold, or greater than negative controls is indicative of a antibody inhibits angiogenesis. Percentage (%) of inhibition of angiogenesis, cell proliferation and/or tumor growth by a chimeric anti-endoglin antibody of less than 2-fold greater than negative controls is indicative of an antibody that does not inhibit angiogenesis.

Binding of a chimeric antibody to endoglin can partially (e.g., 5%, 10%, 20%, 30%, 40%, 50%, 60%, 70%, 80%, 90%, 95%, 98%, 99% or any number therein) or completely inhibit angiogenesis, cell proliferation and/or tumor growth. The neutralizing or inhibiting activity of a chimeric antibody can be determined using an *in vitro* assay and/or *in vivo* using art-recognized assays such as those described herein or otherwise known in the art.

Antibodies described herein are useful in detection or diagnostic applications as described in more detail below. Antibodies described herein are useful for binding to endoglin, which, in turn, can inhibit angiogenesis as described herein.

### ANTI-VEGF AGENTS

Vascular endothelial growth factor (VEGF) has been identified as a protein that induces proliferation and migration of endothelial cells *in vitro,* and blood vessel permeabilization and angiogenesis in vivo. It regulates both vascular proliferation and permeability. Also known as vascular permeability factor (VPF), it is unique among pro-angiogenic factors because of its specificity for vascular endothelium and potency. It also functions as an anti-apoptotic factor for endothelial cells in newly formed vessels. VEGF is expressed in tumor cells, macrophages, T cells, smooth muscle cells, kidney cells, mesangial cells, keratinocytes, astrocytes, and osteoblasts.

"Human VEGF" refers in one embodiment to the 165-amino acid human vascular endothelial cell growth factor, and related 121-, 189-, and 206-amino acid vascular endothelial cell growth factors, as described by Leung et al., Science 246: 1306 (1989); and Houck et al., Mol. Endocrin., 5: 1806 (1991) together with the naturally occurring allelic and processed forms of those growth factors. The term "VEGF" also refers to VEGFs from non-human species such as mouse, rat or primate. In some instances, VEGF from a specific species is indicated by terms such as hVEGF for human VEGF, mVEGF for murine VEGF, and etc. The term "VEGF" is also used to refer to truncated forms of the polypeptide comprising amino acids 8 to 109 or 1 to 109 of the 165-amino acid human vascular endothelial cell growth factor. The amino acid positions for a "truncated" native VEGF are numbered as indicated in the native VEGF sequence. For example, amino acid position 17 (methionine) in truncated native VEGF is also position 17 (methionine) in native VEGF. The truncated native VEGF has binding affinity for the KDR and Flt-1 receptors comparable to native VEGF. According to one embodiment, the VEGF is a human VEGF.

The VEGF family comprises seven members, including VEGF-A, VEGF-B, VEGF-C, VEGF-D, VEGF-E, VEGF-F, and placenta growth factor (PIGF). All of them have a common structure of eight cysteine residues in a VEGF homology domain. In addition, in relation to VEGF-A, there are six different isoforms, and VEGF-A165 is the main isoform. All these isoforms have distinct and overlapping functions in angiogenesis. The VEGF gene is located on chromosome 6p.21. The different members of VEGF family have different physical and biological properties and they act through specific tyrosine kinase receptors (VEGFR-1, VEGFR-2, and VEGFR-3). The VEGFR-3 receptor and its ligands, VEGF-C and VEGF-D, are associated with lymphangiogenesis, while PIGF is linked to arteriogenesis.

Two high affinity receptors for VEGF have been characterized, VEGFR-1/Flt1 (fms-like tyrosine kinase-1) and VEGFR-2/Kdr/Flk-1 (kinase insert domain containing receptor/fetal liver kinase-1). A third receptor, VEGFR-3 is also known. These receptors are classified in the PDGF-receptor family. However, the VEGF receptors have seven immunoglobulin-like loops in their extracellular domains (as opposed to five in other members of the PDGF family) and a longer kinase insert. The expression of VEGF receptors occurs mainly in vascular endothelial cells, although some may also be present on monocytes and on melanoma cell lines. Only endothelial cells have been shown to proliferate in response to VEGF, and endothelial cells from different sources show different responses. Thus, the signals mediated through VEGFR-1, VEGFR-2 and VEGFR-3 appear to be cell-type specific.

VEGFR-1 and VEGFR-2 bind VEGF 165 with high affinity (Kd about 20 pM and 200 pM, respectively). The Flk-1 receptor has also been shown to undergo autophosphorylation in response to VEGF. VEGFR-2 mediated signals causing striking changes in the morphology, actin reorganization and membrane ruffling of porcine aortic endothelial cells over-expressing this receptor. In these cells, VEGFR-2 also mediated ligand-induced chemotaxis and mitogenicity; whereas VEGFR-1 transfected cells lacked mitogenic responses to VEGF. In contrast, VEGF had a strong growth stimulatory effect on rat sinusoidal endothelial cells expressing VEGFR-1. Phosphoproteins co-precipitating with VEGFR-1 and VEGFR-2 are distinct, suggesting that different signaling molecules interact with receptor specific intracellular sequences.

VEGF represents one target for antitumor therapies because its expression is upregulated in a range of solid tumors. VEGF is a major regulator of angiogenesis, the growth of new vessels from pre-existing vessels. This process is fundamental to the growth of solid tumors, which rely on the formation of new blood vessels. Certain small molecule therapeutic agents are able to target vascular endothelial growth factor receptor ("VEGFR"); such targeting by small molecule therapeutics can result in anti-cancer effects. VEGF receptor-targeted agents indirectly block tumor growth, through the inhibition of new vessel formation. Inhibiting VEGF-induced angiogenesis can exert an anti-tumor or improved anti-tumor effect without significantly inhibiting VEGF stimulation of macrophages, osteoclasts or chondroclasts.

In one embodiment provided herein, a VEGF antagonist is an antibody including, but not limited to, monoclonal antibody, a chimeric antibody, human and a humanized antibody. According one specific embodiment, the anti-VEGF antibody is bevacizumab (AVASTIN®). According to another embodiment, the anti-VEGF antibody is selected from the group consisting of a Fab, Fab', a F(ab)'₂, single-chain Fv (scFv), a half-antibody, a single chain binding polypeptide, a Fv fragment, a diabody and a linear antibody. Thus, in an embodiment the anti-VEGF antibody is ranibizumab (LUCENTIS®).

A "VEGF antagonist" refers to a molecule capable of neutralizing, blocking, inhibiting, abrogating, reducing or interfering with VEGF activities including its binding to VEGF or one or more VEGF receptors or the nucleic acid encoding them. In some instances, the VEGF antagonist binds VEGF or a VEGF receptor. According to one embodiment, a VEGF antagonist binds to VEGF and inhibits VEGF-induced endothelial cell proliferation *in vitro.*

According to one embodiment, a VEGF antagonist binds to VEGF or a VEGF receptor with greater affinity than a non-VEGF or non-VEGF receptor. According to another embodiment, a VEGF antagonist binds to VEGF or a VEGF receptor with a Kd of between 1 uM and 1 µM. According to another embodiment, the VEGF antagonist binds to VEGF or a VEGF receptor between 500 nM and 1 µM.

An "anti-VEGF antibody" is an antibody that binds to VEGF with sufficient affinity and specificity. An anti-VEGF antibody of the invention can be used as a therapeutic agent in targeting and interfering with diseases or conditions wherein the VEGF activity is involved. An anti-VEGF antibody will usually not bind to other VEGF homologues such as VEGF-B or VEGF-C, or to other growth factors such as P1GF, PDGF or bFGF.

The anti-VEGF antibody "Bevacizumab," also known as "rhuMAb VEGF" or "AVASTIN®," is a recombinant humanized anti-VEGF monoclonal antibody generated according to methods described by Presta et al. (1997) Cancer Res. 57:4593-4599. It contains mutated human IgG1 framework regions and antigen-binding complementarity-determining regions from the murine anti-hVEGF monoclonal antibody A.4.6.1 that blocks binding of human VEGF to its receptors. Approximately 93% of the amino acid sequence of Bevacizumab, including most of the framework regions, is derived from human IgG1, and about 7% of the sequence is derived from the murine antibody A4.6.1. Bevacizumab has a molecular mass of about 149,000 Daltons and is glycosylated. Amino acid sequences associated with the murine monoclonal version and humanization of Bevacizumab are provided as SEQ ID NOS: 5-11.

In certain embodiments, the VEGF receptor inhibitor to be administered in a combination composition is a small molecule inhibitor of VEGF. In other embodiments, the VEGF receptor inhibitor to be administered in a combination composition is an anti-VEGF antibody. In one non-limiting example, the VEGF receptor inhibitor to be administered in a combination composition is bevacizumab (AVASTIN®). Exemplary, non-limiting dosages for bevacizumab are discussed in more detail below and in the examples.

### MODIFIED ANTIBODIES OR PORTIONS THEREOF

Antibodies described herein can further comprise a therapeutic moiety for use in therapeutic applications.

Antibodies described herein can also be used as immunoconjugates. As used herein, for purposes of the specification and claims, immunoconjugates refer to conjugates comprised of the chimeric anti-endoglin antibodies or fragments thereof according to the present invention and at least one therapeutic label. Therapeutic labels include antitumor agents and angiogenesis-inhibitors. Such antitumor agents are known in the art and include, but not limited to, toxins, drugs, enzymes, cytokines, radionuclides, photodynamic agents, and angiogenesis inhibitors. Toxins include, but are not limited to, ricin A chain, mutant Pseudomonas exotoxins, diphtheria toxoid, streptonigrin, boamycin, saporin, gelonin, and pokeweed antiviral protein. Drugs include daunorubicin, methotrexate, and calicheamicins. Radionuclides include radiometals. Cytokines include, but are not limited to, transforming growth factor (TGF)-β, interleukins, interferons, and tumor necrosis factors. Photodynamic agents include, but are not limited to, porphyrins and their derivatives. Additional therapeutic labels will be known in the art and are also contemplated herein. The methods for complexing the anti-endoglin mAbs or a fragment thereof with at least one antitumor agent are well known to those skilled in the art (i.e., antibody conjugates as reviewed by Ghetie et al., 1994, Pharmacol. Ther. 63:209-34). Such methods may utilize one of several available heterobifunctional reagents used for coupling or linking molecules. Additional radionuclides are further described herein along with additional methods for linking molecules, such as therapeutic and diagnostic labels.

Antibodies can be modified using techniques known in the art for various purposes such as, for example, by addition of polyethylene glycol (PEG). PEG modification (PEGylation) can lead to one or more of improved circulation time, improved solubility, improved resistance to proteolysis, reduced antigenicity and immunogenicity, improved bioavailability, reduced toxicity, improved stability, and easier formulation (for a review see, Francis et al., International Journal of Hematology 68:1-18, 1998).

Fc portions of antibodies can be modified to increase half-life of the in circulation in blood when administered to a patient. Modifications can be determined using conventional means in the art such as, for example, described in U.S. Patent No. 7,217,798.

Other methods of improving the half-life of antibody-based fusion proteins in circulation are also known such as, for example, described in U.S. Patent Nos. 7,091,321 and 6,737,056. Additionally, antibodies may be produced or expressed so that they do not contain fucose on their complex N-glycoside-linked sugar chains. The removal of the fucose from the complex N-glycoside-linked sugar chains is known to increase effector functions of the antibodies and antigen-binding fragments, including but not limited to, antibody dependent cell-mediated cytotoxicity (ADCC) and complement dependent cytotoxicity (CDC). Similarly, antibodies that can bind endoglin can be attached at their C-terminal end to all or part of an immunoglobulin heavy chain derived from any antibody isotype, e.g., IgG, IgA, IgE, IgD and IgM and any of the isotype subclasses, particularly IgG1, IgG2b, IgG2a, IgG3 and IgG4.

Additionally, the antibodies described herein can also be modified so that they are able to cross the blood-brain barrier. Such modification of the antibodies described herein allows for the treatment of brain diseases such as glioblastoma multiforme (GBM). Exemplary modifications to allow proteins such as antibodies to cross the blood-brain barrier are described in US Patent Publication 20070082380.

Glycosylation of immunoglobulins has been shown to have significant effects on their effector functions, structural stability, and rate of secretion from antibody-producing cells (Leatherbarrow et al., Mol. Immunol. 22:407 (1985)). The carbohydrate groups responsible for these properties are generally attached to the constant (C) regions of the antibodies. For example, glycosylation of IgG at asparagine 297 in the C_{H} 2 domain is required for full capacity of IgG to activate the classical pathway of complement-dependent cytolysis (Tao and Morrison, J. Immunol. 143:2595 (1989)). Glycosylation of IgM at asparagine 402 in the C_{H} 3 domain is necessary for proper assembly and cytolytic activity of the antibody (Muraoka and Shulman, J. Immunol. 142:695 (1989)). Removal of glycosylation sites as positions 162 and 419 in the C_{H} 1 and C_{H}3 domains of an IgA antibody led to intracellular degradation and at least 90% inhibition of secretion (Taylor and Wall, Mol. Cell. Biol. 8:4197 (1988)). Additionally, antibodies may be produced or expressed so that they do not contain fucose on their complex N-glycoside-linked sugar chains. The removal of the fucose from the complex N-glycoside-linked sugar chains is known to increase effector functions of the antibodies and antigen-binding fragments, including but not limited to, antibody dependent cell-mediated cytotoxicity (ADCC) and complement dependent cytotoxicity (CDC). These "defucosylated" antibodies may be produced through a variety of systems utilizing molecular cloning techniques known in the art, including but not limited to, transgenic animals, transgenic plants, or cell-lines that have been genetically engineered so that they no longer contain the enzymes and biochemical pathways necessary for the inclusion of a fucose in the complex N-glycoside-linked sugar chains (also known as fucosyltransferase knock-out animals, plants, or cells). Non-limiting examples of cells that can be engineered to be fucosyltransferase knock-out cells include CHO cells, SP2/0 cells, NS0 cells, and YB2/0 cells.

Glycosylation of immunoglobulins in the variable (V) region has also been observed. Sox and Hood reported that about 20% of human antibodies are glycosylated in the V region (Proc. Natl. Acad. Sci. USA 66:975 (1970)). Glycosylation of the V domain is believed to arise from fortuitous occurrences of the N-linked glycosylation signal Asn-Xaa-Ser/Thr in the V region sequence and has not been recognized in the art as playing a role in immunoglobulin function.

Glycosylation at a variable domain framework residue can alter the binding interaction of the antibody with antigen. The present invention includes criteria by which a limited number of amino acids in the framework or CDRs of a chimeric immunoglobulin chain are chosen to be mutated (e.g., by substitution, deletion, or addition of residues) in order to increase the affinity of an antibody.

Affinity for binding an antigen can, generally, be modulated by introducing one or more mutations into the V region framework, typically in areas adjacent to one or more CDRs and/or in one or more framework regions. Typically, such mutations involve the introduction of conservative amino acid substitutions that either destroy or create the glycosylation site sequences but do not substantially affect the hydropathic structural properties of the polypeptide. Typically, mutations that introduce a proline residue are avoided. Glycosylation of antibodies is further described in U.S. Patent No. 6,350,861.

Antibodies can be formulated for short-term delivery or extended (long term) delivery.

Antibodies that bind to endoglin can also be used for purification of endoglin and/or to detect endoglin levels in a sample or patient to detect or diagnose a disease or disorder associated with endoglin as described in more detail below.

Chimeric antibodies which bind endoglin generated using such methods can be tested for one or more of their binding affinity, avidity, and neutralizing capabilities. Useful chimeric antibodies can be used to administer a patient to prevent, inhibit, manage or treat a condition disease or disorder associated with angiogenesis.

Antibodies can be evaluated for one or more of binding affinity, association rates, disassociation rates and avidity. In one aspect, antibodies can be evaluated for their ability to neutralize the activity of endoglin or VEGF. Measurement binding affinity, association rates, disassociation rates and avidity can be accomplished using art-recognized assays including, but not limited to, an enzyme-linked-immunosorbent assay (ELISA), Scatchard Analysis, BIACORE analysis (Surface Plasmon Resonance), etc., as well as other assays commonly used and known to those of ordinary skill in the art.

Measurement of binding of antibodies to endoglin and/or the ability of the antibodies, for example, to inhibit angiogenesis, can be determined using, for example, an enzyme-linked-immunosorbent assay (ELISA), a competitive binding assay, an ELISPOT assay, or any other useful assay known in the art. These assays are commonly used and well-known to those of ordinary skill in the art.

In one non-limiting embodiment, an ELISA assay can be used to measure the binding capability of specific antibodies that bind to endoglin.

Assays, such as an ELISA, also can be used to identify antibodies thereof which exhibit increased specificity for endoglin in comparison to other antibodies thereof. Assays, such as an ELISA, also can be used to identify antibodies thereof with bind to epitopes across one or more polypeptides and across one or more species of endoglin or VEGF. The specificity assay can be conducted by running parallel ELISAs in which a test antibodies is screened concurrently in separate assay chambers for the ability to bind one or more epitopes on different species of the polypeptide containing the endoglin epitopes to identify antibodies thereof that bind to endoglin. Another technique for measuring apparent binding affinity familiar to those of skill in the art is a surface plasmon resonance technique (analyzed on a BIACORE 2000 system) (Liljeblad, et al., Glyco. J. 2000, 17:323-329). Standard measurements and traditional binding assays are described by Heeley, R. P., Endocr. Res. 2002,28:217-229.

Chimeric antibodies to endoglin can also be assayed for their ability to treat various diseases and conditions associated with angiogenesis in connection with various forms of cancer (e.g., primary tumors, recurring tumors, and metastases). Any suitable assay known to one of skill in the art can be used to monitor such effects. Several such techniques are described herein. In one example, the antibodies described herein are assayed for their ability to bind endoglin. In another example, affinity constants for the antibodies described herein are determined by surface plasmon resonance (SPR). In yet another example, the antibodies described herein are assayed for their effect on the inhibition of angiogenesis.

### II. Compositions

Each of the compounds described herein can be used as a composition when combined with an acceptable carrier or excipient. Such compositions are useful for *in vitro* or *in vivo* analysis or for administration to a subject *in vivo* or *ex vivo* for treating a subject with the disclosed compounds.

Provided herein are compositions (medicaments) containing a chimeric anti-endoglin antibody capable of inhibiting one or more of the biological activities of endoglin, such as angiogenic activity.

Provided herein are compositions (medicaments) containing an anti-VEGF antibody (or antigen-binding fragment thereof) capable of inhibiting one or more of the biological activities of VEGF, such as its mitogenic activity in one embodiment, or angiogenic activity.

Also provided herein are compositions (medicaments) containing a combination of a chimeric anti-endoglin antibody and an anti-VEGF antibody (or antigen-binding fragment thereof).

Compositions containing a chimeric anti-endoglin antibody can be administered sequentially or simultaneously with a composition containing an anti-VEGF antibody (or antigen-binding fragment thereof). Such administrations include, but are not limited to, administration within about four weeks of each other, within about three weeks of each other, within about two weeks of each other, within about a week of each other, within a day of each other, within about 12 hours of each other, within about 6 hours of each other, within about 3 hours of each other, within about 1 hour of each other, within about 30 minutes of each other, on the same day, at the same time, or a combination thereof. When multiple doses of the composition of the present invention and/or the combined therapeutic moiety are contemplated, it is understood that doses of each can be empirically determined using known doses and concentrations based on the age, height, weight, health and other physical characteristics of a subject using standards of commercially available products.

When compositions are administered sequentially, a composition comprising a chimeric anti-endoglin antibody described herein can be, for example, administered prior to and/or after an anti-VEGF antibody (or antigen-binding fragment thereof). Alternatively, a composition comprising a chimeric anti-endoglin antibody described herein is administered after an anti-VEGF antibody (or antigen-binding fragment thereof).

When compositions are administered simultaneously, a composition containing a chimeric anti-endoglin antibody can be administered at the same site, or at a different site than a composition containing an anti-VEGF antibody.

In yet another embodiment, provided herein are compositions (medicaments) containing a chimeric anti-endoglin antibody and an anti-VEGF antibody (or antigen-binding fragment thereof), capable of inhibiting one or more of the biological activities of endoglin and VEGF, respectively, such as mitogenic activity, cell proliferation, tumor growth, neovascularization, or angiogenic activity.

One would understand that treatment regimens may include one or more administrations of each of the compositions described herein. A composition can be administered in a single dose or multiple doses. Administration of separate compositions may be by the same route or by different routes.

In one embodiment, a composition is administered every one to three weeks for six to 12 cycles or until tumor progression. The method can further include the step of administering a composition every one to twelve weeks for up to two years. In another non-limiting example, the concurrent administration of a chimeric anti-endoglin antibody and an anti-VEGF antibody (or antigen-binding fragment thereof) occurs at week 1, followed by additional administration of the compositions at week one, two, three or four, wherein the concurrent administration is repeated for six to twelve cycles or until tumor progression and followed by administration of the compositions every one to twelve weeks for up to two years.

In one example of treatment for cancer in a patient described herein, the treatment includes surgical removal of the cancer and administration of a chimeric anti-endoglin antibody and an anti-VEGF antibody (or antigen-binding fragment thereof) at one to three weeks for 12 months or until tumor progression followed by concurrent administration of an anti-endoglin antibody and an anti-VEGF antibody (or antigen-binding fragment thereof) in a dose at one to 12 weeks. Additionally, the concurrent administration of a chimeric anti-endoglin antibody and an anti-VEGF antibody (or antigen-binding fragment thereof) can be repeated every one to three weeks for up to 6 cycles. Optionally, the method further includes administering a chimeric anti-endoglin antibody and an anti-VEGF antibody (or antigen-binding fragment thereof) every one to twelve weeks for up to two years. It will be understood that treatment regimens can be combined with monitoring methods provided herein to determine if and when additional doses of a chimeric anti-endoglin antibody and an anti-VEGF antibody (or antigen-binding fragment thereof) need be administered.

Combination therapy may provide a synergistic and/or beneficial effect or may allow lower doses of a combination to provide a greater margin of safety. The invention encompasses treatment protocols that enhance the prophylactic or therapeutic effect of a chimeric anti-endoglin antibody and an anti-VEGF (or antigen-binding fragment thereof) for preventing, managing, treating or ablation of cancer or other diseases.

In one embodiment, an additional therapeutic treatment such as, for example, an angiogenesis inhibitor (as described herein) is administered to a subject. The composition containing such an additional therapeutic treatment can be administered in combination (either sequentially or simultaneously) with the other compositions described herein.

In one treatment for cancer provided herein, an additional therapeutic treatment includes surgical removal of the cancer, irradiation, one or more chemotherapeutic agents, or a combination thereof, and concurrent administration of one or more compositions described herein. In one aspect, administration of a composition can be, for example, a 20 minute intravenous infusion.

Thus compositions can include, in addition to active ingredient, a pharmaceutically acceptable excipient, carrier, buffer, stabilizer or other materials well known to those in the art. Such materials should be non-toxic and should not interfere with the efficacy of the active ingredient(s). The precise nature of the carrier or other material will depend on the route of administration.

Formulations comprising an antibody or antigen-binding fragment, identified by the methods described herein can be prepared for storage by mixing the protein having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are those that are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN®, PLURONICS® or polyethylene glycol (PEG).

Acceptable carriers are physiologically acceptable to the administered patient and retain the therapeutic properties of the compounds with/in which it is administered. Acceptable carriers and their formulations are and generally described in, for example, Remington' pharmaceutical Sciences (18th Edition, ed. A. Gennaro, Mack Publishing Co., Easton, PA 1990). One exemplary carrier is physiological saline. The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject compounds from the administration site of one organ, or portion of the body, to another organ, or portion of the body, or in an *in vitro* assay system. Each carrier is acceptable in the sense of being compatible with the other ingredients of the formulation and not injurious to a subject to whom it is administered. Nor should an acceptable carrier alter the specific activity of the subject compounds.

In one aspect, provided herein are pharmaceutically acceptable or physiologically acceptable compositions including solvents (aqueous or non-aqueous), solutions, emulsions, dispersion media, coatings, isotonic and absorption promoting or delaying agents, compatible with administration. Compositions or formulations, therefore, refer to a composition suitable for therapeutic and/or diagnostic use in a subject. Compositions and formulations include an amount of a compound described herein and a pharmaceutically or physiologically acceptable carrier.

Compositions can be formulated to be compatible with a particular route of administration (i.e., systemic or local). Thus, compositions include carriers, diluents, or excipients suitable for administration by various routes.

In another embodiment, the compositions can further comprise, if needed, an acceptable additive in order to improve the stability of the compounds in composition and/or to control the release rate of the composition. Acceptable additives do not alter the specific activity of the subject compounds. Exemplary acceptable additives include, but are not limited to, a sugar such as mannitol, sorbitol, glucose, xylitol, trehalose, sorbose, sucrose, galactose, dextran, dextrose, fructose, lactose and mixtures thereof. Acceptable additives can be combined with acceptable carriers and/or excipients such as dextrose. Alternatively, exemplary acceptable additives include, but are not limited to, a surfactant such as polysorbate 20 or polysorbate 80 to increase stability of the peptide and decrease gelling of the solution. The surfactant can be added to the composition in an amount of 0.01% to 5% of the solution. Addition of such acceptable additives increases the stability and half-life of the composition in storage.

Compositions can be administered, for example, by injection, including, but not limited to, subcutaneous, intravitreal, intradermal, intravenous, intra-arterial, intraperitoneal, or intramuscular injection. Excipients and carriers for use in formulation of compositions for each type of injection are contemplated herein. The following descriptions are by example only and are not meant to limit the scope of the compositions. Compositions for injection include, for example, aqueous solutions (where water soluble) or dispersions, and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). The carrier can be a solvent or dispersion medium containing, for example, water, ethanol, polyol (for example, glycerol, propylene glycol, and liquid polyetheylene glycol, and the like), and suitable mixtures thereof. Fluidity can be maintained, for example, by the use of a coating such as lecithin, by the maintenance of the required particle size in the case of dispersion and by the use of surfactants. Antibacterial and antifungal agents include, for example, parabens, chlorobutanol, phenol, ascorbic acid and thimerosal. Isotonic agents, for example, sugars, polyalcohols such as manitol, sorbitol, and sodium chloride may be included in the composition. The resulting solutions can be packaged for use as is, or lyophilized; the lyophilized preparation can later be combined with a sterile solution prior to administration. For intravenous, injection, or injection at the site of affliction, the active ingredient can be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be included, as needed. Sterile injectable solutions can be prepared by incorporating an active ingredient in the required amount in an appropriate solvent with one or a combination of ingredients enumerated above, as required, followed by filtered sterilization. Generally, dispersions are prepared by incorporating the active ingredient into a sterile vehicle which contains a basic dispersion medium and the required other ingredients from those enumerated above. In the case of sterile powders for the preparation of sterile injectable solutions, the methods of preparation are vacuum drying and freeze drying which yields a powder of the active ingredient plus any additional desired ingredient from a previously sterile-filtered solution thereof.

Compositions can be conventionally administered intravitreally, sub-cutaneous, or via intravitreal implant.

Compositions can be conventionally administered intravenously, such as by injection of a unit dose, for example. For injection, an active ingredient can be in the form of a parenterally acceptable aqueous solution which is substantially pyrogen-free and has suitable pH, isotonicity and stability. One can prepare suitable solutions using, for example, isotonic vehicles such as Sodium Chloride Injection, Ringer's Injection, Lactated Ringer's Injection. Preservatives, stabilizers, buffers, antioxidants and/or other additives may be included, as required. Additionally, compositions can be administered via aerosolization. (Lahn et al., Aerosolized Anti-T-cell-Receptor Antibodies Are Effective against Airway Inflammation and Hyperreactivity, Int. Arch. Allegery Immuno., 134: 49-55 (2004)).

In one embodiment, the composition is lyophilized, for example, to increase shelf-life in storage. When the compositions are considered for use in medicaments or any of the methods provided herein, it is contemplated that the composition can be substantially free of pyrogens such that the composition will not cause an inflammatory reaction or an unsafe allergic reaction when administered to a human patient. Testing compositions for pyrogens and preparing compositions substantially free of pyrogens are well understood to one or ordinary skill of the art and can be accomplished using commercially available kits.

Acceptable carriers can contain a compound that stabilizes a composition, increases or delays absorption, or increases or delays clearance. Such compounds include, for example, carbohydrates, such as glucose, sucrose, or dextrans; low molecular weight proteins; compositions that reduce the clearance or hydrolysis of peptides; or excipients or other stabilizers and/or buffers. Agents that delay absorption include, for example, aluminum monostearate and gelatin. Detergents can also be used to stabilize or to increase or decrease the absorption of compositions, including liposomal carriers. To protect from digestion the compound can be complexed with a composition to render it resistant to acidic and enzymatic hydrolysis, or the compound can be complexed in an appropriately resistant carrier such as a liposome. Means of protecting compounds from digestion are known in the art (see, e.g., Fix (1996) Pharm Res. 13:1760 1764; Samanen (1996) J. Pharm. Pharmacol. 48:119 135; and U.S. Pat. No. 5,391,377, describing lipid compositions for oral delivery of therapeutic agents).

The phrase "pharmaceutically acceptable" refers to molecular entities and compositions that are physiologically tolerable and do not typically produce an allergic or similar untoward reaction, such as gastric upset, dizziness and the like, when administered to a subject.

The term "unit dose" when used in reference to a therapeutic composition refers to physically distinct units suitable as unitary dosage for subjects, each unit containing a predetermined quantity of active material calculated to produce the desired therapeutic effect in association with the required diluent; i.e., carrier, or vehicle.

The compositions can be administered in a manner compatible with the dosage formulation, and in a therapeutically effective amount. The quantity to be administered depends on the subject to be treated, capacity of the subject's immune system to utilize the active ingredient, and degree of binding capacity desired. Precise amounts of active ingredient required to be administered depend on the judgment of the practitioner and are peculiar to each individual. Suitable regimes for initial administration and booster shots are also variable, but are typified by an initial administration followed by repeated doses at one or more hour intervals by a subsequent injection or other administration. Alternatively, continuous intravenous infusions sufficient to maintain concentrations in the blood are contemplated.

One embodiment contemplates the use of the compositions described herein to make a medicament for treating a condition, disease or disorder described herein. Medicaments can be formulated based on the physical characteristics of the patient/subject needing treatment, and can be formulated in single or multiple formulations based on the stage of the condition, disease or disorder. Medicaments can be packaged in a suitable package with appropriate labels for the distribution to hospitals and clinics wherein the label is for the indication of treating a subject having a disease described herein. Medicaments can be packaged as a single or multiple units. Instructions for the dosage and administration of the compositions can be included with the packages as described below. The present application is directed to medicaments of a chimeric anti-endoglin antibody described herein above and a pharmaceutically acceptable carrier. In another embodiment, the present application is directed to medicaments of an anti-VEGF antibody (or antigen-binding fragment thereof) described herein above and a pharmaceutically acceptable carrier. In yet another embodiment, the present application is directed to medicaments of a chimeric anti-endoglin antibody and an anti-VEGF antibody (or antigen-binding fragment thereof) described herein above and a pharmaceutically acceptable carrier.

In one embodiment of the present invention, the compositions are formulated to be free of pyrogens such that they are acceptable for administration to human patients. Testing compositions for pyrogens and preparing compositions free of pyrogens are well understood to one of ordinary skill in the art.

One embodiment of the present invention contemplates the use of any of the compositions of the present invention to make a medicament for treating a disorder of the present invention. Medicaments can be formulated based on the physical characteristics of the patient/subject needing treatment, and can be formulated in single or multiple formulations based on the disorder. Medicaments can be packaged in a suitable package with appropriate labels for the distribution to hospitals and clinics wherein the label is for the indication of treating a disorder as described herein in a subject. Medicaments can be packaged as a single or multiple units. Instructions for the dosage and administration of the compositions can be included with the packages.

### III. Methods of Use

Provided herein are compositions comprising a chimeric antibody that preferentially binds to endoglin and an antagonistic anti-VEGF antibody for use in treating a subject (human or non-human). In certain instances, the uses include administering a single composition containing both a chimeric anti-endoglin antibody and an anti-VEGF antibody to a subject. In other instances, the uses include administering a single composition containing both a chimeric anti-endoglin antibody and bevacizumab to a subject. In other instances, the uses include administering a single composition containing both a chimeric anti-endoglin antibody and ranibizumab to a subject.

An effective response of the present invention is achieved when the subject experiences partial or total alleviation or reduction of signs or symptoms of illness, and specifically includes, without limitation, prolongation of survival. The expected progression-free survival times may be measured in months to years, depending on prognostic factors including the number of relapses, stage of disease, and other factors. Prolonging survival includes without limitation times of at least 1 month (mo), about at least 2 mos., about at least 3 mos., about at least 4 mos., about at least 6 mos., about at least 1 year, about at least 2 years, about at least 3 years, about at least 4 years, about at least 5 years, etc. Overall or progression-free survival can be also measured in months to years. Alternatively, an effective response may be that a subject's symptoms or cancer burden remain static and do not worsen. Further indications of treatment of indications are described in more detail below.

Compositions of antibodies described herein can be used as non-therapeutic agents (e.g., as affinity purification agents). Generally, in one such embodiment, a protein of interest is immobilized on a solid phase such a Sephadex resin or filter paper, using conventional methods known in the art. The immobilized protein is contacted with a sample containing the target of interest (or fragment thereof) to be purified, and thereafter the support is washed with a suitable solvent that will remove substantially all the material in the sample except the target protein, which is bound to the immobilized antibody. Finally, the support is washed with another suitable solvent, such as glycine buffer, pH 5.0, which will release the target protein. In addition to purification, compositions can be used for detection, diagnosis and therapy of diseases and disorders associated with endoglin, VEGF and angiogenesis.

The term "contacting" as used herein refers to adding together a solution or composition of a compound with a liquid medium bathing the polypeptides, cells, tissue or organ from an organism. Alternately, "contacting" refers to mixing together a solution or composition of a compound, with a liquid such as blood, serum, or plasma derived from an organism. For *in vitro* applications, a composition can also comprise another component, such as dimethyl sulfoxide (DMSO). DMSO facilitates the uptake of the compounds or solubility of the compounds. The solution comprising the test compound may be added to the medium bathing the cells, tissues, or organs, or mixed with another liquid such as blood, by utilizing a delivery apparatus, such as a pipette-based device or syringe-based device. For *in vivo* applications, contacting can occur, for example, via administration of a composition to a patient by any suitable means; compositions with pharmaceutically acceptable excipients and carriers have been described in more detail above.

A "subject" or "patient" (e.g., a mammal such as a human or a non-human animal such as a primate, rodent, cow, horse, pig, sheep, camel, llama, etc.) can be a mammal who exhibits one or more clinical manifestations and/or symptoms of a disease or disorder described herein. In certain situations, a subject may be asymptomatic and yet still have clinical manifestations of the disease or disorder. An antibody can be conjugated to a therapeutic moiety or be a fusion protein containing a therapeutic moiety. An antibody can be conjugated to a detectable moiety or be a fusion protein containing a detectable moiety. In one embodiment, the antibody can be conjugated to both a therapeutic moiety and a detectable moiety. An antibody can be conjugated to, or recombinantly engineered with, an affinity tag (e.g., a purification tag). Affinity tags such as, for example, His6 tags, avidin, etc. are conventional in the art.

Antibodies or thereof provided herein are such that they can be conjugated or linked to a therapeutic moiety and/or an imaging or a detectable moiety and/or an affinity tag. Methods for conjugating or linking polypeptides are well known in the art. Associations (binding) between compounds and labels include any means known in the art including, but not limited to, covalent and non-covalent interactions, chemical conjugation as well as recombinant techniques.

"Angiogenesis" is used herein to include all aspects of blood vessel maintenance and development. Thus, angiogenesis includes the formation of new capillary blood vessels (whether *de novo* or from preexisting vessels) leading to neovascularization as well as the maintenance and control of the existing vasculature and small blood vessels. Angiogenesis is a complex process which includes a series of sequential steps including endothelial cell-mediated degradation of vascular basement membrane and interstitial matrices, migration of endothelial cells, proliferation of endothelial cells, and formation of capillary loops by endothelial cells. Angiogenesis is inclusive of the growth and/or development of new blood vessels (also referred to as neovascularization), dilation of the small vessels, excessive or prolonged vascular growth, and maintenance of the existing vasculature.

The term "angiogenesis-associated disease" is used herein, for purposes of the specification and claims, to mean certain pathological processes in humans where angiogenesis is abnormally prolonged. This further includes angiogenesis conditions and diseases, such as those diseases and conditions related to, caused by, or associated with angiogenesis. Non-limiting examples of such diseases include various forms of cancers and metastases, macular degeneration, CNV, diabetic retinopathy, or proliferative vitreoretinopathy. The antibodies described herein can be used to treat an angiogenesis-associated disease by binding endoglin and inhibiting angiogenesis.

The term "anti-angiogenic therapy" is used herein to mean therapy targeted to cells and/or vasculature expressing endoglin (expressed at higher levels on proliferating vasculature as compared to quiescent vasculature); this further includes therapy that is directed against angiogenesis (i.e., the formation of new capillary blood vessels leading to neovascularization), therapy that is directed against existing vasculature and/or excessive vascularization or blood vessel growth, therapy directed towards the dilation of small vessels, and therapy directed to a disease or condition (e.g., vascular targeting therapy). Exemplary diseases or conditions contemplated within the invention include, but are not limited to, various forms of cancer and metastases.

The terms "recurrence," "relapse" or "relapsed" refer to the return of a cancer or disease after clinical assessment of the disappearance of disease. A diagnosis of distant metastasis or local recurrence can be considered a relapse.

The term "maintenance therapy" refers to scheduled retreatment that is given to help maintain a previous treatment's effects. Maintenance therapy is often given to help keep cancer in remission or prolong a response to a specific therapy regardless of disease progression.

The term "progression-free survival" in oncology refers to the length of time during and after treatment that a cancer does not grow. Progression-free survival includes the amount of time patients have experienced a complete response or a partial response, as well as the amount of time patients have experienced stable disease.

In one aspect, provided herein are compositions of the invention for use in preventing or treating a cancer or a metastasis in a subject. Such a patient can be symptomatic or asymptomatic.

In some cases, administration of the composition prolongs life of the patient being treated, reduces tumor volume, eliminates a tumor, decreases cell proliferation, increases apoptosis of tumor cells, or a combination thereof.

If needed, the uses can further include surgical removal of the cancer and/or administration of an additional anti-cancer agent or treatment. Anti-cancer agents have been provided elsewhere herein.

In one aspect, symptoms of the patient suffering from cancer are ameliorated. Amelioration can be manifested as, for example, reduction in pain, reduced tumor size, elimination of tumors, prevention of increases in tumor size or progression or of disease, prevention of formation of metastasis, or inhibition of metastatic growth, or a combination thereof.

In one aspect, administration of the compositions reduces or eliminates the need for the patient to undergo surgery or treatment with one or more additional anti-cancer agents or treatments.

### Treatment with Chimeric Anti-Endoglin Antibodies and Anti-VEGF Agents

Provided herein are compositions for use in preventing or treating one or more diseases or disorders associated with angiogenesis/neovascularization, excessive vascularization, tumor growth, tumor cell proliferation or small vessel dilation, the compositions containing a chimeric anti-endoglin antibody and a composition containing an anti-VEGF antibody. They may for adminsistration at the same time or at different times, thereby preventing, treating, ameliorating, or lessening the disease or its severity.

As used herein, "prevention" refers to prophylaxis, prevention of onset of symptoms, prevention of progression of a disease or disorder associated with angiogenesis or correlated with endoglin activity. As used herein, "inhibition," "treatment" and "treating" are used interchangeably and refer to, for example, stasis of symptoms, prolongation of survival, partial or full amelioration of symptoms, and partial or full eradication of a tumor or metastases.

Compositions can be administered to a patient in a therapeutically effective amount that are effective for producing some desired therapeutic effect by inhibiting a disease or disorder at a reasonable benefit/risk ratio applicable to any medical treatment. For the administration of the present compositions to human patients, the compositions can be formulated by methodology known by one in the art. A therapeutically effective amount is an amount achieves at least partially a desired therapeutic or prophylactic effect in an organ or tissue. The amount of a chimeric anti-endoglin antibody or an anti-VEGF antibody necessary to bring about prevention and/or therapeutic treatment of a disease or disorder is not fixed *per se.* The amount of antibody administered may vary with the type of disease, extensiveness of the disease, and size of the mammal suffering from the disease or disorder. In one embodiment, two antibodies described herein are administered to a patient in combination as described above. Administration in combination can refer to administration in a single composition or in separate compositions.

"administering" is referred to herein as providing one or more compositions to a patient in a manner that results in the composition being inside the patient's body. Such an administration can be by any route including, without limitation, locally, regionally or systemically by subcutaneous, intravitreal, intradermal, intravenous, intra-arterial, intraperitoneal, or intramuscular administration (e.g., injection).

Actual dosage levels of the active ingredients in the compositions can be varied so as to obtain an amount of the active ingredient that is effective to achieve the desired therapeutic response for a particular patient, composition, and mode of administration, without being toxic to the patient. The selected dosage level will depend upon a variety of factors including the activity of the particular compound employed, the route of administration, the time of administration, the rate of excretion of the particular compound being employed, the duration of the treatment, other drugs, compounds and/or materials used in combination with the particular composition employed, the age, sex, weight, condition, general health and prior medical history of the patient being treated, and like factors well known in the medical arts.

The antibodies described herein can be administered to a subject in various dosing amounts and over various time frames. Non-limiting doses include about 0.01 mg/kg, about 0.05 mg/kg, about 0.1 mg/kg, about 0.5 mg/kg, about 1 mg/kg, about 5 mg/kg, about 10 mg/kg, about 20 mg/kg, about 30 mg/kg, or any integer in between. Additionally, the dose(s) of an antibody can be administered twice a week, weekly, every two weeks, every three weeks, every 4 weeks, every 6 weeks, every 8 weeks, every 12 weeks, or any combination of weeks therein. Dosing cycles are also contemplated such as, for example, administering antibodies one or twice a week for 2, 3, 4, 5 or 6 weeks, followed by 1, 2, 3, 4, 5, or 6 weeks without therapy. Alternatively, depending upon the response of a subject to therapy, cycling time between treatments can be 1, 2, 3, 4, 5, 6, 7, 8, 9, 10, 11 or 12 months. Additional dosing cycles including, for example, different combinations of the doses and weekly cycles described herein are also contemplated within the invention.

"Contacting" is defined herein as a means of bringing a composition as provided herein in physical proximity with a cell, organ, tissue or fluid as described herein. Contacting encompasses systemic or local administration of any of the compositions provided herein and includes, without limitation, *in vitro, in vivo* and/or *ex vivo* procedures and methods. "Combining" and "contacting" are used interchangeably herein and are meant to be defined in the same way.

A physician or veterinarian can readily determine and prescribe the effective amount (ED50) of the composition required. For example, the physician or veterinarian could start doses of the compounds employed in the composition at levels lower than that required in order to achieve the desired therapeutic effect and gradually increase the dosage until the desired effect is achieved. Alternatively, a dose can remain constant.

Compositions can be administered to a patient by any convenient route such as described above. Regardless of the route of administration selected, the compounds of the present invention, which can be used in a suitable hydrated form, and/or the compositions, are formulated into acceptable dosage forms such as described below or by other conventional methods known to those in the art.

Toxicity and therapeutic efficacy of compounds can be determined by standard procedures in cell cultures or experimental animals, e.g., for determining the LD50 (the dose lethal to 50% of the population) and the ED₅₀ (the dose therapeutically effective in 50% of the population). The dose ratio between toxic and therapeutic effects is the therapeutic index and it can be expressed as the ratio LD50/ED50. While compounds that exhibit toxic side effects may be used, care should be taken to design a delivery system that targets such compounds to the site of affected tissue in order to minimize potential damage to healthy cells and, thereby, reduce side effects.

Data obtained from cell culture assays and/or animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound, a therapeutically effective dose can be estimated initially from cell culture assays. A dose can be formulated in animal models to achieve a circulating plasma concentration arrange that includes the IC50 (i.e., the concentration of the test compound which achieves a half-maximal inhibition) as determined in cell culture. Levels in plasma can be measured, for example, by high performance liquid chromatography. Such information can be used to more accurately determine useful doses in humans. Compositions containing combinations of compounds can also be assessed using any of these methods.

In one embodiment, the invention contemplates inhibition of angiogenesis in a tissue. The extent of angiogenesis in a tissue and, therefore, the extent of inhibition achieved can be evaluated by a variety of methods, such as are described herein.

The unique specificity of the antibodies which recognize (e.g., preferentially bind) endoglin or VEGF and inhibits angiogenesis, provides diagnostic and therapeutic uses for diseases characterized by angiogenesis (neovascularization), small vessel dilation, excessive vascularization, tumor cell proliferation, and/or tumor growth. Antibodies can be administered to a subject suffering from various forms of cancer (primary tumors and metastases).

One would understand that, in addition to administration of the compositions described herein, it is contemplated herein that a subject can also be treated with one or more additional angiogenesis inhibitors.

The term "angiogenesis inhibitor" is used herein, for purposes of the specification and claims, to mean a compound or molecule including, but not limited to, peptides, proteins, enzymes, polysaccharides, oligonucleotides, DNA, RNA, recombinant vectors, and drugs which function to inhibit angiogenesis. Angiogenesis inhibitors are known in the art and all types are contemplated herein. Non-limiting examples of compounds and molecules include natural and synthetic biomolecules such as paclitaxel, O-(chloroacetyl-carbomyl) fumagillol ("TNP-470" or "AGM 1470"), thrombospondin-1, thrombospondin-2, angiostatin, human chondrocyte-derived inhibitor of angiogenesis ("hCHIAMP"), cartilage-derived angiogenic inhibitor, platelet factor-4, gro-beta, human interferon-inducible protein 10 ("IP10"), interleukin 12, Ro 318220, tricyclodecan-9-yl xanthate ("D609"), irsogladine, 8,9-dihydroxy-7-methyl-benzo[b] quinolizinium bromide ("GPA 1734"), medroxyprogesterone, a combination of heparin and cortisone, glucosidase inhibitors, genistein, thalidomide, diamino-antraquinone, herbimycin, ursolic acid, and oleanolic acid. Non-limiting examples of antibodies include those directed towards molecules such as VEGF, VEGF receptor, or different epitopes of endoglin. Additionally, small molecular inhibitors of VEGF receptor are known and contemplated herein. Non-limiting examples of VEGF receptor inhibitors include ranibizumab (Lucentis), aflibercept (VEGF-Trap), sunitinib (Sutent), sorafenib (Nexavar), axitinib, pegaptanib and pazopanib.

One example of a VEGF receptor inhibitor is ranibizumab. Exemplary ocular dosages for ranibizumab include about 0.5 mg, administered intravitreally monthly. Another example of a VEGF receptor inhibitor is VEGF-Trap. Exemplary dosages for VEGF-Trap include about 0.5 - about 10 mg/kg administered every 2 or 3 weeks. Exemplary ocular dosages for VEGF-Trap include about 0.5 - about 2.0 mg administered intravitreally monthly or quarterly.

Exemplary regimens for sunitinib include about 50 mg administered for 4 weeks, followed by 2 weeks of no drug. Treatment regimens can be repeated in a cyclic or acyclic basis. Exemplary dosages for sorafenib include about 400 mg administered daily. Exemplary dosages for axitinib include about 3, about 5, or about 10 mg administered twice daily. Exemplary dosages for pegaptanib include about 0.3- about 3 mg administered intravitreally every 6 weeks. Exemplary dosages for pazopanib include about 200 - about 1000 mg administered daily.

Multiple combinations of these VEGF receptor inhibitors can be administered with the compositions described herein. In one embodiment, combinations may result in the use of lower doses for the described antibodies or antigen binding. Such alterations in dosing may result from synergistic effects of the combinations of the antibodies.

### Cancer

CD105 is associated with tumor angiogenesis and is strongly up-regulated in the endothelium of various tumor tissues compared with that in normal tissues. CD105 is up-regulated in a wide range of tumor endothelia. Additionally, there is stronger expression of CD105 in tumor endothelium than corresponding normal tissues. Thus, the inhibition of angiogenesis with chimeric anti-endoglin antibodies represents a treatment option for cancerous tumors. The compositions described herein can be used to treat cancerous tumors and metastases. The compositions can also be used in the formulations of medicaments for the treatment cancerous tumors and metastases.

VEGF represents one target for antitumor therapies because its expression is upregulated in a range of solid tumors. VEGF is a major regulator of angiogenesis, the growth of new vessels from pre-existing vessels. This process is fundamental to the growth of solid tumors, which rely on the formation of new blood vessels. Certain small molecule therapeutic agents are able to target vascular endothelial growth factor receptor ("VEGFR"); such targeting by small molecule therapeutics can result in anti-cancer effects. VEGF receptor-targeted agents indirectly block tumor growth, through the inhibition of new vessel formation. Inhibiting VEGF-induced angiogenesis can exert an anti-tumor or improved anti-tumor effect without significantly inhibiting VEGF stimulation of macrophages, osteoclasts or chondroclasts.

The term "tumor" is used herein to refer to a cancerous tissue expressing endoglin and/or VEGF (as compared to expression by normal tissue of the same type). Tumors can include solid tumors and semi-solid tumors. Non-limiting examples of tumors include human leukemias, including non-T-cell-type (non-T) acute lymphoblastic leukemia (ALL), myelo-monocytic leukemia; and human solid and semi-solid tumors, with its surrounding vasculature expressing endoglin at moderate to high levels (as compared to expression by normal tissue of the same type) including angiosarcoma, breast carcinoma, stomach cancer, colon carcinoma, Hodgkins lymphoma, lymphoma, glioblastoma multiforme (GBM), lung carcinoma, melanoma, myeloma, lymphoma, osteosarcoma, ovarian carcinoma, parotid tumor, pharyngeal carcinoma, prostate carcinoma, hepatocellular carcinoma, renal carcinoma, and rectosigmoid carcinoma.

A cancerous tissue to be treated is, for example, an endothelial tissue expressing an abnormal level of endoglin and/or VEGF.

In the absence of neovascularization of tumor tissue, the tumor tissue does not obtain the required nutrients, slows in growth, ceases additional growth, regresses and ultimately becomes necrotic resulting in killing of the tumor. Provided herein are methods of inhibiting tumor neovascularization by inhibiting tumor angiogenesis. Similarly, provided herein are methods of inhibiting tumor growth.

The methods are also particularly effective against the formation of metastases because their formation requires vascularization of a primary tumor so that the metastatic cancer cells can exit the primary tumor and their establishment in a secondary site requires neovascularization to support growth of the metastases.

It will be appreciated that a "subject suffering from a cancer/metastasis" of the invention may express a mutant protein (tumor associated antigen) or a mutant gene and not yet be symptomatic for the disease. In one non-limiting example of colon cancer (which is associated with the mutant K-ras protein), a subject with a mutant K-ras protein in some cells of the colon is a subject to be treated even though that subject may not yet be symptomatic for colon cancer. "Signs or symptoms of illness" represent clinically recognized manifestations or indications of disease.

By "treating" a subject suffering from tumor or metastasis, it is meant that the subject's symptoms are partially alleviated, totally alleviated, or remain static following treatment. A patient that has been treated can exhibit a partial or total alleviation of tumor load. This is intended to encompass prophylaxis, therapy and cure. In one non-limiting example, a subject suffering from a highly metastatic cancer (e.g., breast cancer) is treated where additional metastasis either do not occur, or are reduced in number as compared to a subject who does not receive treatment. In another non-limiting example, a subject is treated where the subject's solid cancer either becomes reduced in size or does not increase in size as compared to a subject who does not receive treatment. In yet another non-limiting example, the number of cancer cells in a treated subject either does not increase or is reduced as compared to the number of cancer cells in a subject who does not receive treatment. Improvement can also be defined, for example, as decreased cell proliferation, decreased numbers of cells, increased apoptosis, and/or increased survival of the subject being treated.

As further used herein, treatment of cancer includes stasis, partial or total elimination of a cancerous growth or tumor. Treatment or partial elimination includes, for example, a fold reduction in growth or tumor size and/or volume such as about 2-fold, about 3-fold, about 4-fold, about 5-fold, about 10-fold, about 20-fold, about 50-fold, or any fold reduction in between. Similarly, treatment or partial elimination can include a percent reduction in growth or tumor size and/or volume of about 1%, 2%, 3%, 4%, 5%, 10%, 20%, 30%, 40%, 50%, 60%, 70%, 80%, 90%, 95% or any percentage reduction in between.

A tumor or cancer to be treated in the methods described herein includes, but is not limited to, a lung cancer, a gynecologic malignancy, a melanoma, a breast cancer, a brain cancer (e.g., glioblastoma multiforme, "GBM" or a glioma) a pancreatic cancer, an ovarian cancer, a uterine cancer, a colorectal cancer, a prostate cancer, a kidney cancer, a head cancer, a liver cancer (hepatocellular cancer), a neck cancer, a kidney cancer (renal cell cancer), an penile cancer, a stomach cancer, a thyroid cancer, a bladder cancer, a sarcoma, a carcinoma, a myeloma, and lymphoma. In one embodiment, a tumor to be treated is a solid or semi-solid tumor. In another embodiment, a tumor to be treated is a primary tumor. In another embodiment, a tumor to be treated is a metastatic tumor. In one embodiment, a tumor or cancer to be treated is of epithelial origin. In another embodiment, the cancer to be treated is myeloma. In another embodiment, the cancer to be treated is ovarian cancer. In another embodiment, the cancer to be treated is kidney/renal cancer. In yet another embodiment, the cancer to be treated is hepatocellular/liver cancer.

### Lung cancer

In one aspect, provided herein are compositions for use in treating lung cancer. The most common type of lung cancer is non-small cell lung cancer (NSCLC), which accounts for approximately 80-85% of lung cancers and is divided into squamous cell carcinomas, adenocarcinomas, and large cell undifferentiated carcinomas. Small cell lung cancer accounts for 15-20% of lung cancers.

Lung cancer staging is an assessment of the degree of spread of the cancer from its original source. It is an important factor affecting the prognosis and potential treatment of lung cancer. Non-small cell lung carcinoma is staged from IA ("one A"; best prognosis) to IV ("four"; worst prognosis). Small cell lung carcinoma is classified as limited stage if it is confined to one half of the chest and within the scope of a single radiotherapy field; otherwise, it is extensive stage.

Non-small cell lung cancer may be staged using EUS (endoscopic ultrasound) or CT or MRI scan or at surgery to classify the extent of disease according to the TNM system. These subjects undergo staging as part of the process of considering prognosis and treatment. The AJCC recommends TNM staging followed by further grouping.

Primary tumor (T): TX: The primary tumor cannot be assessed, or there are malignant cells in the sputum or bronchoalveolar lavage but not seen on imaging or bronchoscopy; Tis: Carcinoma in situ. T0: No evidence of primary tumor. T1: Tumor less than 3 cm in its greatest dimension, surrounded by lung or visceral pleura and without bronchoscopic invasion into the main bronchus. T2: A tumor with any of: more than 3 cm in greatest dimension; extending into the main bronchus (but more than 2 cm distal to the carina), and obstructive pneumonitis (but not involving the entire lung). T3: A tumor with any of: invasion of the chest wall, diaphragm, mediastinal pleura, or parietal pericardium; extending into the main bronchus, within 2 cm of the carina, but not involving the carina; and obstructive pneumonitis of the entire lung. T4: A tumor with any of: invasion of the mediastinum, heart, great vessels, trachea, esophagus, vertebra, or carina; separate tumor nodules in the same lobe; and malignant pleural effusion. Lymph nodes (N): NX: Lymph nodes cannot be assessed; N0: No lymph nodes involved; N1: Metastasis to ipsilateral peribronchial or ipsilateral hilar lymph nodes; N2: Metastasis to ipsilateral mediastinal or subcarinal lymph nodes; and N3: Metastasis to any of: ipsilateral supraclavicular lymph nodes; ipsilateral scalene lymph nodes; and contralateral lymph nodes. Distant metastasis (M): MX: Distant metastasis cannot be assessed; M0: No distant metastasis; and M1: Distant metastasis is present.

### Uterine cancers / Gynecologic Malignancy

Uterine cancers may refer to any of several different types of cancer which occur in the uterus, namely: uterine sarcomas (e.g., sarcomas of the myometrium, or muscular layer of the uterus, are most commonly leiomyosarcomas); endometrial cancer; and cervical cancer.

In another aspect, provided herein are compositions for use in treating endometrium cancer. Endometrial cancer is a cancer that starts in the endometrium, the inner lining of the uterus. Some of the examples of the cancer of uterus and endometrium include, but are not limited to, adenocarcinomas, adenoacanthomas, adenosquamous carcinomas, papillary serous adenocarcinomas, clear cell adenocarcinomas, uterine sarcomas, stromal sarcomas, malignant mixed mesodermal tumors, and leiomyosarcomas.

In another aspect, the compositions are for use in treating cervical cancer, preferably an adenocarcinoma in the cervix epithelial. Two main types of this cancer exist: squamous cell carcinoma and adenocarcinomas. The former constitutes about 80-90% of all cervical cancers and develops where the ectocervix (portion closest to the vagina) and the endocervix (portion closest to the uterus) join. The latter develop in the mucous-producing gland cells of the endocervix. Some cervical cancers have characteristics of both of these and are called adenosquamous carcinomas or mixed carcinomas.

### Ovarian cancer

In another aspect, provided herein are compositions for use in treating ovarian cancer, including epithelial ovarian tumors.

Ovarian cancer is classified according to the histology of the tumor, obtained in a pathology report. Surface epithelial-stromal tumor, also known as ovarian epithelial carcinoma, is the most typical type of ovarian cancer. It includes serous tumor, endometrioid tumor and mucinous cystadenocarcinoma. Sex cord-stromal tumor, including estrogen-producing granulosa cell tumor and virilizing Sertoli-Leydig cell tumor or arrhenoblastoma, accounts for 8% of ovarian cancers. Germ cell tumor accounts for approximately 30% of ovarian tumors but only 5% of ovarian cancers because most germ cell tumors are teratomas and most teratomas are benign. Germ cell tumor tends to occur in young women and girls. The prognosis depends on the specific histology of germ cell tumor, but overall is favorable. Mixed tumors contain elements of more than one of the above classes of tumor histology.

Ovarian cancer can also be a secondary cancer, the result of metastasis from a primary cancer elsewhere in the body. Common primary cancers are breast cancer and gastrointestinal cancer (in which case the ovarian cancer is a Krukenberg cancer). Surface epithelial-stromal tumor can originate in the peritoneum (the lining of the abdominal cavity), in which case the ovarian cancer is secondary to primary peritoneal cancer, but treatment is basically the same as for primary surface epithelial-stromal tumor involving the peritoneum.

Ovarian cancer staging is by the FIGO staging system and uses information obtained after surgery, which can include a total abdominal hysterectomy, removal of both ovaries and fallopian tubes, the omentum, and pelvic (peritoneal) washings for cytology. The AJCC stage is the same as the FIGO stage.

Stage I refers to ovarian cancer limited to one or both ovaries: IA - involves one ovary; capsule intact; no tumor on ovarian surface; no malignant cells in ascites or peritoneal washings; IB - involves both ovaries; capsule intact; no tumor on ovarian surface; negative washings; and IC - tumor limited to ovaries with any of the following: capsule ruptured, tumor on ovarian surface, positive washings.

Stage II refers to pelvic extension or implants: IIA - extension or implants onto uterus or fallopian tube; negative washings; IIB - extension or implants onto other pelvic structures; negative washings; and IIC - pelvic extension or implants with positive peritoneal washings

Stage III refers to microscopic peritoneal implants outside of the pelvis; or limited to the pelvis with extension to the small bowel or omentum: IIIA - microscopic peritoneal metastases beyond pelvis; IIIB - macroscopic peritoneal metastases beyond pelvis less than 2 cm in size; and IIIC - peritoneal metastases beyond pelvis > 2 cm or lymph node metastases

Stage IV refers to distant metastases to the liver or outside the peritoneal cavity.

Para-aortic lymph node metastases are considered regional lymph nodes (Stage IIIC).

In some embodiments, the compositions described herein treat an ovarian cancer selected from the following: an adenocarcinoma in the ovary and an adenocarcinoma that has migrated from the ovary into the abdominal cavity.

### Melanoma

A melanoma is a malignant tumor of melanocytes which are found predominantly in skin but also in the bowel and the eye (uveal melanoma). It is one of the rarer types of skin cancer but causes the majority of skin cancer related deaths. Malignant melanoma is a serious type of skin cancer caused by uncontrolled growth of pigment cells, called melanocytes. Melanomas also include, but are not limited to, a choroidea melanoma, malignant melanomas, cutaneous melanomas and intraocular melanomas.

Melanoma may be divided into the following types: Lentigo maligna, Lentigo maligna melanoma, superficially spreading melanoma, acral lentiginous melanoma, mucosal melanoma, nodular melanoma, polypoid melanoma, desmoplastic melanoma, amelanotic melanoma, soft-tissue melanoma, and uveal melanoma. Melanoma stages are as follows:
Stage 0 - melanoma in situ (Clark Level I).
Stage I/II - invasive melanoma: T1a: less than 1.00 mm primary, without ulceration, Clark Level II-III; T1b: less than 1.00 mm primary, with ulceration or Clark Level IV-V; and T2a: 1.00-2.00 mm primary, without ulceration.
Stage II - High Risk Melanoma: T2b: 1.00-2.00 mm primary, with ulceration; T3a: 2.00-4.00 mm primary, without ulceration; T3b: 2.00-4.00 mm primary, with ulceration; T4a: 4.00 mm or greater primary without ulceration; and T4b: 4.00 mm or greater primary with ulceration.
Stage III - Regional Metastasis: N1: single positive lymph node; N2: 2-3 positive lymph nodes or regional skin/in-transit metastasis; and N3: 4 positive lymph nodes or lymph node and regional skin/in transit metastases.
Stage IV - Distant Metastasis: M1a: Distant Skin Metastasis, Normal LDH; M1b: Lung Metastasis, Normal LDH; and M1c: Other Distant Metastasis OR Any Distant Metastasis with Elevated LDH.

In one embodiment, the compositions described herein are for use in treating a melanoma

### Colon Cancer and Colorectal Cancer

Colorectal cancer (also called colon cancer or large bowel cancer) includes cancerous growths in the colon, rectum (anus) and appendix. With 655,000 deaths worldwide per year, it is the third most common form of cancer and the second leading cause of cancer-related death in the Western world. Many colorectal cancers are thought to arise from adenomatous polyps in the colon. These mushroom-like growths are usually benign, but some may develop into cancer over time.

In another embodiment, Dukes classification may be used to classify colorectal cancer based on stages A-D. Stage A refers to colorectal cancer that is limited to mucosa (i.e., has not invaded through the bowel wall). Stage B1 refers to extending into muscularis propria, but not penetrating through it (i.e., lymph nodes have not been invaded); whereas Stage B2 cancer has penetrated through the muscularis propria, but not penetrating through it (i.e., lymph nodes have not been invaded). Stage C1 refers to cancer that extends into the muscularis propria, but not penetrating through it (i.e., lymph nodes are involved); whereas Stage C2 refers to cancer that extends into the muscularis propria and penetrating through it (i.e., lymph nodes are involved). Stage D refers to distant metastatic spread. The TNM system may also be used to stage colorectal cancer according to conventional means known in the art.

### Breast cancer

Several types of breast cancer exist that may be treated by the methods described herein. A lobular carcinoma *in situ* and a ductal carcinoma *in situ* are breast cancers that have developed in the lobules and ducts, respectively, but have not spread to the fatty tissue surrounding the breast or to other areas of the body. Infiltrating (or invasive) lobular and ductal carcinoma are cancers that have developed in the lobules and ducts, respectively, and have spread to either the breast's fatty tissue and/or other parts of the body. In one aspect, provided herein are compositions for use in treating breast cancer, such as a ductal carcinoma in duct tissue in a mammary gland, a breast cancer that is Her2- and/or ER- and/or PR-.Other cancers of the breast that would benefit from treatment by the methods are medullary carcinomas, colloid carcinomas, tubular carcinomas, and inflammatory breast cancer.

In one embodiment, breast cancer is staged according to the TNM system. Prognosis is closely linked to results of staging, and staging is also used to allocate patients to treatments both in clinical trials and clinical practice.

Briefly, the information for staging is as follows: **TX:** Primary tumor cannot be assessed. T0: No evidence of tumor. Tis: Carcinoma in situ, no invasion; T1: Tumor is 2 cm or less; T2: Tumor is more than 2 cm but not more than 5 cm; T3: Tumor is more than 5 cm; T4: Tumor of any size growing into the chest wall or skin, or inflammatory breast cancer. **NX:** Nearby lymph nodes cannot be assessed N0: cancer has not spread to regional lymph nodes. N1: cancer has spread to 1 to 3 maxillary or one internal mammary lymph node N2: cancer has spread to 4 to 9 maxillary lymph nodes or multiple internal mammary lymph nodes N3: One of the following applies: cancer has spread to 10 or more maxillary lymph nodes, or cancer has spread to the lymph nodes under the clavicle (collar bone), or cancer has spread to the lymph nodes above the clavicle, or cancer involves maxillary lymph nodes and has enlarged the internal mammary lymph nodes, or cancer involves 4 or more maxillary lymph nodes, and tiny amounts of cancer are found in internal mammary lymph nodes on sentinel lymph node biopsy. **MX:** presence of distant spread (metastasis) cannot be assessed. M0: no distant spread. M1: spread to distant organs (not including the supraclavicular lymph node) has occurred.

### Pancreatic cancer

In another aspect, provided herein are compositions for use in treating pancreatic cancer selected from the following: an epitheliod carcinoma in the pancreatic duct tissue and an adenocarcinoma in a pancreatic duct. The most common type of pancreatic cancer is an adenocarcinoma, which occurs in the lining of the pancreatic duct.

### Prostate Cancer

In one other aspect, provided herein are compositions for use in treating prostate cancer selected from the following: an adenocarcinoma or an adenocarcinoma that has migrated to the bone. Prostate cancer develops in the prostate organ in men, which surrounds the first part of the urethra. The prostate has several cell types but 99% of tumors are adenocarcinomas that develop in the glandular cells responsible for generating seminal fluid.

There are two schemes commonly used to stage prostate cancer. The most common is the TNM system, which evaluates the size of the tumor, the extent of involved lymph nodes, and any metastasis (distant spread). As with many other cancers, these are often grouped into four stages (I-IV). Another scheme, used less commonly, is the Whitmore-Jewett stage.

Briefly, Stage I disease is cancer that is found incidentally in a small part of the sample when prostate tissue was removed for other reasons, such as benign prostatic hypertrophy, and the cells closely resemble normal cells and the gland feels normal to the examining finger. In Stage II more of the prostate is involved and a lump can be felt within the gland. In Stage III, the tumor has spread through the prostatic capsule and the lump can be felt on the surface of the gland. In Stage IV disease, the tumor has invaded nearby structures, or has spread to lymph nodes or other organs. Grading is based on cellular content and tissue architecture from biopsies (Gleason) which provides an estimate of the destructive potential and ultimate prognosis of the disease.

### Head and Neck Cancers

Head and neck cancers (e.g., oral, laryngeal, nasopharyngeal, esophageal, etc.), refer to a group of biologically similar cancers originating from the upper aerodigestive tract, including the lip, oral cavity (mouth), nasal cavity, paranasal sinuses, pharynx, and larynx. Most head and neck cancers are squamous cell carcinomas, originating from the mucosal lining (epithelium) of these regions. Head and neck cancers often spread to the lymph nodes of the neck, and this is often the first (and sometimes only) manifestation of the disease at the time of diagnosis. Head and neck cancer is strongly associated with certain environmental and lifestyle risk factors, including tobacco smoking, alcohol consumption, and certain strains of the sexually transmitted human papillomavirus. Management of patients with head and neck cancers remains a formidable task. Cancers such as, hypopharyngeal cancer, laryngeal cancer, nasopharyngeal cancer, oropharyngeal cancer, may be treated using the compounds described herein.

In one embodiment, the compositions described herein are for use in treating a head or neck cancer.

### Kidney cancer

In another aspect, provided herein are compositions for use in treating kidney cancer. Kidney cancer (also called renal cell cancer, renal cell carcinoma, renal adenocarcinoma, and hypernephroma) is a disease in which malignant cells are found in the lining of tubules in the kidney. Renal cell carcinoma is the most common form of kidney cancer arising from the proximal renal tubule. It is the most common type of kidney cancer in adults, responsible for approximately 80% of cases.

### Liver Cancer

In another aspect, provided herein are compositions for use in treating primary liver cancer (cancer that begins in the liver). Primary liver cancer can occur in both adults and children. Liver cancer is characterized by the presence of malignant hepatic tumors - tumors or growths on or in the liver. They may be discovered on medical imaging (even for a different reason than the cancer itself), or may be present in patients as an abdominal mass, abdominal pain, jaundice, or some other liver dysfunction. There are several types of liver cancer.

Hemangiomas: These are the most common type of benign liver tumor. They start in blood vessels. Most of these tumors do not cause symptoms, they do not need treatment. Some may bleed and need to be removed if it is mild to severe.

Hepatic adenomas: These benign epithelial liver tumors develop in the liver. They are, in most cases, located in the right hepatic lobe and are frequently seen as solitary. The size of adenomas range from 1 to 30 cm. Symptoms associated with hepatic adenomas are all associated with large lesions which can cause intense abdominal pain.

Focal nodular hyperplasia: Focal nodular hyperplasia (FNH) is the second most common tumor of the liver. This tumor is the result of a congenital arteriovenous malformation hepatocyte response. This process is one in which all normal constituents of the liver are present, but the pattern by which they are presented is abnormal. Even though those conditions exist the liver still seems to perform in the normal range.

Hepatocellular Cancer: Hepatocellular cancer (HCC) is the most common cancer of the liver. It is associated with alcohol abuse and hepatitis B infection and is particularly prevalent in Asia. The majority of HCC is detected at a time when cure by surgical resection is not possible; systemic treatment of un-resectable HCC is associated with survival of less than one year.

In one embodiment, the compositions described herein are for use in treating a liver cancer.

### Lymphoma

Lymphoma is a type of cancer that originates in lymphocytes of the immune system. They often originate in lymph nodes, presenting as an enlargement of the node (a tumor). Lymphomas are closely related to lymphoid leukemias, which also originate in lymphocytes but typically involve only circulating blood and the bone marrow (where blood cells are generated in a process termed haematopoesis) and do not usually form tumors. There are many types of lymphomas, and in turn, lymphomas are a part of the broad group of diseases called hematological neoplasms. Some forms of lymphoma are indolent (e.g. small lymphocytic lymphoma), compatible with a long life even without treatment, whereas other forms are aggressive (e.g. Burkitt's lymphoma), causing rapid deterioration and death.

The WHO Classification, published in 2001 and updated in 2008; http://en.wikipedia.org/wiki/Lymphoma - cite_note-isbn92-832-2411-6-2#cite_note-isbn92-832-2411-6-2 is the latest classification of lymphoma and is based upon the foundations laid within the "Revised European-American Lymphoma classification" (REAL). This system groups lymphomas by cell type (i.e., the normal cell type that most resembles the tumor) and defining phenotypic, molecular or cytogenetic characteristics. There are three large groups: the B cell, T cell, and natural killer cell tumors. Other less common groups are also recognized. Hodgkin's lymphoma, although considered separately within the WHO (and preceding) classifications, is now recognized as being a tumor of, albeit markedly abnormal, lymphocytes of mature B cell lineage.

In one embodiment, the compositions described herein are for use in treating a lymphoma.

### Sarcoma

A sarcoma is a cancer of the connective tissue (bone, cartilage, fat) resulting in mesoderm proliferation.

This is in contrast to carcinomas, which are of epithelial origin (breast, colon, pancreas, and others). However, due to an evolving understanding of tissue origin, the term "sarcoma" is sometimes applied to tumors now known to arise from epithelial tissue. The term soft tissue sarcoma is used to describe tumors of soft tissue, which includes elements that are in connective tissue, but not derived from it (such as muscles and blood vessels).

Sarcomas are given a number of different names, based on the type of tissue from which they arise. For example, osteosarcoma arises from bone, chondrosarcoma arises from cartilage, and leiomyosarcoma arises from smooth muscle. Sarcomas strike people in all age ranges, but they are very rare, accounting for only 1% of all cases of cancer. GIST is the most common form of sarcoma, with approximately 3000-3500 cases per year in the United States. This should be compared with breast cancer, with approximately 200,000 cases per year in North America.

Approximately 50% of bone sarcomas and 20% of soft tissue sarcomas are diagnosed in people under the age of 35. Some sarcomas, such as leiomyosarcoma, chondrosarcoma, and gastrointestinal stromal tumor (GIST), are more common in adults than in children. Most high grade bone sarcomas, including Ewing's sarcoma and osteosarcoma, are much more common in children and young adults.

In one embodiment, the compositions described herein are for use in treating a sarcoma.

### Carcinoma

A carcinoma is any malignant cancer that arises from epithelial cells. Carcinomas invade surrounding tissues and organs and may metastasize, or spread, to lymph nodes and other sites.

Carcinoma, like all neoplasia, is classified by its histopathological appearance. Adenocarcinoma and squamous cell carcinoma, two common descriptive terms for tumors, reflect the fact that these cells may have glandular or squamous cell appearances respectively. Severely anaplastic tumors might be so undifferentiated that they do not have a distinct histological appearance (undifferentiated carcinoma).

Sometimes a tumor is referred to by the presumptive organ of the primary (e.g., carcinoma of the prostate) or the putative cell of origin (hepatocellular carcinoma, renal cell carcinoma).

Adenocarcinoma is a malignant tumor originating in the epithelial cells of glandular tissue and forming glandular structures. This is common in the lung (forming 30-40% of all lung carcinomas). It is found peripherally, arising from goblet cells or type II pneumocytes.

Squamous cell carcinoma results from squamous metaplasia. This accounts for 20-30 percent of lung tumors and is usually hilar in origin.

Small cell carcinoma is almost certainly due to smoking. These metastasize early, and may secrete ADH (lowering patient sodium concentration).

Large cell undifferentiated carcinomas account for 10-15 percent of lung neoplasms. These are aggressive and difficult to recognize due to the undifferentiated nature. These are most commonly central in the lung.

### Sinonasal undifferentiated carcinoma.

In one embodiment, the compositions described herein are for use in treating a carcinoma.

### Myeloma

Multiple myeloma (also known as MM, myeloma, plasma cell myeloma, or as Kahler's disease after Otto Kahler) is a cancer of plasma cells. These immune cells are formed in bone marrow, are numerous in lymphatics and produce antibodies. Myeloma is regarded as incurable, but remissions may be induced with steroids, chemotherapy, thalidomide and stem cell transplants. Myeloma is part of the broad group of diseases called hematological malignancies.

Multiple myeloma develops in post-germinal center B lymphocytes. A chromosomal translocation between the immunoglobulin heavy chain gene (on the fourteenth chromosome, locus 14q32) and an oncogene (often 11q13, 4p16.3, 6p21, 16q23 and 20q11) is frequently observed in patients with multiple myeloma. This mutation results in dysregulation of the oncogene which is thought to be an important initiating event in the pathogenesis of myeloma. The result is proliferation of a plasma cell clone and genomic instability that leads to further mutations and translocations. The chromosome 14 abnormality is observed in about 50% of all cases of myeloma. Deletion of (parts of) the thirteenth chromosome is also observed in about 50% of cases.

Production of cytokines (especially IL-6) by the plasma cells causes much of their localized damage, such as osteoporosis, and creates a microenvironment in which the malignant cells thrive. Angiogenesis (the attraction of new blood vessels) is increased.

In one embodiment, the compositions described herein are for use in treating a myeloma.

### Stomach cancer

Stomach or gastric cancer can develop in any part of the stomach and may spread throughout the stomach and to other organs; particularly the esophagus, lungs and the liver. Stomach cancer causes about 800.000 deaths worldwide per year.

Metastasis occurs in 80-90% of individuals with stomach cancer, with a six month survival rate of 65% in those diagnosed in early stages and less than 15% of those diagnosed in late stages.

Stomach cancer is often asymptomatic or causes only nonspecific symptoms in its early stages. By the time symptoms occur, the cancer has generally metastasized to other parts of the body, one of the main reasons for its poor prognosis.

In one embodiment, the compositions described herein are for use in treating a stomach cancer.

### Thyroid cancer

Thyroid neoplasm or thyroid cancer usually refers to any of four kinds of malignant tumors of the thyroid gland: papillary, follicular, medullary or anaplastic. Papillary and follicular tumors are the most common. They grow slowly and may recur, but are generally not fatal in patients under 45 years of age. Medullary tumors have a good prognosis if restricted to the thyroid gland and a poorer prognosis if metastasis occurs. Anaplastic tumors are fast-growing and respond poorly to therapy.

Thyroid cancer is usually found in a euthyroid patient, but symptoms of hyperthyroidism or hypothyroidism may be associated with a large or metastatic well-differentiated tumor. Nodules are of particular concern when they are found in those under the age of 20. The presentation of benign nodules at this age is less likely, and thus the potential for malignancy is far greater.

Thyroid cancers can be classified according to their pathological characteristics. The following variants can be distinguished (distribution over various subtypes may show regional variation): papillary thyroid cancer (up to 75%); follicular thyroid cancer (up to 15%); medullary thyroid cancer (up to 8%); and anaplastic thyroid cancer (less than 5%). The follicular and papillary types together can be classified as "differentiated thyroid cancer". These types have a more favorable prognosis than the medullary and undifferentiated types. Thyroid adenoma is a benign neoplasm of the thyroid.

In one embodiment, the compositions described herein are for use in treating a thyroid cancer.

### Bladder cancer

Bladder cancer refers to any of several types of malignant growths of the urinary bladder. It is a disease in which abnormal cells multiply without control in the bladder. The bladder is a hollow, muscular organ that stores urine; it is located in the pelvis. The most common type of bladder cancer begins in cells lining the inside of the bladder and is called transitional cell carcinoma (sometimes urothelial cell carcinoma).

90% of bladder cancers are transitional cell carcinoma. The other 10% are squamous cell carcinoma, adenocarcinoma, sarcoma, small cell carcinoma and secondary deposits from cancers elsewhere in the body.

The following stages are used to classify the location, size, and spread of the cancer, according to the TNM (tumor, lymph node, and metastasis) staging system: Stage 0: Cancer cells are found only on the inner lining of the bladder. Stage I: Cancer cells have proliferated to the layer beyond the inner lining of the urinary bladder but not to the muscles of the urinary bladder. Stage II: Cancer cells have proliferated to the muscles in the bladder wall but not to the fatty tissue that surrounds the urinary bladder. Stage III: Cancer cells have proliferated to the fatty tissue surrounding the urinary bladder and to the prostate gland, vagina, or uterus, but not to the lymph nodes or other organs. Stage IV: Cancer cells have proliferated to the lymph nodes, pelvic or abdominal wall, and/or other organs. Recurrent: Cancer has recurred in the urinary bladder or in another nearby organ after having been treated.

Bladder TCC is staged according to the 1997 TNM system: Ta Non-invasive papillary tumor; T1 Invasive but not as far as the muscular bladder layer; T2 Invasive into the muscular layer; T3 Invasive beyond the muscle into the fat outside the bladder; and T4 Invasive into surrounding structures like the prostate, uterus or pelvic wall.

In one embodiment, the compositions described herein are for use in treating a bladder cancer.

### Ocular Conditions Involving Angiogenesis

### Macular Degeneration Conditions and Diabetic Retinopathy

In one aspect, the present invention provides compositions for use in treating diabetic retinopathy, macular degeneration, choroidal neovascularization or neovascular glaucoma in a patient.

Macular degeneration (AMD) is the loss of photoreceptors in the portion of the central retina, termed the macula, responsible for high-acuity vision. Degeneration of the macula is associated with abnormal deposition of extracellular matrix components and other debris in the membrane between the retinal pigment epithelium and the vascular choroid. This debris-like material is termed drusen. Drusen is observed with a funduscopic eye examination. Normal eyes may have maculas free of drusen, yet drusen may be abundant in the retinal periphery. The presence of soft drusen in the macula, in the absence of any loss of macular vision, is considered an early stage of AMD. Macular degeneration is characterized by choroidal neovascularization (CNV), the development of abnormal blood vessels beneath the retinal pigment epithelium (RPE) layer of the retina. These vessels break through the Bruch's membrane, disrupting the retinal pigmented epithelium, bleed, and eventually cause macular scarring which results in profound loss of central vision (disciform scarring).

Choroidal neovascularization (CNV) commonly occurs in macular degeneration in addition to other ocular disorders and is associated with proliferation of choroidal endothelial cells, overproduction of extracellular matrix, and formation of a fibrovascular subretinal membrane. Retinal pigment epithelium cell proliferation and production of angiogenic factors appears to effect choroidal neovascularization.

Diabetic retinopathy (DR) is an ocular disorder characterized by excessive angiogenesis that develops in diabetes due to thickening of capillary basement membranes, and lack of contact between pericytes and endothelial cells of the capillaries. Loss of pericytes increases leakage of the capillaries and leads to breakdown of the blood-retina barrier. Diabetic retinopathy is the result of microvascular retinal changes. Hyperglycemia-induced pericyte death and thickening of the basement membrane lead to incompetence of the vascular walls. These damages change the formation of the blood-retinal barrier and also make the retinal blood vessels become more permeable. Small blood vessels - such as those in the eye - are especially vulnerable to poor blood sugar (blood glucose) control. An over-accumulation of glucose and/or fructose damages the tiny blood vessels in the retina. Macular edema can also develop when the damaged blood vessels leak fluid and lipids onto the macula. These fluids make the macula swell, which blurs vision. This damage also results in a lack of oxygen at the retina.

As the disease progresses, the lack of oxygen in the retina stimulates angiogenesis along the retina and in the clear, gel-like vitreous humour that fills the inside of the eye. Without timely treatment, these new blood vessels can bleed, cloud vision, and destroy the retina. Fibrovascular proliferation can also cause tractional retinal detachment. The new blood vessels can also grow into the angle of the anterior chamber of the eye and cause neovascular glaucoma.

Proliferative vitreoretinopathy is associated with cellular proliferation of cellular and fibrotic membranes within the vitreous membranes and on the surfaces of the retina. Retinal pigment epithelium cell proliferation and migration is common with this ocular disorder. The membranes associated with proliferative vitreoretinopathy contain extracellular matrix components such as collagen types I, II, and IV and fibronectin, and become progressively fibrotic.

Age-related macular degeneration (AMD) and diabetic retinopathy are the two leading causes of blindness in the developed world. The recent approval of the macromolecules LUCENTIS®, AVASTIN®, and MACUGEN® have improved the treatment options available for AMD patients. LUCENTIS® is a Fab and AVASTIN® is a monoclonal antibody. They both bind vascular endothelial growth factor (VEGF) and have demonstrated the most impressive results to date treating AMD; however, only a minority of treated patients experience a significant improvement in visual acuity. Anti-angiogenic therapy focused on a target other than VEGF may overcome some of the limitations associated with agents that target the VEGF pathway.

The chimeric anti-endoglin antibodies described herein can be used to treat or prevent macular degeneration, CNV, diabetic retinopathy, or proliferative vitreoretinopathy. Described herein are compositions for use in treating or preventing macular degeneration, CNV, diabetic retinopathy, or proliferative vitreoretinopathy. The chimeric anti-endoglin antibodies described herein can also shrink blood vessels, inhibit endothelial cell proliferation associated with ocular disease, clear symptoms of bleeding, treat cloudy vision, provide stasis of vision loss, and/or prevent leakage of blood vessels. The chimeric anti-endoglin antibodies described herein can also be used in medicaments for the treatment of macular degeneration, CNV, diabetic retinopathy or proliferative vitreoretinopathy.

Additionally, chimeric anti-endoglin antibodies described herein can also be used in combination with known therapies and/or compounds for the treatment of macular degeneration, CNV, diabetic retinopathy or proliferative vitreoretinopathy. Examples of such compounds include, but are not limited to, bevacizumab (AVASTIN®), ranibizumab (LUCENTIS®), VEGF-Trap, sunitinib (SUTENT®), sorafenib (NEXAVAR®), axitinib, pegaptanib, pazopanib or MACUGEN®. In addition to the modes of administration described herein, the chimeric anti-endoglin antibodies can be administered via intravitreal routes. Non-limiting examples of intravitreal modes of administration include intravitreal injection and the use of intravitreal implants.

Patients can be assessed for improvement and responsiveness to treatment. Treatment includes, but is not limited to, decreasing the macular edema, decreased areas of CNV, and increased visual acuity. Measurements of symptoms are as known in the art and are further described in the examples below.

### Chronic Inflammatory Diseases

Any of a variety of tissues or organs comprised of organized tissues, can support angiogenesis in disease conditions including skin, muscle, gut, connective tissue, joints, bones and the like tissue in which blood vessels can invade upon angiogenic stimuli. Thus, a tissue to be treated may be an inflamed tissue and the angiogenesis to be inhibited may be inflamed tissue angiogenesis where there is neovascularization of inflamed tissue.

### Inflammatory Bowel Diseases

Angiogenesis plays an important role in inflammatory bowel disease (IBD). IBD is an umbrella term for a set of bowel and intestinal diseases or conditions including Crohn's disease and ulcerative colitis. Crohn's disease is typically characterized by inflammation of the small and large bowel, whereas ulcerative colitis is generally localized to the colon. Abnormal or pathological angiogenesis is central to both Crohn's disease and ulcerative colitis. Both diseases involve increased microvascular density and microvascular dysfunction, and this angiogenesis is temporally related with tissue pathology and the inflammatory cycles found in both diseases. Endoglin is known to be expressed in these tissues and to play a role in the dysregulation of angiogenesis during IBD. (Chidlow et al., Am. J. Physiol. Gastrointest. Liver Physiol., 293:5-18 (2007)).

The chimeric anti-endoglin antibodies described herein can be used to treat IBD. Additionally, chimeric anti-endoglin antibodies can be used for the treatment of Crohn's disease or ulcerative colitis. The chimeric anti-endoglin antibodies can also be used in combination with surgery and/or known therapies for IBD, Crohn's disease or ulcerative colitis. Examples of such known therapies include, but are not limited to, Aminosalicylates (e.g., Mesalamine), corticosteroids (e.g., budesonide, prednisone, etc.), antibiotics (e.g., metronidizole, etc.), immunosuppresive drugs (e.g., azathioprine, 6-mercaptopurine, methotrexate, Tacrolimus, and cyclosporine, etc.), and biologic drugs such as proteins and antibodies (e.g., infliximab, etc.).

Treatment of IBD can be assessed by decreased vascularization of the inflamed tissue. Treatment can also be assessed by stasis, resolution, and/or healing of the ulcerative lesions which characterize IBD.

### Diabetic Nephropathy & Renal Transplantation Ischemia

Diabetic nephropathy is a major cause of morbidity and mortality in both type 1 and type 2 diabetics. It is the leading cause of end-stage renal disease world-wide. Diabetic nephropathy is characterized by glomerular microvascular injury due to the increased synthesis of pro-angiogenic factors. These pro-angiogenic factors cause increased endothelial cell proliferation and subsequent angiogenesis, and endoglin is known to be upregulated in chronic renal disease. This angiogenesis results in destruction of the glomeruli and finally renal failure.

Similar effects are seen in renal transplantation resulting in ischemia and failure of the transplanted organ. The upregulation of endoglin results in upregulated angiogenesis and inflammation in the kidney. Conversely, studies with endoglin null mice show significantly reduced renal damage after transplantation/ischemia and increased organ survival.

The chimeric anti-endoglin antibodies described herein can be used to treat or prevent diabetic nephropathy, renal failure following transplantation, and/or ischemic renal injury following transplantation.

Described herein are methods of treating or preventing diabetic nephropathy, renal failure following transplantation, and/or ischemic renal injury following transplantation via the administration of the antibodies described herein. The chimeric anti-endoglin antibodies described herein can also be used in medicaments for the treatment of diabetic nephropathy, renal failure following transplantation, and/or ischemic renal injury following transplantation. Additionally, chimeric anti-endoglin antibodies described herein can also be used in combination with known therapies and/or compounds for the treatment of diabetic nephropathy, renal failure following transplantation, and/or ischemic renal injury following transplantation.

Patients can be assessed with respect to the efficacy of treatment by, for example, improvement in renal function.

### Rheumatoid Arthritis & Osteoarthritis

Rheumatoid arthritis is characterized by excessive angiogenesis, and is well understood in this regard. The inflammation and destruction found in the synovial fluids is directly related to the increased angiogenesis found surrounding and in the synovial tissues. Numerous pro-angiogenic factors are present in the affected tissues of rheumatoid arthritis patients.

Osteoarthritis is a group of chronic disabling conditions that affects the synovial joints. Angiogenesis and inflammation are integral processes in the pathophysiology of the disease, and they contribute to joint damage through a variety of mechanisms, including but not limited to, stimulation of MMP production and endochondral ossification. Additionally, angiogenesis in osteoarthritis induces further innervation, which develops into a feedback loop where each continues to stimulate the other.

The chimeric anti-endoglin antibodies described herein can be used to treat or prevent rheumatoid arthritis and osteoarthritis. Described herein are methods of treating or preventing rheumatoid arthritis and osteoarthritis via the administration of one or more of the compositions described herein. The humanized chimeric anti-endoglin antibodies described herein can also be used in medicaments for the treatment of rheumatoid arthritis and osteoarthritis.

Two well accepted composite measures of improvement of RA in trials are: the Paulus Criteria and The American College of Rheumatology Criteria (ACR). Paulus Criteria is defined as improvement in 4 of the following: tender and swollen joint counts, morning stiffness, patient assessment of disease activity, physician assessment of disease activity and erythrocyte sedimentation rate (ESR) rage. The level of improvement is set as a percentage improvement of each of these variables i.e. a Paulus 20 classification indicates a responder who has shown 20% improvement in 4 of the 6 parameters.

Rheumatoid arthritis can also be assessed using American College of *Rheumatology* (*ACR*) Scoring. Briefly, ACR Classification Criteria for Determining Clinical Remission in Rheumatoid Arthritis is assessed by the presence of 5 or more of the following factors present at least two consecutive months:
a. Morning stiffness < 15 minutes;
b. No fatigue;
c. No joint pain;
d. No joint tenderness or pain on motion;
e. No soft tissue swelling in joints or tendon sheaths; and
f. ESR (Westergren method) < 30 mm/hour for a female or 20 mm/hour for a male.

Exclusions may occur and include: clinical manifestations of active vasculitis, pericarditis, pleuritis or myositis, and unexplained recent weight loss or fever attributable to rheumatoid arthritis will prohibit a designation of complete clinical remission (Pinals RS, et.al.: Arthritis Rheum 24:1308, 1981). Additionally, ACR Classification Criteria of Functional Status in Rheumatoid Arthritis includes classification based on the following patient abilities:
Class I: Completely able to perform usual activities of daily living (self-care, vocational, and avocational);
Class II: Able to perform usual self-care and vocational activities, but limited in avocational activities;
Class III: Able to perform usual self-care activities, but limited in vocational and avocational activities; and
Class IV: Limited ability to perform usual self-care, vocational, and avocational activities.

Osteoarthritis can also be assessed using ACR scoring. ACR Clinical Classification Criteria for Osteoarthritis of the hip is assessed utilizing patient history, physical examination and laboratory findings: a patient is assessed for pain in the hip and one of the following:
(1) Internal hip rotation of less than 15 degrees and ESR less than or equal to 45 degrees mm/hour or hip flexion less than or equal to 115 degrees if ESR is unavailable; or
(2) Internal hip rotation of less than 15 degrees, pain associated with internal hip, morning stiffness of the hip for less than or equal to 60 minutes and the patient is over 50 years of age.

Using history, physical examination, laboratory and radiographic findings, traditional format is pain in the hip and two of the following indications: ESR less than 20 mm/hour, radiographic femoral and/or acetabular osteophytes, or radiographic joint space narrowing (superior, axial, and/or medial). A classification tree is as follows: pain in the hip in association with (1) radiographic femoral and/or acetabular osteophytes or (2) ESR less than or equal to 20 mm/hour and radiographic axial joint space narrowing (Altman, R, et al.: Arthritis Rheum 34:505, 1991).

### ACR Clinical Classification Criteria for Osteoarthritis of the knee

ACR Clinical Classification Criteria for Osteoarthritis of the knee is assessed using history and physical examination utilizing the following criteria: pain in the knee in connection with three (3) of the following:
(1) a patient is over 50 years of age;
(2) less than 30 minutes of morning stiffness;
(3) Crepitus on active motion;
(4) bony tenderness;
(5) bony enlargement; and
(6) no palpable warmth of synovium.

Using patient history, physical examination and radiographic findings, pain in the knee can be assessed in connection with one of the following patient characteristics: (1) a patient is over 50 years of age; (2) less than 30 minutes of morning stiffness; and (3) Crepitus on active motion and osteophytes. Using history, physical examination and laboratory findings: pain in the knee can be assessed inn connection with five (5) of the following characteristics:
(1) a patient is over 50 years of age;
(2) less than 30 minutes of morning stiffness;
(3) Crepitus on active motion;
(4) bony tenderness;
(5) bony enlargement;
(6) No palpable warmth of synovium;
(7) ESF is less than 40 mm/hour;
(8) Rheumatoid factor (RF) of less than 1:40; and
(9) Synovial Fluid (SF) signs of osteoarthritis.
See, e.g., Altman, R, et al.: Arthritis Rheum 29:1039, 1986.

ACR Clinical Classification Criteria for Osteoarthritis of the hand can be assessed as follows: pain, aching or stiffness in the hand in connection with three (3) of the following: (1) hard tissue enlargement of two or more of the following joints (second and third distal interphalangeal, the second and third proximal interphalangeal, and the first carpometacarpal joints of both hands; (2) hard tissue enlargement of two or more distal interphalangeal joints; (3) less than three swollen MCP joints and (4) deformity of at least one of the joints listed above in (1).

### Combination therapy

In accordance with the embodiments described herein, the compositions described herein can be administered alone or in combination with one or more additional active or inactive agents. When combinations are used, simultaneous or sequential administration of the chimeric endoglin antibodies and the anti-VEGF antibodies (antigen-binding fragments thereof) can be used.

Compounds can be, as needed, administered in combination with one or more additional therapeutic treatments including, but not limited to, adriamycin, cyclophosphamide, paclitaxel, pemetrexed, temozolomide, oxaliplatin, erbitux, vectibix, sorafenib, sunitinib, gefitinib, erlotinib, 5-fluorouracil (5-FU) irinotecan, topotecan, leucovorin, VELCADE®, lenalidomide, thalidomide, xeloda, taxotere and many other conventional cancer therapies described herein. As used herein, "radiation" refers to, for example, microwaves, ultraviolet (UV), infrared (IR), or alpha-, beta- or gamma-radiation. Radiation can be "focused" or locally delivered using conventional techniques to target radiation to the site of one or more tumors without radiating the entire body. One would understand that the listing of therapeutic regimens listed below represents conventional therapies, but the present invention encompasses other known therapeutic regimens which are not specifically disclosed herein.

In one embodiment, the cancer is ovarian cancer and the one or more additional therapeutic treatments is surgery, chemotherapy (e.g., doxorubicin, doxil, gemcitabine, Rubitecan, and platinum-based chemotherapeutics such as cisplatin, carboplatin and oxaliplatin), melphalan, topoisomerase I inhibitors such as topotecan and irinotecan, taxane-based therapy, hormones, radiation therapy, whole body hypothermia, isoflavone derivatives such as Phenoxodial, cytotoxic macrolides such as Epothilones, angiogenesis inhibitors such as bevacizumab, signal transduction inhibitors such as trastuzumab, gene therapy, RNAi therapy, immunotherapy, monoclonal antibodies, phosphatidylinositol-like kinase inhibitors such as rapamycin, or any combination thereof. The combination therapy of the antibodies described herein with the ovarian cancer therapies may also provide for lower doses of either therapy, or both, due to a synergistic effect from the co-administration of the therapies.

In one embodiment, the cancer is renal/kidney cancer and the one or more additional therapeutic treatments is surgery, chemotherapy, pazopanib, interferon-alpha or IL-2. In yet another embodiment, the additional agent is a VEGF receptor inhibitor. Non-limiting examples of VEGF receptor inhibitors include those described above, ranibizumab (LUCENTIS®), aflibercept (VEGF-Trap), sunitinib (SUTENT®), sorafenib (NEXAVAR®), axitinib, pegaptanib and pazopanib. The combination therapy of the antibodies described herein with the kidney cancer therapies may also provide for lower doses of either therapy, or both, due to a synergistic effect from the co-administration of the therapies.

In one embodiment, the cancer is myeloma and the one or more additional therapeutic treatments is surgery, radiotherapy, VELCADE®, lenalidomide, or thalidomide. In one embodiment the additional agent is VELCADE®. The dosages for any of these therapies are known in the art and can be adjusted with combination therapy accordingly.

In one embodiment, the cancer is prostate cancer and the one or more additional therapeutic treatments is surgery, radiotherapy (e.g., external beam or brachytherapy), hormonal deprivation (androgen suppression including with abiraterone), heat shock protein 90 (HSP90) inhibitors, chemotherapy (e.g., docetaxel, platinum-based chemotherapy such as cisplatin, carboplatin, satraplatin and oxaliplatin, taxane, estramustine), prednisone or prednisolone, cholesterol-lowering drugs such as statins, leutinizing hormone-releasing hormone (LHRH) agonists, RNAi therapy, dendritic cell-based therapies, whole tumor cells genetically modified to secrete granulocyte macrophage - colony stimulating factor (GM-CSF) (also known as GVAX), or any combination thereof. In yet another embodiment, the additional agent is a VEGF receptor inhibitor. Non-limiting examples of VEGF receptor inhibitors include ranibizumab (LUCENTIS®), aflibercept (VEGF-Trap), sunitinib (SUTENT®), sorafenib (NEXAVAR®), axitinib, pegaptanib and pazopanib.

In one embodiment, the cancer is lung cancer and the one or more additional therapeutic treatments is surgery, radiotherapy (e.g., thoracic radiotherapy, radiation therapy with charged particles, Uracil-tegafur and Platinum-based chemotherapy (e.g., cisplatin, carboplatin, oxaliplatin, etc.) and vinorebline, Erlotinib (TARCEVA®), Gefitinib (IRESSA®), anti-epidermal growth factor receptor antibodies (e.g., Cetuximab), small molecule inhibitors of tyrosine kinases, direct inhibitors of proteins involved in lung cancer cell proliferation, Aurora kinase inhibitors, laser-induced thermotherapy, RNAi therapy, whole tumor cells genetically modified to secrete granulocyte macrophage - colony stimulating factor (GM-CSF) (also known as GVAX), or any combination thereof. Additional therapeutic treatments include Taxol or pemetrexed. In yet another embodiment, the additional agent is a VEGF receptor inhibitor. Non-limiting examples of VEGF receptor inhibitors include ranibizumab (LUCENTIS®), aflibercept (VEGF-Trap), sunitinib (SUTENT®), sorafenib (NEXAVAR®), axitinib, pegaptanib and pazopanib. The dosages for any of these therapies are known in the art and can be adjusted with combination therapy accordingly.

In one embodiment, the cancer is breast cancer and the one or more additional therapeutic treatments is surgery, monoclonal antibodies (e.g., Her-2 antibodies, herceptin), adjuvant chemotherapy such as single agent chemotherapy or combination chemotherapy (e.g., anthracycline- and taxane-based polychemotherapies, taxol, or target-specific trastuzumab with or without endocrine manipulation with or without PMRT, vinorelbine), adriamycin, cyclophosphamide, xeloda, taxotere, selective estrogen receptor modulators such as Tamoxifen and Raloxifene, allosteric estrogen receptor modulators such as Trilostane, radiation (e.g., interstitial brachytherapy, Mammosite device, 3-dimensional conformal external radiation and intraoperative radiotherapy), Aromatase inhibitors that suppress total body synthesis (e.g., anastrozole, exemestane and letrozole), RNAi therapy, intravenous analogs of rapamycin that are immunosuppressive and anti-proliferative such as Temsirolimus (CCI779), or any combination thereof. A review of methods for conducting three-dimensional *in vitro* tissue culture models of breast cancer are described by Kim et al., Breast Cancer Research Treatment 85(3): 281-91 (2004). Other *in vivo* and *in vitro* models for testing cancers are known and can be used to test antibodies described herein. In yet another embodiment, the additional agent is aVEGF receptor inhibitor. Non-limiting examples of VEGF receptor inhibitors include ranibizumab (LUCENTIS®), aflibercept (VEGF-Trap), Sunitinib (SUTENT®), Sorafenib (NEXAVAR®), Axitinib, Pegaptanib and Pazopanib. The dosages for any of these therapies are known in the art and can be adjusted with combination therapy accordingly.

In one embodiment, the cancer is colon cancer and the one or more additional therapeutic treatments is surgery, radiation therapy, and chemotherapy (e.g., 5-fluorouracil (5-FU), levamisole, leucovorin or semustine (methyl CCNU)), N-[2-(dimethylamino)ethyl]acridine-4-carboxamide and other related carboxamide anticancer drugs; non-topoisomerase II inhibitors, irinotecan, liposomal topotecan, taxane class of anticancer agents (e.g., paclitaxel or docetaxel), a compound of the xanthenone acetic acid class (e.g., 5,6-dimethylanthenone-4-acetic acid PMAA), laminarin, site-selective cyclic AMP Analogs (e.g., 8-chloroadenosine 3',5'-cyclic phosphate), pyranoindole inhibitors of Cox-2, carbazole inhibitors of Cox-2, tetrahydrocarbazole inhibitors of Cox-2, indene inhibitors of Cox-2, localized inhibitors of NSAIDS (e.g., anthranilic acids, aspirin (5-acetylsalicylic acid), azodisal sodium, carboheterocyclic acids, carprofen, chlorambucil, diclophenac, fenbufen, fenclofenac, fenoprofen, flufenamic acid, flurbiprofen, fluprofen, furosemide, gold sodium thiomalate, ibuprofen, indomethacin, indoprofen, ketoprofen, lonazolac, loxoprofen, meclofenamic acid, mefanamic acid, melphalan, naproxen, penicillamine, phenylacetic acids, proprionic acids, salicylic acids, salazosulfapyridine, sulindac, tolmetin, a pyrazolone butazone propazone NSAID, meloxicam, oxicams, piroxicam, feldene, piroxicam beta cyclodextran, tenoxicam. etodolac, and oxaprozin), an inhibitor of HER-2/neu, RNAi therapy, GM-CSF, monoclonal antibodies (e.g., anti-Her-2/neu antibodies, anti-CEA antibodies, A33 (HB 8779), 100-210 (HB 11764) and 100-310 (HB 11028)), erbitux, vectibix, hormonal therapy, pyrimidineamines, camptothecin derivatives (e.g., CPT- 11), folinic acid (FA), Gemcitabine, Ara-C, platinum-based chemotherapeutics such as cisplatin, carboplatin and oxaliplatin, a cGMP-specific phosphodiesterase inhibitor, or any combination thereof. In one embodiment the additional therapeutic treatment is a combination of 5-FU, leucovorin and oxaliplatin (FOLFOX). In one embodiment, the additional therapeutic treatment is a combination of 5-FU, irinotecan and leucovorin (IFL). In one embodiment, the additional agent is eribtux. In one embodiment, the additional agent is vectibix. In yet another embodiment, the additional agent is a VEGF receptor inhibitor. Non-limiting examples of VEGF receptor inhibitors include ranibizumab (LUCENTIS®), aflibercept (VEGF-Trap), sunitinib (SUTENT®), sorafenib (NEXAVAR®), axitinib, pegaptanib and pazopanib. The dosages for any of these therapies are known in the art and can be adjusted with combination therapy accordingly.

In one embodiment, the cancer is pancreatic cancer and the one or more additional therapeutic treatment is a combination of therapeutic treatments is surgery, radiation therapy (RT), Fluorouracil (5-FU) and RT, systemic therapy, stenting, Gemcitabine (GEMZAR®), Gemcitabine and RT, Cetuximab, erlotinib (TARCEVA®), chemoradiation, or any combination thereof. In yet another embodiment, the additional agent is a VEGF receptor inhibitor. Non-limiting examples of VEGF receptor inhibitors include ranibizumab (LUCENTIS®), afliberceipt (VEGF-Trap), sunitinib (SUTENT®), sorafenib (NEXAVAR®), axitinib, pegaptanib and pazopanib.

Patients can be assessed with respect to symptoms at one or more multiple time points including prior to, during, and after treatment regimens. Treatment can result in improving the subject's condition and can be assessed by determining if one or more of the following factors has occurred: decreased tumor size, decreased cell proliferation, decreased numbers of cells, decreased neovascularization, increased apoptosis, or decreased survival of at least a portion of the cells comprising the cell proliferative disorder. One or more of these occurrences may, in some cases, result in partial or total elimination of the cancer and prolongation of survival of the patient. Alternatively, for terminal stage cancers, treatment may result in stasis of disease, better quality of life and/or prolongation of survival.

### Functional Assays

Compositions described herein can be assessed in a variety of *in vitro, in vivo* and *ex vivo* assays. Any suitable assay known to one of skill in the art can be used to monitor such effects. Several such techniques are described herein.

### Assaying for CD105 Signaling and Function

CD105 (endoglin) is a member of the TGF-β receptor family that is expressed by proliferating endothelial cells, and normal levels of CD105 are needed for endothelial cell proliferation. CD105 is strongly expressed in the angiogenic vasculature of solid tumors, is involved in angiogenesis/vascular development and is an ancillary transforming growth factor β (TGF-β) receptor. CD105 is a homodimeric cell membrane glycoprotein that is expressed on leukemia cells and endothelial cells. Two isoforms of CD105, L-endoglin (170 kDa) and S-endoglin (160 kDa), differing in the amino acid sequence of their cytoplasmic tails, have been characterized.

CD105 expression is increased by cellular hypoxia through the production of hypoxia-inducible factor-1-α (HIF-1-α) and protects hypoxic cells from apoptosis. CD105 acts to modulate signaling of multiple kinase receptor complexes of the TGF-β superfamily, including TGF-β receptors (TGF-βR), activin receptor-like kinases (ALK) and activin receptors. In the absence of CD105, activation of TGF-β receptors results in phosphorylation of SMAD proteins that inhibit endothelial cell growth. However, activation of CD105 by TGF-β modulates SMAD protein phosphorylation. The end result is release of the growth inhibitory effects of TGF-β receptor activation on endothelial cells.

Prevention of CD105 activation by an anti-CD105 antibody acts synergistically with TGF-β to suppress endothelial cell growth. TGF-β can stimulate two distinct type I receptor/SMAD signaling pathways with opposite effects in endothelial cells. The TGF-β/ALK5 signaling pathway (A) leads to inhibition of cell proliferation and migration, whereas the TGF-β/ALK1 pathway (B) induces endothelial cell proliferation and migration. CD105, an accessory TGF-β receptor, highly expressed during angiogenesis, is essential for ALK1 signaling. In the absence of CD105, TGF-β/ALK5 signaling is predominant and maintains quiescent endothelium. High CD105 expression stimulates the ALK1 pathway and indirectly inhibits ALK5 signaling, thus promoting the activation state of angiogenesis.

In one non-limiting embodiment, the chimeric antibodies can be assessed with respect to inhibiting angiogenesis and endothelial cell proliferation. Binding of chimeric anti-endoglin antibodies to HUVECs does not prevent subsequent binding of TGF-β to HUVECs. Thus, direct suppression of the endothelial cell growth by anti-endoglin antibodies represents one of the underlying mechanisms by which anti-angiogenic and tumor-suppressive effects are observed *in vivo.* In another embodiment, the chimeric antibodies can be assessed with respect to blocking angiogenesis by preventing Smad1/5/8 phosphorylation and/or signaling. CD105 participates in the promotion of angiogenesis through signaling of the TGF-β/ALK1, which in turn involves the decrease and/or blockage of the phosphorylation of Smad2/3 proteins. In yet another embodiment, the chimeric antibodies can be assessed with respect to blocking angiogenesis by enhancing Smad2/3 phosphorylation and/or signaling.

Methods and techniques to assay the blocking or inhibitory effect of the chimeric antibodies provided herein on the TGF-β/ALK1 signaling pathway and/or the phosphorylation of Smad1/5 include, but are not limited to, known molecular techniques. By way of example, western blotting with antibodies specific to any of the proteins in the TGF-β/ALK5 or TGF-β/ALK1 pathways can be used to determine the inhibitory and/or stimulatory effect of the chimeric anti-endoglin antibodies disclosed herein on the TGF-β/ALK5 or TGF-β/ALK1 pathways. Similarly, detection of mRNA or regulation of the mRNA for the proteins involved in the TGF-β/ALK5 or TGF-β/ALK1 pathways can be used to assay the inhibitory and/or stimulatory effect of the chimeric antibodies disclosed herein. Additional methods for the assaying cell signaling for the TGF-β/ALK5 or TGF-β/ALK1 pathways are known in the art and are contemplated herein.

Activity of the chimeric anti-endoglin antibodies disclosed herein can be assessed using art recognized assays by, for example, binding assays such ELISAs, competitive ELISAs, surface plasmon resonance, and effect on HUVEC cells as described in more detail below.

### Assays for VEGF Signaling and Function

Antibodies that specifically inhibit VEGF binding to the VEGF receptor VEGFR2 (KDR/Flk-1) can be assessed using competition and/or functional assays. Assays include, but are not limited to, competition assays based upon an ELISA. In competition assays, one pre-mixes or admixes VEGF with varying amounts of the test antibodies (e.g., 100-fold to 1000-fold molar excess) and determines the ability of the test antibodies to reduce VEGF binding to VEGFR2. VEGF can be pre-labeled and detected directly, or can be detected using a (secondary) anti-VEGF antibody or a secondary and tertiary antibody detection system. An ELISA format of such a competition assay is one such format, but any type of immunocompetition assay may be conducted.

VEGF binding to VEGFR2 in the presence of a completely irrelevant antibody (including non-blocking anti-VEGF monoclonal antibodies) is the control high value (100%) in such a competition assay. In a test assay, a significant reduction in VEGF binding to VEGFR2 in the presence of a test antibody is indicative of an antibody that significantly inhibits VEGF binding to the VEGF receptor VEGFR2 (KDR/Flk-1).

Another binding assay to identify and/or confirm that an antibody inhibits VEGF binding to the VEGF receptor VEGFR2 (KDR/Flk-1) is a co-precipitation assay. A co-precipitation assay tests the ability of an antibody to block the binding of VEGF to one or more receptors in solution. In such an assay, VEGF or detectably-labeled VEGF is incubated with a suitable form of the receptor.

There are many formats for conducting immunoprecipitation or co-precipitation assays. In the present case, a "suitable form of the receptor" may be a VEGFR2 receptor or the extracellular domain of the receptor. Immunoprecipitation with then require, as well as the standard reagents, the presence of an antibody against a VEGFR2 receptor or an epitope on the extracellular domain of the receptor distinct from the site to which VEGF binds.

Irrespective of the suitable receptor, the immunoprecipitation or co-precipitation assays are conducted with controls. The ability of VEGF alone to bind to the chosen receptor can be confirmed by precipitation in the absence of an anti-VEGF antibody. Parallel incubations can be conducted in the presence or absence of an antibody with known binding properties to act as a control. Assays using both a blocking control and non-blocking control antibody can be run in parallel.

Any bound immunological species are then immunoprecipitated, e.g., by incubation with an effective immunoprecipitating composition, such as a Protein A composition or Protein A sepharose beads. The precipitate is then tested for the presence of VEGF. Where the VEGF in the initial incubation was detectably-labeled VEGF, such as radio-labeled VEGF, any VEGF in the immunoprecipitates can be detected directly. Any non-labeled VEGF in the immunoprecipitates may be detected by other suitable means, e.g., by gel separation and immunodetection with an anti-VEGF antibody.

The ability of an antibody to block VEGF binding to a VEGF receptor, such as VEGFR2, in such a co-precipitation assay can be readily quantitated, although qualitative results are also valuable. Quantification can be achieved by direct measurement of labeled VEGF or, e.g., by densitometric analyses of immunodetected VEGF. Antibodies that exhibit a reproducible, i.e., consistently observed, ability to inhibit VEGF binding to VEGFR2 can thus be detected, and useful antibodies can be chosen according to the quantitative criteria outlined above.

Anti-VEGF antibodies that do not significantly inhibit VEGF binding to the VEGF receptor VEGFR1 (Flt-1) can also be readily identified by conducting co-precipitation assays as described above, but using VEGFR1 rather than VEGFR2. Therefore, anti-VEGF antibodies that inhibit VEGF binding to the VEGF receptor VEGFR2 (KDR/Flk-1) without significantly inhibiting VEGF binding to the VEGF receptor VEGFR1 (Flt-1) can also be readily identified using such methods.

Functional assays to identify and/or confirm that an antibody significantly inhibits VEGF binding to the VEGF receptor VEGFR2 (KDR/Flk-1) can also be used. These are generally related to the identification of VEGFR2 as the receptor responsible for certain defined biological responses. Although less typically used than the foregoing competition-type assays, which are conducted in cell-free systems and are most reproducible, reliable, labor-saving and cost-effective, the following assays are nonetheless useful.

For example, a VEGFR2-blocking, anti-VEGF antibody may be identified by testing for the ability to inhibit VEGF-mediated endothelial cell growth (inhibiting the mitogenic activity of VEGF). Any suitable assay may be employed using any of a variety of endothelial cells in the presence of VEGF with or without test antibodies. Duplicate assays can be run in parallel, such as those without VEGF and those with control antibodies of defined properties (both blocking and non-blocking). Endothelial cell growth may be determined and accurately quantified by any acceptable means of determining cell number, including colorimetric assays.

An antibody with an ability to inhibit VEGF-mediated endothelial cell growth will generally exhibit a consistently observed inhibition of VEGF-mediated endothelial cell growth of about 25%, about 30%, about 35%, about 40%, about 45%, about 50%, about 60%, about 70%, about 80%, about 90%, about 95% or so. Inhibition in such ranges will indicate an antibody with properties sufficient to inhibit angiogenesis in vivo. Antibodies with more significant inhibitory activity are not excluded from the invention.

Further functional assays to identify antibodies include assays to test blocking of VEGF-induced phosphorylation. Any suitable assay may be employed using any of a variety of endothelial cells that express any form of native or recombinant phosphorylatable VEGFR2. Cells are incubated with VEGF in the presence or absence of the antibody to be tested for a suitable time period. Duplicate assays can be run in parallel, such as those without VEGF and those with control antibodies of defined properties (both blocking and non-blocking).

Yet further functional assays to identify VEGFR2-blocking, anti-VEGF antibodies in accordance with the present invention are assays to test inhibition of VEGF-induced vascular permeability. Although any such assay may be used, one suitable assay is the Miles permeability assay, wherein animals such as guinea pigs are injected with a dye, such as Evan's blue dye, and the appearance of the dye in the animal skin is determined after the provision of VEGF in the presence or absence of test antibodies. Duplicate studies can be conducted in parallel, such as those without VEGF and those with control antibodies of defined properties (both blocking and non-blocking). The appearance of dye in the animal skin is typically as spots, such as blue spots, in the back of the animal, which can be photographed and measured.

### SCID / Nude Mice

One method for assaying tumor growth makes use of SCID mouse, as follows: subconfluent human M21 melanoma cells are harvested, washed, and resuspended in sterile PBS (20 x 10⁶ per mL). SCID mice are injected subcutaneously with 100 µL of M21 human melanoma cell (2 x 10⁶) suspension. Three days after tumor cell injection, mice are either untreated or treated intravenously or intraperitoneally (for example, 100 µg/mouse) with one or more control or test compositions. The mice are treated daily for 24 days. Tumor size is measured with calipers and the volume estimated using the formula V = (L x W²)/2, where V is equal to the volume, L is equal to the length, and W is equal to the width.

One method for assaying tumor growth makes use of nude mouse, as follows: MDA-MB-435 tumor cells (0.4×10⁶ cells/mouse) in 50 µl PBS are orthotopically implanted in the mammary fat pad of female nude mice (five to six weeks old). When tumors reached a mean volume of approximately 50-80 mm³, mice are randomized (at least 10/group) and intravenous or intraperitoneal treatment with one or more antibodies at 1 µg (0.05 mg/kg) per dose, 10 µg (0.5 mg/kg), 100 µg (5 mg/kg) or 200 µg (10 mg/kg), or 100 µg control antibody in 100 µl PBS, or vehicle PBS 100 µl twice per week is initiated; in some studies, an untreated group can also bee valuated. Tumor size is measured with calipers and the volume estimated using the formula V = (L x W²)/2, where V is equal to the volume, L is equal to the length, and W is equal to the width.

### BALB/c Syngeneic Mouse Models

Alternatively, BALB/c syngeneic mouse models can also be utilized to assess tumor growth and inhibition thereof by the antibodies or described herein as exemplified by, for example, Tsujie et al., Int. J. Oncology, 29: 1087-1094 (2006).

### Chimeric Mice

Another assay measures angiogenesis in a chimeric mouse:human mouse model and is referred to as the chimeric mouse assay. The assay has been described in detail by others, and further has been described herein to measure angiogenesis, neovascularization, and regression of tumor tissues. See Yan, et al. (1993) J. Clin. Invest. 91:986-996.

The chimeric mouse assay is a useful assay model for in vivo angiogenesis because the transplanted skin grafts closely resemble normal human skin histologically and neovascularization of whole tissue is occurring wherein actual human blood vessels are growing from the grafted human skin into the human tumor tissue on the surface of the grafted human skin. The origin of the neovascularization into the human graft can be demonstrated by immunohistochemical staining of the neovasculature with human-specific endothelial cell markers.

The chimeric mouse assay demonstrates regression of neovascularization based on both the amount and extent of regression of new vessel growth. Furthermore, it is easy to monitor effects on the growth of any tissue transplanted upon the grafted skin, such as a tumor tissue. Finally, the assay is useful because there is an internal control for toxicity in the assay system. The chimeric mouse is exposed to any test reagent, and therefore the health of the mouse is an indication of toxicity. Other animal models described herein and known in the art can also be utilized in the methods described herein.

### Rabbit Eye Assay

Another measure of angiogenesis is an in vivo rabbit eye model and is referred to as the rabbit eye assay. The rabbit eye assay has been described in detail by others, and has been used to measure both angiogenesis and neovascularization in the presence of angiogenic inhibitors as exemplified by D'Amato et al. (1994) Proc. Natl. Acad. Sci. USA, 91(9): 4082-4085.

The rabbit eye assay is a recognized assay model for in vivo angiogenesis because the neovascularization process, exemplified by rabbit blood vessels growing from the rim of the cornea into the cornea, is easily visualized through the naturally transparent cornea of the eye. Additionally, both the extent and the amount of stimulation or inhibition of neovascularization or regression of neovascularization can easily be monitored over time.

Finally, the rabbit is exposed to any test reagent, and therefore the health of the rabbit is an indication of toxicity of the test reagent.

Briefly, chicken chorioallantoic membrane (CAM) assays are performed and the effects on the developing vasculature are recorded at 48 hours after implantation of a 0.5% carboxymethylcellulose pellet containing one or more control or test compounds. Corneal neovascularization is induced by an implanted pellet of poly(hydroxyethyl methacrylate) (Hydron; Interferon Sciences, New Brunswick, NJ) containing 650 ng of the potent angiogenic protein basic fibroblast growth factor (bFGF) bound to sucralfate (sucrose aluminum sulfate; Bukh Meditec, Copenhagen). The addition of sucralfate to the pellet protects the bFGF from degradation and provides for its slow release, thus producing consistent aggressive angiogenesis that is more pronounced than that induced by bFGF / Hydron alone. Release of bFGF from pellets containing sucralfate/Hydron can be detected *in vitro* for up to 4 days after the pellets are formed compared to just 1 day for pellets with Hydron alone. Pellets are made by mixing 110 µl of saline containing 12 µg of recombinant bFGF (Takeda, Osaka) with 40 mg of sucralfate; this suspension is added to 80 µl of 12% (wt/vol) Hydron in ethanol. Aliquots (10 µl) of this mixture are then pipetted onto Teflon pegs and allowed to dry producing approximately 17 pellets.

A pellet is implanted into corneal micropockets of each eye of an anesthetized female New Zealand White rabbit, 2 mm from the limbus, followed by a single topical application of erythromycin ointment on the surface of the cornea. Histologic examination on consecutive days demonstrates progressive blood vessel growth into the cornea toward the pellet with only rare inflammatory cells seen. This angiogenic response is not altered by severe immune suppression with total body irradiation, and pellets with sucralfate alone do not induce angiogenesis. New vessels are primarily induced by the bFGF rather than by inflammation. The animals are fed daily from 2 days after implantation by gastric lavage with one or more compounds suspended in 0.5% carboxymethylcellulose or vehicle alone. Immunosuppressed animals receive total body radiation of 6 Gy for 6 minutes immediately prior to implantation of the pellets. This dose of radiation results in a marked leukocytopenia with >80% reduction in the leukocyte count by day 2 and >90% reduction by day 3, results that are consistent with previous reports.

Animals are examined with a slit lamp every other day in a masked manner by the same corneal specialist (M.S.L.). The area of corneal neovascularization is determined by measuring with a reticule the vessel length (L) from the limbus and the number of clock hours (C) of limbus involved. A formula is used to determine the area of a circular band segment: C/12 x 3.1416 [r² - (r - L)²], where r = 6 mm, the measured radius of the rabbit cornea. The uniform contiguous band of neovascularization adjacent to the pellet is measured, thus, the total inhibition of neovascularization can be assessed.

### Mouse Matrigel Pug Angiogenesis Assays

To confirm the effects of a composition on angiogenesis, a mouse Matrigel plug angiogenesis assay can be used. Various growth factors (e.g., IGF-1, bFGF or VEGF) (250 ng) and Heparin (0.0025 units per/mL) are mixed with growth factor reduced Matrigel as previously described (Montesano, et al., J. Cell Biol. 1983, 97:1648-1652; Stefansson, et al., J. Biol. Chem. 2000, 276:8135-8141). Compositions described herein or control antibodies can be included in the Matrigel preparations utilizing one or more dosage groups of animals. In control experiments, Matrigel is prepared in the absence of growth factors. Mice are injected subcutaneously with 0.5 mL of the Matrigel preparation and allowed to incubate for one week. Following the incubation period, the mice are sacrificed and the polymerized Matrigel plugs surgically removed. Angiogenesis within the Matrigel plugs is quantified by two established methods, including immunohistochemical analysis and hemoglobin content (Furstenberger, et al., Lancet. 2002, 3:298-302; Volpert, et al., Cancer Cell 2002, 2(6):473-83; and Su, et al., Cancer Res. 2003, 63:3585-3592). For immunohistochemical analysis, the Matrigel plugs are embedded in OCT, snap frozen and 4 µm sections prepared. Frozen sections are fixed in methanol/acetone (1:1). Frozen sections are stained with polyclonal antibody directed to CD31. Angiogenesis is quantified by microvascular density counts within 20 high powered (200X) microscopic fields.

Hemoglobin content can be quantified as described previously (Schnaper, et al., J. Cell Physiol. 1993, 256:235-246; Montesano, et al., J. Cell Biol. 1983, 97:1648-1652; Stefansson, et al., J. Biol. Chem. 2000, 276:8135-8141; and Gigli, et al., J. Immunol. 1986, 100:1154-1164). The Matrigel implants are snap frozen on dry ice and lyophilized overnight. The dried implants are resuspended in 0.4 mL of 1.0% saponin (Calbiochem) for one hour, and disrupted by vigorous pipetting. The preparations are centrifuged at 14,000 X g for 15 minutes to remove any particulates. The concentration of hemoglobin in the supernatant is then determined directly by measuring the absorbency at 405 nm and compared to a standard concentration of purified hemoglobin.

### Methods of Assaying Cell Migration

Assays for cell migration have been described in the literature, e.g., by Brooks, et al., J. Clin. Invest 1997, 99:1390-1398 and methods for measuring cell migration are known to those of skill in the art. In one method for measuring cell migration described herein, membranes from transwell migration chambers are coated with substrate (here, endoglin and/or VEGF), the transwells washed, and non-specific binding sites blocked with BSA. Tumor cells from sub-confluent cultures are harvested, washed, and resuspended in migration buffer in the presence or absence of assay antibodies. After the tumor cells are allowed to migrate to the underside of the coated transwell membranes, the cells remaining on the top-side of the membrane are removed and cells that migrate to the under-side are stained with crystal violet. Cell migration is then quantified by direct cell counts per microscopic field.

### Methods of Assaying Cell Proliferation

Cell proliferation can be assayed by methods known to those of skill in the art. As described herein, subconfluent human endothelial cells (HUVECs) can be resuspended in proliferation buffer containing low (5.0%) serum in the presence or absence of CM (25 µL) from ECV or ECVL cells, and endothelial cells allowed to proliferate for 24 hours. Proliferation can be quantified by measuring mitochondrial dehydrogenase activity using a commercially available WST-1 assay kit (Chemicon). Also, as described herein, proliferation can be quantified by measuring ³H incorporation using standard methods. (She et al., Int. J. Cancer, 108: 251-257 (2004)).

Other methods of assessing cell proliferation are known in the art and are contemplated herein. Further non-limiting examples are described in more detail in the examples.

### Methods of Inducing CDC, ADCC and Opsonization

Various therapies have been directed to augmenting the body's natural immune response to transformed cells. Conventional effector methods include complement dependent cytolysis ("CDC"), antibody dependent cellular cytotoxicity ("ADCC") and phagocytosis (clearance by reticuloendothelial system after the target cell is coated with immunoglobulin). It is known that in the presence of antibodies, certain effector cells, such as lymphoid cells having surface bound receptors for the Fc regions of antibodies, mediate an antibody dependent cellular cytoxicity ("ADCC") reaction against target cells. By means of ADCC, these effector cells exert cytolytic activity against such target cells.

Two types of ADCC reactions have been demonstrated *in vitro.* In classical ADCC reactions, effector cells attach to antibody-coated target cells and subsequently cause cytolysis of the target cells (A. H. Greenberg et al., "Characteristics Of The Effector Cells Mediating Cytotoxicity Against Antibody-Coated Target Cells," Immunology, 21, p. 719 (1975)). This attachment between effector and target cell results from the interaction of the Fc region of the antibody coating the target cell and the Fc receptor of the effector cell. One disadvantage of this type of ADCC reaction is that it may be hampered by circulating antigen-antibody complexes, often associated with various diseases, which compete with the target-cell bound antibody for the Fc receptors of the effector cells (I. C. M. MacLennan, "Competition For Receptors For Immunoglobulin On Cytotoxic Lymphocytes," Clin. Exp. Immunol., 10, p. 275 (1972)). Due to this drawback of classical ADCC, a second type of ADCC reaction - antibody-directed ADCC - can be utilized. In antibody-directed ADCC, the target-specific antibody is first attached to the effector cell and the resulting complex is then "directed," via the antibody, to its specific antigen on the target cell surface. Advantageously, antibody-directed ADCC may not be affected by the presence of antigen-antibody complexes circulating in the host system. The interaction between antibodies and effector cells via Fc region/Fc receptor attachment is normally weak. And, in some instances, antibodies do not remain associated with effector cells for a period of time sufficient to permit lysis of target cells. In view of this potential problem, antibodies have been attached to the effector cells using pre-treatment with polyethylene glycol and a mixture of phthalate oils (J. F. Jones and D. M. Segal, "Antibody-Dependent Cell Mediated Cytolysis (ADCC) With Antibody-Coated Effectors: New Methods For Enhancing Antibody Binding And Cytolysis," J. Immunol., 125, pp. 926-33 (1980)). The applicability of this method for *in vivo* treatments, however, may be diminished by the toxic effects that any polyethylene glycol and phthalate oil residues on the antibody-effector cell complex may have on the body.

Alternatively, a method has been proposed for enhancing antibody-directed ADCC by adjuvant chemotherapy with cytotoxic drugs (I. R. Mackay et al., "Effect On Natural Killer And Antibody-Dependent Cellular Cytotoxicity Of Adjuvant Cytotoxic Chemotherapy Including Melphalan In Breast Cancer," Cancer Immunol. Immunother., 16, pp. 98-100 (1983)). Assays for testing for ADCC are well-known in the art, such as for example, US Patent No. 5,756,097.

Accordingly, the present embodiments provide antibodies that can bind to cells having a role in neovascularization or angiogenesis of that can enhance phagocytosis and killing of the cells and thereby enhance protection *in vivo.* Also provided are other antibodies and functional fragments thereof that immunoreact, specifically bind to, or preferentially bind to a binding site or epitope to which such antibodies can bind and which have the same effect.

The antibodies can also be opsonic, or exhibit opsonic activity, for cells having a role in neovascularization or angiogenesis (e.g., endothelial cells). As those in the art recognize, "opsonic activity" refers to the ability of an opsonin (generally either an antibody or the serum factor C3b) to bind to an antigen or cell receptor to promote attachment of the antigen or cell receptor to a phagocyte and thereby enhance phagocytosis. Certain cells become extremely attractive to phagocytes such as neutrophils and macrophages when coated with an opsonic antibody and their rate of clearance from the bloodstream is strikingly enhanced. Opsonic activity may be measured in any conventional manner as described, for example, in US Patent No. 6,610,293.

In another non-limiting embodiment, a patient having a neovascular disorder or an angiogenesis dependent disorder sheds antigens/peptides (e.g., endoglin) from the angiogenesis. These antigens/peptides can be "tumor associated antigens." Such patients can be systemically administered an antibody to the antigen/peptide (e.g., endoglin) and can initiate any of the pathways described herein to induce CDC, ADCC, opsonization, or any other form of cell-mediated killing.

### Additional Assays

Other assays known in the art can also be used to test the effect of the compositions described herein such as, for example, those described in the examples below.

### PACKAGES AND KITS

In still further embodiments, the present application concerns kits for use with the compounds described above. Chimeric antibodies that preferentially bind to endoglin and antibodies that preferentially bind to VEGF can be provided in a kit. The kits can include one or more suitable container means.

The container means of the kits will generally include at least one vial, test tube, flask, bottle, syringe and/or other container means, into which the at least one polypeptide can be placed, and/or preferably, suitably aliquoted. The kits can include a means for containing at least one fusion protein, detectable moiety, reporter molecule, and/or any other reagent containers in close confinement for commercial sale. Such containers may include injection and/or blow-molded plastic containers into which the desired vials are retained. Kits can also include printed material for use of the materials in the kit.

Packages and kits can additionally include a buffering agent, a preservative and/or a stabilizing agent in a formulation. Each component of the kit can be enclosed within an individual container and all of the various containers can be within a single package. Kits can be designed for cold storage or room temperature storage.

Additionally, the preparations can contain stabilizers to increase the shelf-life of the kits and include, for example, bovine serum albumin (BSA). Where the compositions are lyophilized, the kit can contain further preparations of solutions to reconstitute the lyophilized preparations. Acceptable reconstitution solutions are well known in the art and include, for example, pharmaceutically acceptable phosphate buffered saline (PBS).

Additionally, the packages or kits provided herein can further include any of the other moieties provided herein such as, for example, one or more reporter molecules and/or one or more detectable moieties/agents.

Packages and kits can further include one or more components for an assay, such as, for example, an ELISA assay. Samples to be tested in this application include, for example, blood, plasma, tissue/tumor sections and secretions, urine, lymph, and products thereof. Packages and kits can further include one or more components for collection of a sample (e.g., a syringe, a cup, a swab, etc.).

Packages and kits can further include a label specifying, for example, a product description, mode of administration and/or indication of treatment. Packages provided herein can include any of the compositions as described herein. The package can further include a label for treating various forms of cancer and their metastases.

The term "packaging material" refers to a physical structure housing the components of the kit. The packaging material can maintain the components sterilely and can be made of material commonly used for such purposes (e.g., paper, corrugated fiber, glass, plastic, foil, ampules, etc.). The label or packaging insert can include appropriate written instructions. Kits, therefore, can additionally include labels or instructions for using the kit components in any method described herein. A kit can include a compound in a pack, or dispenser together with instructions for administering the compound in a method described herein.

Further provided are kits which utilize the diagnostic methods and assays described herein. In some embodiments, a kit comprises reagents for the detection of a gene or genes whose expression levels have been correlated with sensitivity or resistance to an angiogenesis inhibitor in a sample of cancer cells from a patient. In some embodiments, the gene or genes are selected from VEGF, VEGF receptor, and CD105. In some embodiments, the kit comprises VEGF. In some embodiments, the kit comprises VEGF receptor. In some embodiments, the kit comprises CD105. In some embodiments, the kit comprises at least two of VEGF, VEGF receptor, and CD105. In some embodiments, the kit comprises at least two genes that have been correlated with sensitivity to an angiogenesis inhibitor. In some embodiments, the kit comprises at least two genes that have been correlated with resistance to an angiogenesis inhibitor. In some embodiments, the kit comprises at least one gene that has been correlated with sensitivity to an angiogenesis inhibitor and one gene that has been correlated with resistance to an angiogenesis inhibitor.

In still further embodiments, a kit comprises reagents for the detection of VEGF, VEGF receptor, and CD105 expression levels in a sample of tumor cells from a patient to be treated; and a dose or doses an inhibitor, including but not limited to antibodies described herein, in a variety of dosage forms, such as capsules, caplets, gel caps, powders for suspension, etc. It is further contemplated that kits contain reagents for the detection of VEGF, VEGF receptor, and CD105 expression levels in a sample of tumor cells from a patient to be treated will further comprise any of the aforementioned embodiments of the kits for co-administration of at least one additional angiogenesis inhibitor.

Instructions can include instructions for practicing any of the methods described herein including treatment methods. Instructions can additionally include indications of a satisfactory clinical endpoint or any adverse symptoms that may occur, or additional information required by regulatory agencies such as the Food and Drug Administration for use on a human subject.

The instructions may be on "printed matter," e.g., on paper or cardboard within or affixed to the kit, or on a label affixed to the kit or packaging material, or attached to a vial or tube containing a component of the kit. Instructions may additionally be included on a computer readable medium, such as a disk (floppy diskette or hard disk), optical CD such as CD- or DVD-ROM/RAM, magnetic tape, electrical storage media such as RAM and ROM, IC tip and hybrids of these such as magnetic/optical storage media.

### EXAMPLES

The application may be better understood by reference to the following non-limiting examples, which are provided as exemplary embodiments of the application.

### EXAMPLE 1

### BIAcore (Surface Plasmon Resonance: SPR) Analysis

### Chimeric Anti-Endoglin Antibody Binding

Affinity of antibodies can be assessed using, for example, BIAcore analysis using standard protocols. Briefly, anti-histidine tag antibody is coupled to a BIAcore chip for the capture of His-tagged recombinant human endoglin which will in turn be used to measure the binding of a chimeric anti-endoglin antibody. Development of the SPR assay is performed in a minimum of 2 chip preparation batches plus 8 analytical batches. The following parameters are assessed in the development of the assay:

### (a) Coupling of anti-his antibody to CM5 chips

An anti-his tag antibody is coupled to a BIAcore CM5 chip by conventional amine chemistry using EDC/NHS. The reaction conditions (concentration and pH) will be optimized

### (b) Binding of human endoglin and regeneration of Biosensor Chip

Conditions are tested for binding of human endoglin and regenerating the chip using various buffers (based on previous experience) to elute the bound antibody. Once a candidate method for regeneration has been developed, the binding capacity and background of a single chip surface are measured over at least 25 cycles. The target is to obtain on average an increase in background < 10 RU per cycle and decrease in capacity < 1 % per cycle.

### (c) Binding of human endoglin

The dose response of human endoglin is measured in order to determine a suitable concentration to approach maximal binding.

### (d) Binding of chimeric anti-endoglin antibody

The dose response of chimeric anti-endoglin antibody is measured in order to determine a suitable range for kinetic or equilibrium binding experiments (which can include comparison of relative kinetic constants, kₐ and k_{d} or a comparison of relative potency by the parallel line method).

### (e) Pre-validation experiments

The binding experiments is repeated at least three times under the chosen conditions using different chips, different flow cells and on different occasions in order to obtain preliminary information about the precision and accuracy of the measurements. All BIAcore experiments are carried out at 25°C in HBS-EP running buffer.

### Anti-VEGF Antibody Binding

Affinity of antibodies can be assessed using, for example, BIAcore analysis using standard protocols. Briefly, anti-histidine tag antibody is coupled to a BIAcore chip for the capture of His-tagged recombinant human VEGF which will in turn be used to measure the binding of a anti-VEGF antibody. Development of the SPR assay is performed in a minimum of 2 chip preparation batches plus 8 analytical batches. The following parameters are assessed in the development of the assay:

### (a) Coupling of anti-his antibody to CM5 chips

An anti-his tag antibody is coupled to a BIAcore CM5 chip by conventional amine chemistry using EDC/NHS. The reaction conditions (concentration and pH) will be optimized.

### (b) Binding of human VEGF and regeneration of Biosensor Chip

Conditions are tested for binding of human VEGF and regenerating the chip using various buffers (based on previous experience) to elute the bound antibody. Once a candidate method for regeneration has been developed, the binding capacity and background of a single chip surface are measured over at least 25 cycles. The target is to obtain on average an increase in background < 10 RU per cycle and decrease in capacity < 1 % per cycle.

### (c) Binding of human VEGF

The dose response of human VEGF is measured in order to determine a suitable concentration to approach maximal binding.

### (d) Binding of anti- VEGF antibody

The dose response of chimeric anti-VEGF antibody is measured in order to determine a suitable range for kinetic or equilibrium binding experiments (which can include comparison of relative kinetic constants, kₐ and k_{d} or a comparison of relative potency by the parallel line method).

### (e) Pre-validation experiments

The binding experiments is repeated at least three times under the chosen conditions using different chips, different flow cells and on different occasions in order to obtain preliminary information about the precision and accuracy of the measurements. All BIAcore experiments are carried out at 25°C in HBS-EP running buffer.

### EXAMPLE 2

### ELISA for Chimeric Anti-Endoglin Antibody Binding

An ELISA can be used to assay binding of chimeric anti-endoglin antibodies to endoglin. Briefly, an ELISA is performed according to the following steps:
1. Coat a Nunc Maxisorp plate with MAB9811-01 (polyclonal anti-endoglin antibody) at 1500 ng/ml in PBS, 100 µl/well. Cover the plate with a sealer and incubate overnight (16-24 hours) at 4°C.
2. Wash the plate 2X with -200 µl of PBS (without Tween).
3. Add 200 µl/well of BSA blocking solution (1% BSA) and incubate 60 minutes at room temperature.
4. Wash the plate 3X with PBS containing Tween (PBS-T) using the BioTek plate washer.
5. Add 100 µl/well of CD105 (R&D Systems Cat 1097-EN) at 100 ng/ml in PBS-T with 0.1% BSA and incubate 60 minutes at room temperature.
6. Wash the plate 3X with PBS-T using the BioTek plate washer.
7. In test wells: add 100 µl/well of chimeric anti-endoglin antibodies at 20, 10, 4, 2, 1, 0.5 and 0.2 ng/ml (diluted in PBS-T with 0.1% BSA) and incubate 60 minutes at room temperature. In negative control wells: add 100 µl/well of isotype matched control antibody.
8. Wash the plate 3X with PBS-T using the BioTek plate washer.
9. Add 100 µl/well of Goat anti-Human IgG conjugated to HRP (Jackson Immunoresearch), diluted 1:10000 in PBS-T with 0.1% BSA to all wells; incubate 30-60 minutes at room temperature.
10. Wash the plate 5X with PBS-T using the BioTek plate washer.
11. Add 100 µl/well of TMB substrate solution and incubate uncovered in the dark for 15 minutes.
12. Stop the reaction by addition of 100 µl/well of TMB Stop Solution.

Samples are run in triplicate and the optical density is read to construct a standard curve and determine the binding constant. Statistical analysis is conducted using the Student's t-test or another standard test.

One would understand that a similar protocol can be used to test for binding of antibodies to VEGF.

### EXAMPLE 3

### Antibody Avidity and Number of Available Epitopes on Endoglin-Expressing Cells.

Antibody avidity and number of available epitopes on endoglin-expressing cells can be assessed utilizing Scatchard plot analyses using standard protocols.

Briefly, Scatchard plot analyses of direct binding of radiolabeled chimeric anti-endoglin antibodies to endoglin-expressing KM-3 leukemia cells and sub-confluent proliferating HUVECs are carried out. The purified anti-endoglin antibodies are individually radiolabeled with ¹²⁵I using Iodo-Gen and according to standard methods known to those skilled in the art. The radiolabeled chimeric anti-endoglin antibodies are assayed for the iodine atoms per IgG molecule on the average, respectively. Titration experiments are carried out using a fixed amount (0.1 µg) of each ¹²⁵I-labeled mAb and 2-fold serial increments of endoglin-expressing KM-3 or HUVEC cells to determine antigen-binding activity. Analysis of Scatchard plot of binding data is carried out according to known methods. An equilibrium constant and an average maximal number of mAb bound/cell are estimated by this analysis.

### EXAMPLE 4

### Western Blots Assay for Blocking Activity

The ability of chimeric anti-endoglin antibodies to block CD105 stimulated activation of cells that express CD105 can be assayed via western blots to detect the phosphorylation of the proteins involved in the CD105 signaling pathway.

Western blot analyses are performed to identify phosphorylated Smad1/5/8 or Smad2 as according to known western blotting techniques. PSmad1 and PSmad2 antibodies specifically recognize phosphorylated Smad1/5 or phosphorylated Smad2 in non-transfected endothelial cells. Primary antibodies against Smad1, Smad2, Smad5, Id1 (Santa Cruz) and endoglin are utilized to detect molecules in samples. Detection is performed by enhanced chemoluminescence (ECL).

### EXAMPLE 5

### Inhibition of HUVEC growth and ³H-thymidine incorporation assay

A number of assays are available to assess inhibition of cell growth.

In one example, HUVECs are cultured in 75-cm² flasks (Falcon, Becton-Dickinson, Franklin Lakes, NJ) in a CO₂ incubator at 37°C under sub-confluent conditions. Cells are detached by incubating with Hanks' balanced salt solution with 15 mM EDTA in 25 mM HEPES buffer, pH 7.3, at 37°C for 15 min. After washing twice with ice-cold PBS, cells are re-suspended in endothelial cell growth medium at a concentration of 25,000 cells/ml. In additional experiments, human umbilical vein endothelial cells (HUVECs) are suspended and cultured in an endothelial cell growth medium free of FBS and bovine brain extracts. A 200 µl aliquot of cell suspension is seeded to each well of 96-well culture plates. Cells are cultured at 37°C in a CO₂ incubator overnight before chimeric anti-endoglin antibodies, anti-VEGF antibodies, a combination of chimeric anti-endoglin antibodies and anti-VEGF antibodies, control IgG or TGF-β1 are added in triplicate. Culture plates are kept in the incubator for 72 hr, during which fresh media and antibodies or controls are replaced every 24 hr. ³H-thymidine (1 µCi) is added into each well and the plates are incubated for 20 hr. Cells are washed with PBS followed by treatment with 100 µl/well trypsin-EDTA (0.05% trypsin, 0.53 mM EDTA) at 37°C for 15 min. Cells are harvested onto glass fiber filters (Wallac Printed FiltermatA) using Harvester 96 (TOMTEC, Hamden, CT) and ³H-radioactivity is determined in a Trilux 1540 MicroBeta Liquid Scintillation and Luminescence Counter (Wallac, Turku, Finland).

### EXAMPLE 6

### Inhibition of HUVEC growth by MTS assay

A number of assays are available to assess inhibition of endothelial cell growth.

In one example, HUVECs are cultured in 96 well plates (Falcon, Becton-Dickinson, Franklin Lakes, NJ) in a CO₂ incubator at 37°C under sub-confluent conditions at 5,000 cells per well in EGM-2 media (Clonetics, Walkersville, MD) containing 0.5% fetal bovine sera and 30 ng/mL of VEGF. Cells are allowed to adhere to the culture flask for at least 24 hours and then incubated with anti-VEGF antibody with or without chimeric anti-endoglin antibody. Culture plates are kept in the incubator for 72 hr, during which fresh media and antibodies or controls are replaced every 24 hr. Following three days of treatment with antibody, MTS tetrazolium compound is added to wells for one hour and absorbance is quantified at 490 nm according to the manufacturer's instructions (Cell Titer 96 Aqueous One Solution Cell Proliferation Assay, Promega). All samples are run in triplicate.

### EXAMPLE 7

### Assay for Inhibition of Cell Migration

Migration (chemokinesis) as a measure of cell proliferation and activation is measured using a Boyden chamber.

Briefly, cell migration is assessed as follows: a Costar nucleopore filter (8 mm pore) is coated with tibronectin overnight at 4°C. The chamber is washed with phosphate-buffered saline (PBS) and the lower chamber was filled with DMEM with or without serum and with or without TGF-β3. Cells are trypsinized and suspended at a final concentration of 50,000 cells/ml in DMEM in the presence of a control antibody, a chimeric anti-endoglin antibody, an anti-VEGF antibody or a combination thereof. A 150 µl aliquot of the cell suspension is added to the upper chamber and incubated at 37°C. After 16 hrs, the cells are washed and the upper surface is wiped to remove the non-migrating cells. The membranes are fixed in methanol, washed with water, stained and the numbers of cells present on the lower surface are counted.

### EXAMPLE 8

### ADCC Assay

The antibodies described herein can be assessed with respect to their ability to generate IL-2 activated natural killer (NK) cells and to induce ADCC using, for example, the following protocols.

### NK Isolation and Generation of IL-2 Activated NK Cells

PBMC are isolated and allowed to rest for 24 hrs at 4°C in RPMI with 10% FBS. PBMC are then placed in RPMI with 2% FBS (Total Volume = 50 mL), and 10mL of the cell suspension are plated in a petri dish. PBMC are incubated for 2 hrs at 37°C and the non-adherent cells are collected. NK cells are cultured at 8 X 10⁶/mL with 1000 U/mL IL-2 for 48 hrs, followed by normal culturing for 5-8 days before using in an assay.

### Natural Cytotoxicity and ADCC Assays

NK cells are scraped from the culture and collected in a 50mL conical tube. Cells are washed once with RPMI Complete and Spun at 1200 rpm for 10 minutes. NK cells are then re-suspended in 5mL RPMI Complete media and counted. Prior to performing the assay, the NK cell count is normalized to a effector: target ratio of 10:1. Normalized NK cells are plated and 10 µL of chimeric anti-endoglin antibodies added into designated wells and incubated for 30 minutes at 37°C. Control samples include untreated or control-antibody treated cell populations.

Target cells of interest are collected ((HUVEC cells), washed, spun at 1200 rpm for 10 minutes, and re-suspended in 5 mL RPMI Complete media. Target cells are washed again and re-suspended in Serum Free RPMI to a final concentration of 1 x 10⁶ cells/mL. Target cells are then labeled with a final concentration of 5ug/mL Calcein AM for 1 hr at 37°C, followed by a two washes with RPMI Complete. Target cells are then re-suspended and added to the NK cell wells. The target cell/NK cell combination is incubated at 37°C for 4 hours. After incubation, the plates are spun at 1200 rpm for 5 minutes, and the cells are washed and re-suspended in DPBS. The fluorescence is read using Excitation/Emission of 450/530 nm and the emission is a measure of the cell killing mediated by the antibodies.

### Example 9

### Dosages for Administration

Optimal dosages of the antibodies described herein can be determined using art-recognized methods and as described above.

In one non-limiting embodiment, the antibodies described herein can be administered to a subject in various dosing amounts and over various time frames. Non-limiting doses include about 0.01 mg/kg, about 0.05 mg/kg, about 0.1 mg/kg, about 0.5 mg/kg, about 1 mg/kg, about 5 mg/kg, about 10 mg/kg, about 20 mg/kg, about 30 mg/kg, or any integer in between. Additionally, the dose(s) of an antibody can be administered twice a week, weekly, every two weeks, every three weeks, every 4 weeks, every 6 weeks, every 8 weeks, every 12 weeks, or any combination of weeks therein. Dosing cycles are also contemplated such as, for example, administering antibodies one or twice a week for 4 weeks, followed by two weeks without therapy. Additional dosing cycles including, for example, different combinations of the doses and weekly cycles described herein are also contemplated herein.

In another embodiment, Bevacizumab (AVASTIN®) can be administered based on the following dosages and regimens:

| **Tumor type** | **Dose regimen** |
|---|---|
| Refractory solid tumors | Administration of 2.5, 5, 7.5, 10 or 15 mg/kg intravenously weekly or every 3 weeks followed by 7.5 or 15mg/kg intravenously weekly every 3 weeks. |
| Advanced solid tumors | Administration of 5 or 10 mg/kg intravenously every 7 or 14 days. |
| Colorectal cancer | Administration of 5 mg/kg intravenously on day 1, given every 1 or 2 weeks ± 2 days for a total of three doses. |
| | Administration of 5 mg/kg intravenously on day 1 every 1 or 2 weeks for 10 cycles. |
| | Administration of 7.5 mg/kg intravenously on day 1 every 1 or 3 weeks for 6 cycles. |
| Lung cancer | Administration of 15 mg/kg intravenously on day 1 every 1 or 3 weeks for 3 or 6 cycles. |
| Brain cancer (Gliosarcomas and Gliomas) | Administration of 10 mg/kg weekly or every other week for 6 cycles. |
| | Four (4) cycles of administration of 10 mg/kg every 7 or 14 days. |
| | Administration of 15 mg/kg every week or every 3 weeks. |
| Ovarian cancer | Administration of 15 mg/kg intravenously on day 1, followed by administration of 15 mg/kg intravenously every 7 or 21 days for 5 cycles. |
| Neuroendocrine Carcinoma | Administration of 15 mg/kg intravenously over 90 minutes every 7 or 21 days. |
| Cervical cancer | Administration of 10 mg/kg intravenously on days 1, 7 and 15 |
| Breast cancer | Administration of 10 mg/kg intravenously every 1 or 2 weeks for 2 or 4 cycles. |
| Prostate cancer | Intravenous administration every week or every 3 weeks for a total of 17 cycles for a total of 1 year. |
| Liver cancer | Administration of 10 mg/kg intravenously every 7 or 14 days, repeating the cycle every 28 days. |
| Pancreatic cancer | Administration of 10 to 15 mg/kg intravenously over 90 minutes every 1 or 2 weeks. |
| Metastatic Head and Neck Cancer | Administration of 15 mg/kg intravenously every 1 or every 3 weeks. |

### EXAMPLE 10

### Endoglin (CD105) Expression on Solid Tumor Types

Expression of endoglin on solid tumors was assessed using immunohistochemistry. Frozen and acetone-fixed human carcinoma samples were allowed to react with a 10,000-fold dilution of anti-endoglin antibody SN6j ascites or an isotype-matched control IgG ascites and stained with DAKO staining kits. Counterstaining was performed with hematoxylin. SN6j bound to blood vessels within the tumor, while isotype-matched control IgG did not show any staining. All tumor types tested demonstrated endoglin expression within the tumor vasculature.

| **Tumor** | **Number of Specimens** | **Reactivity (0,+,++,+++)** |
|---|---|---|
| Bladder | 2 | +++ |
| Bone | 1 | +++ |
| Breast | 21 | +++ |
| Colon | 4 | +++ |
| Esophagus | 1 | +++ |
| Liver | 1 | +++ |
| Lung | 3 | +++ |
| Lymphoma | 7 | +++ |
| Ovary | 2 | +++/++ |
| Pancreas | 1 | +++ |
| Penis | 1 | +++ |
| Rectum | 2 | +++ |
| Stomach | 2 | +++/++ |
| Thyroid | 3 | +++/++ |

### EXAMPLE 11

### Anti-angiogenic Therapy of Preformed Human Breast Cancer Tumors in Human Skin Grafted into SCID Mice

The effect of the antibodies described herein can be assessed with respect to their anti-angiogenic effect on pre-formed human breast cancer tumors in human skin grafted into SCID mice.

Briefly, MCF-7 cells (8 x 10⁶ cells in 0.1 ml PBS) are transplanted intradermally into human full-thickness skin grafted into SCID mice when the grafts showed no signs of inflammation, contraction or rejection. The mice are left untreated until distinct palpable tumors (3 to 6 mm in diameter in most cases) appear. Mice with distinct tumors are divided into groups for the therapeutic studies. Solutions (compositions) containing (1) chimeric anti-endoglin antibody, (2) bevacizumab (AVASTIN®), (3) a combination of a chimeric anti-endoglin antibody and bevacizumab (AVASTIN®), or (4) an isotype-matched control IgG are each diluted with sterile PBS containing mouse serum albumin (0.05% final concentration). For the mAb therapy, 200 µg/0.2 mL test antibody or control IgG is intravenously (i.v.) administered via the tail vein of mice. The administration is given every two to three days.

During the treatment, mice are monitored daily for tumor size and morbidity. Mice are weighed twice a week using an electronic balance (OHAUS™ Model GT210). Tumor size is measured three times a week using an electronic caliper (PRO-MAX 6 inch caliper; Fowler Co., Newton, Mass.) connected to a computer using OptoDemo™ software (Fowler Co.). The measured tumor diameters are converted to tumor volumes using, for example, the following formula: V=length x width x height x pi/6. Statistical analysis of the data for the comparison of different groups of mice is carried out using Student's t-test.

### EXAMPLE 12

### Mouse Model for Ovarian Cancer

To determine the ability of a chimeric anti-endoglin antibody and an anti-VEGF antibody, or to treat ovarian cancer, an ovarian cancer cell line can be used in SCID, transgenic or nude mice.

Briefly, ovarian cancer cells are implanted into SCID, transgenic or nude mice to generate ovarian tumors. Groups of mice bearing established tumors are treated by i.v. administration of escalating doses (starting at 1.8 mg/kg body weight) of chimeric anti-endoglin antibody, bevacizumab (AVASTIN®), a combination of a chimeric anti-endoglin monoclonal antibody (mAb) and bevacizumab (AVASTIN®), or control IgG. The treatment is performed 2 or 3 times per week. Chemotherapy may be used in all groups. The mice are monitored and tumor growth is measured 2 or 3 times per week.

### EXAMPLE 13

### Mouse Model for Kidney Cancer

To determine the ability of a chimeric anti-endoglin antibody and an anti-VEGF antibody to treat kidney cancer, a kidney cancer cell line is used in SCID, transgenic or nude mice.

Briefly, kidney cancer cells are implanted into SCID, transgenic or nude mice to generate kidney tumors. Groups of mice bearing established tumors are treated by i.v. administration of escalating doses (starting at 1.8 mg/kg body weight) of chimeric anti-endoglin antibody, bevacizumab (AVASTIN®), a combination of a chimeric anti-endoglin monoclonal antibody (mAb) and bevacizumab (AVASTIN®), or control IgG. The treatment is performed 2 to 3 times per week. Chemotherapy may be used in all groups. The mice are monitored and tumor growth is measured 2 to 3 times per week.

### EXAMPLE 14

### Mouse Model for Colorectal Cancer

To determine the ability of a chimeric anti-endoglin antibody and an anti-VEGF antibody to treat colorectal cancer, a breast cancer cell line is used in SCID, transgenic or nude mice.

Briefly, breast cancer cells are implanted into SCID, transgenic or nude mice to generate colorectaltumors. Groups of mice bearing established tumors are treated by i.v. administration of escalating doses (starting at 10 mg/kg body weight) of chimeric anti-endoglin antibody, bevacizumab (AVASTIN®), or a combination of a chimeric anti-endoglin monoclonal antibody (mAb) and bevacizumab (AVASTIN®). Control animals are administered a control IgG. The treatment is performed 2 or 3 times per week. Chemotherapy may be used in all groups. The mice are monitored and tumor growth is measured 2 to 3 times per week.

### EXAMPLE 15

### Mouse Model for Brain Cancer

To determine the ability of a chimeric anti-endoglin antibody and an anti-VEGF antibody to treat brain cancer, a glioblastoma multiforme cell line is used in SCID, transgenic or nude mice.

Briefly, glioblastoma multiformet cancer cells are implanted into SCID, transgenic or nude mice to generate breast tumors. Groups of mice bearing established tumors are treated by i.v. administration of escalating doses (starting at 10 mg/kg body weight) of chimeric anti-endoglin antibody, bevacizumab (AVASTIN®), or a combination of a chimeric anti-endoglin monoclonal antibody (mAb) and bevacizumab (AVASTIN®). Control animals are administered a control IgG. The treatment is performed 2 or 3 times per week. Chemotherapy may be used in all groups. The mice are monitored and tumor growth is measured 2 to 3 times per week.

### EXAMPLE 16

### Clinical Trial of combination therapy for Colorectal Cancer

This example describes a randomized, blinded, placebo-controlled, multicenter, Phase II or Phase III study designed to provide a preliminary assessment of the safety and efficacy of combining chimeric anti-endoglin antibody with bevacizumab (AVASTIN®) in patients with colorectal cancer. Approximately about 100 - about 800 patients are enrolled, with about 50 - about 400 patients being assigned to a treatment group and about 50 - about 400 patients assigned to a placebo group. The trial will consist of the administration of intravenous repeating doses of chimeric anti-endoglin antibody at about 0.1 - about 10 mg/kg or placebo every one to three weeks combined with bevacizumab (AVASTIN®) at about 5 mg/kg intravenously on day 1 every 2-3 weeks for 6-10 cycles. Chemotherapy may be used in all groups. The time frame of the study is estimated at about 6 months - about 5 years, with continued therapy for responders as indicated at the end of the initial study. Additional outcome measures are as follows:

Primary outcome measure: overall response rate. One goal of the study is to demonstrate an increase overall response rate from about 40% with bevacizumab (AVASTIN®) plus placebo to about 60% (or more) with bevacizumab (AVASTIN®) plus chimeric anti-endoglin antibody.

Secondary outcome measures that can be assessed include duration of response, time to tumor progression, overall survival, serious and non-serious adverse events. For example, a treatment may prevent progression of the disease (i.e., stasis) or may result in an improvement. Alternately, or in addition, other goals can be measured with respect to one or more of the following: decreased tumor burden, decreased neovascularization, reduced side effects, decreased adverse reactions, and/or increased patient compliance.

### EXAMPLE 17

### Clinical Trial of combination therapy for Kidney Cancer

This example describes a randomized, blinded, placebo-controlled, multicenter, Phase II or Phase III study designed to provide a preliminary assessment of the safety and efficacy of combining chimeric anti-endoglin antibody with Bevacizumab (AVASTIN®) in patients with renal cell cancer (kidney cancer). Approximately about 100 - about 800 patients are enrolled, with about 50 - about 400 patients being assigned to a treatment group and about 50 - about 400 patients assigned to a placebo group. The trial will consist of the administration of intravenous repeating doses of chimeric anti-endoglin antibody at about 0.1 - about 30 mg/kg or placebo every one to three weeks combined with Bevacizumab (AVASTIN®) at about 2.5 - about 15 mg/kg every two weeks for 3-6 cycles or until progression. Interferon may also be used in both treatment arms. The time frame of the study is estimated at about 6 months - about 5 years, with continued therapy for responders as indicated at the end of the initial study. Additional outcome measures are as follows:

Primary outcome measure: progression-free survival. One goal of the study is to demonstrate an increase in progression free survival from about 9-13 months in the Bevacizumab (AVASTIN®) plus placebo arm to about 14-18 months (or more) in the Bevacizumab (AVASTIN®) plus chimeric anti-endoglin antibody arm.

Secondary outcome measures that can be assessed include duration of response, time to tumor progression, overall survival, serious and non-serious adverse events. For example, a treatment may prevent progression of the disease (i.e., stasis) or may result in an improvement. Alternately, or in addition, other goals can be measured with respect to one or more of the following: decreased tumor burden, decreased neovascularization, reduced side effects, decreased adverse reactions, and/or increased patient compliance.

### EXAMPLE 18

### Clinical Trial for combination therapy for Hepatocellular Cancer

This example describes a randomized, blinded, placebo-controlled, multicenter, Phase II or Phase III study designed to provide a preliminary assessment of the safety and efficacy of combining chimeric anti-endoglin antibody with Bevacizumab (AVASTIN®) or sorafenib in patients with hepatocellular cancer (liver cancer). Approximately about 100 - about 800 patients are enrolled, with about 50 - about 400 patients being assigned to a treatment group and about 50 - about 400 patients assigned to a placebo group. The trial will consist of the administration of intravenous repeating doses of chimeric anti-endoglin antibody at about 0.1 - about 30 mg/kg or placebo every one to three weeks combined with Bevacizumab (AVASTIN®) at about 2.5 - about 15 mg/kg every two to three weeks or with sorafenib at about 400mg daily for 3-6 cycles or until progression. The time frame of the study is estimated at about 6 months - about 5 years, with continued therapy for responders as indicated at the end of the initial study. Additional outcome measures are as follows:

Primary Outcome Measures: Progression-free survival. One goal of the study is to demonstrate an increase in progression free survival from about 3-9 months in the Bevacizumab (AVASTIN®) (or sorafenib) plus placebo arm to about 6-12 months (or more) in the Bevacizumab (AVASTIN®) (or sorafenib) plus chimeric anti-endoglin antibody arm.

Secondary outcome measures that can be assessed include duration of response, time to tumor progression, overall survival, serious and non-serious adverse events. For example, a treatment may prevent progression of the disease (i.e., stasis) or may result in an improvement. Alternately, or in addition, other goals can be measured with respect to one or more of the following: decreased tumor burden, decreased neovascularization, reduced side effects, decreased adverse reactions, and/or increased patient compliance.

### EXAMPLE 19

### Clinical Trial for Combination Therapy for Ovarian Cancer

This example describes a randomized, blinded, placebo-controlled, multicenter, Phase II or Phase III study designed to provide a preliminary assessment of the safety and efficacy of combining chimeric anti-endoglin antibody with Bevacizumab (AVASTIN®) in patients with ovarian cancer. Approximately about 100 - about 800 patients are enrolled, with about 50 - about 400 patients being assigned to a treatment group and about 50 - about 400 patients assigned to a placebo group. The trial will consist of the administration of intravenous repeating doses of chimeric anti-endoglin antibody at about 0.1 - about 30 mg/kg or placebo every one to three weeks combined with Bevacizumab (AVASTIN®) at about 15 mg/kg intravenously on day 1, followed by administration of 15 mg/kg intravenously every 21 days for 5 cycles. Chemotherapy may also be used in both treatment arms. The time frame of the study is estimated at 6 months - about 5 years, with continued therapy for responders as indicated at the end of the initial study. Additional outcome measures are as follows:

Primary Outcome Measure: Progression-free survival. One goal of the study is to demonstrate an increase in progression free survival from about 3-6 months in the Bevacizumab (AVASTIN®) plus placebo arm to about 4-12 months (or more) in the Bevacizumab (AVASTIN®) plus chimeric anti-endoglin antibody arm. One goal of the study is to demonstrate an increase overall response rate from about 20% with Bevacizumab (AVASTIN®) plus placebo to about 30% (or more) with Bevacizumab (AVASTIN®) plus chimeric anti-endoglin antibody.

Secondary outcome measures that can be assessed include duration of response, time to tumor progression, overall survival, serious and non-serious adverse events. For example, a treatment may prevent progression of the disease (i.e., stasis) or may result in an improvement. Alternately, or in addition, other goals can be measured with respect to one or more of the following: decreased tumor burden, decreased neovascularization, reduced side effects, decreased adverse reactions, and/or increased patient compliance.

### EXAMPLE 20

### Clinical Trial for combination therapy for Glioblastoma Multiforme

This example describes a randomized, blinded, placebo-controlled, multicenter, Phase II or Phase III study designed to provide a preliminary assessment of the safety and efficacy of combining chimeric anti-endoglin antibody with Bevacizumab (AVASTIN®) in patients with glioblastoma multiforme (brain cancer). Approximately about 100 - about 800 patients are enrolled, with about 50 - about 400 patients being assigned to a treatment group and about 50 - about 400 patients assigned to a placebo group. The trial will consist of the administration of intravenous repeating doses of chimeric anti-endoglin antibody at about 0.1 - about 30 mg/kg or placebo every one to three weeks combined with Bevacizumab (AVASTIN®) at about 2.5 - about 15 mg/kg every two to three weeks for 3-6 cycles or until progression. The time frame of the study is estimated at about 6 months - about 5 years, with continued therapy for responders as indicated at the end of the initial study. Additional outcome measures are as follows:

Primary Outcome Measures: Progression-free survival. One goal of the study is to demonstrate an increase in progression free survival from about 3-9 months in the Bevacizumab (AVASTIN®) plus placebo arm to about 4-12 months (or more) in the Bevacizumab (AVASTIN®) plus chimeric anti-endoglin antibody arm. Secondary outcome measures that can be assessed include duration of response, time to tumor progression, overall survival, serious and non-serious adverse events. For example, a treatment may prevent progression of the disease (i.e., stasis) or may result in an improvement. Alternately, or in addition, other goals can be measured with respect to one or more of the following: decreased tumor burden, decreased neovascularization, reduced side effects, decreased adverse reactions, and/or increased patient compliance

### EXAMPLE 21

### Systemic Toxicology Study in Cynomolgus Monkeys

Cynomolgus monkeys are utilized in a study to address the toxicology of chimeric anti-endoglin antibodies in combination with Bevacizumab (AVASTIN®).

Briefly, monkeys are dosed weekly for three weeks with 10.0 mg/kg, 30.0 mg/kg or 100.0 mg/kg of the chimeric anti-endoglin antibody and 2.5, 5, 7.5, 10 or 15 mg/kg of Bevacizumab (AVASTIN®). Placebo animals are dosed on the same schedule with an appropriate solution in the absence of antibody. The doses are administered as an intravenous bolus over 30 to 60 minutes and at least six animals are dosed at each dose level. Toxicology is assessed via one or more of the following indications: body weight measurements, basic physiologic clinical measurements, serial serum chemistry, hematologic evaluations and histopathological evaluations.

### EXAMPLE 22

### Regional Toxicology Study in Cynomolgus Monkeys

Cynomolgus monkeys are utilized in a study to address the toxicology of chimeric anti-endoglin antibodies in combination with ranibizumab (LUCENTIS®) when given by intravitreal injection.

Briefly, monkeys are dosed by intravitreal injection weekly for six weeks with 0.25, 1.25 and 2.5 mg of chimeric anti-endoglin antibody and 0.5 mg of ranibizumab (LUCENTIS®). Placebo animals are dosed on the same schedule with an appropriate solution in the absence of antibody. The doses are administered as intravitreal injections and at least six animals are dosed at each dose level. Toxicology is assessed via one or more of the following indications: body weight measurements, basic physiologic clinical measurements, serial serum chemistry, hematologic evaluations and histopathological evaluations.

### EXAMPLE 23

### Tubular Network Formation

Angiogenesis can be tested in a two-dimensional in vitro model of tube formation.

In one example, HUVECs are cultured EGM-2 media (Clonetics, Walkersville, MD) containing 5% fetal bovine sera and growth factors in flasks (Falcon, Becton-Dickinson, Franklin Lakes, NJ) in a CO₂ incubator at 37°C under sub-confluent conditions in the presence of anti-VEGF antibody, chimeric anti-endoglin antibody or both anti-VEGF and chimeric anti-endoglin antibodies for eight hours. Irrelevant IgG antibody is included as a separate control. HUVEC cells are then lightly trypsinized and 10,000 to 15,000 cells are inoculated onto polymerized ECMatrix gel (In vitro angiogenesis assay kit, Chemicon). Following overnight incubation, cells are visualized under a microscope and the number of closed polygons is counted, the length of continuous endothelial cells are measured and pictures are taken. All experimental conditions are tested in triplicate.

### EXAMPLE 24

### Sprouting Assays

Angiogenesis can be tested in a three-dimensional in vitro model of sprouting.
HUVECs are isolated from umbilical cords and grown in M199 supplemented with 10% fetal bovine serum (FBS) (GIBCO, Carlsbad, CA) and endothelial cell growth supplement (ECGS) (BD Biosciences, Bedford, MA) at 3° C and 5% CO₂, Passage 2 to 4 HUVEC are used for all experiments (Passage 0 being the primary culture). Lung fibroblasts (LF) are routinely grown in DMEM (GIBCO, Carlsbad, CA) supplemented with 10% FBS at 37° C and 5% CO₂ and used between P10 and P15. Other fibroblast lines, obtainable from ATCC, can also be used.

### Preparing the cells

HUVEC and fibroblasts are expanded in M199/10% FBS/Pen-Strep (1: 100) 1 to 2 days before beading. For HUVEC, medium is switched to EGM-2 (Clonetics, Walkersville, MD) the day before beading. For fibroblasts, medium is switched to EGM-2 the day before embedding. Beading requires approximately 400 HUVEC per bead. Fibroblasts are used at 20,000 cells per well for a 24-well plate. Ninety-six-well plates can also be used with quantities scaled accordingly.

### Cytodex 3 bead preparation

Cytodex 3 microcarrier beads, for example, can be used in the assay (Amersharn Pharmacia Biotech, Piscataway, NJ).

Dry beads (0.5 g) are hydrated and swollen in 50 ml PBS (pH = 7.4) for at least 3 hours at room temperature (RT) in a 50-ml tube and placed it on a rocker.

The beads are allowed to settle (about 15 min). The supernatant is discarded and the beads are washed for a few minutes in fresh PBS (50 ml).

The wash PBS is discarded and replaced with fresh PBS:

The bead suspension is placed in a siliconized glass bottle (from e.g., Windshield Wiper or Sigrnacote). The beads are sterilized by autoclaving for 15 min at 115° C and then stored at 4° C.

### Reagents

### Fibrinogen solution

A fibrinogen solution is made by dissolving 2 mg/ml fibrinogen in DPBS in a 37° C waterbath. The solution is then mixed by inverting the tube rather than vortexing. The percentage of clottable protein can be determined and adjusted accordingly. The solution is then passed through a 0.22-µm filter to sterilize.

### Aprotinin

Lyophilized aprotinin can be reconstituted at 4 U/ml in DI water and sterile filtered. Aliquots of 1 ml each are made and stored at -20° C.

### Thrombin

Thrombin is reconstituted in sterile water at 50 U/ml. Aliquots of 0.5 ml are made and stored at -20° C.

### Coating the beads with HUVEC (Day 1)

HUVEC cells are trypsinzed. Beads are allowed to settle (do not centrifuge), the supernatant is aspirated, and the beads are briefly washed in 1 ml of warm EGM-2 medium. Beads (2500) are mixed with 1 x 10⁶ HUVEC in 1.5 ml of warm EGM-2 medium in a FACS tube and placed vertically in the incubator. (This will be enough for approximately 10 wells; scale up may be done if needed.)

The mixture is incubated for 4 hours at 37° C, inverting and mixing the tube every 20 min. (beads should look like mini golf balls after beading which indicates sufficient coating for sprouting.)

After 4 hours, the coated beads are transferred to a T25 tissue culture flask (Falcon, Bedford, MA) and incubated overnight in 5 ml of EGM-2 medium at 37° C and 5% CO₂.

### Embedding coated beads in fibrin gel (Day 0)

A 2.0 mg/ml fibrinogen solution is prepared as described above and 0.15 Units/ml of aprotinin are added to the fibrinogen solution.

Coated beads are transferred to a 15 mL conical tube and the beads are allowed to settle.

Beads are resuspended in 1 ml of EGM-2 medium and transfered to a 1.5-ml centrifuge tube. The beads are washed three times with 1 ml of EGM-2 medium, mixing by pipetting up and down slowly with a P1000 pipette. The beads are counted on a coverslip and resuspended in a fibrinogen solution at a concentration of 500 beads/ml.

Thrombin (0.625 Units/ml) is added to each well of a 24-well plate. The fibrinogen/bead suspension (0.5 ml) to each well changing the pipette tip for each well.

The thrombin and the fibrinogen/beads are mixed by pipetting up and down gently about four to five times; avoid creating bubbles in the fibrin gel. Control samples either are treated in the absence of antibodies or one or more control antibodies. Test samples are treated with anti-endoglin antibodies alone, anti-VEGF antibodies alone, or a combination thereof. Multiple concentrations of agents can be tested. The fibrinogen/bead solution is allowed to clot for 5 minutes at room temperature and then at 37° C / 5% CO₂ for 15 min. It is important that the plate not be disturbed during the first 5 min of clotting to minimize shearing fibrin, which can result in reduced sprouting.

EGM-2 (1 mL) is added to each well in a drop-wise fashion. Lung fibroblasts are seeded on top of the clot at a concentration of 20,000 cells/well. Replace culture medium with fresh EGM-2 medium every other day until desired growth is achieved.

When the fibrin gel is formed, tiny bubbles may be present in the gel; they will disappear in 3 to 4 days. Sprouting should be apparent between day 2 and 4. Lumen formation begins around day 4 to 5 and sprouts continue to elongate. Newly formed tubes begin to branch around day 4 to 6. By day 6 to 7, the microvessel-like structures begin to anastomose with adjoining tubes; increasing the number of beads per well results in earlier anastomosis.

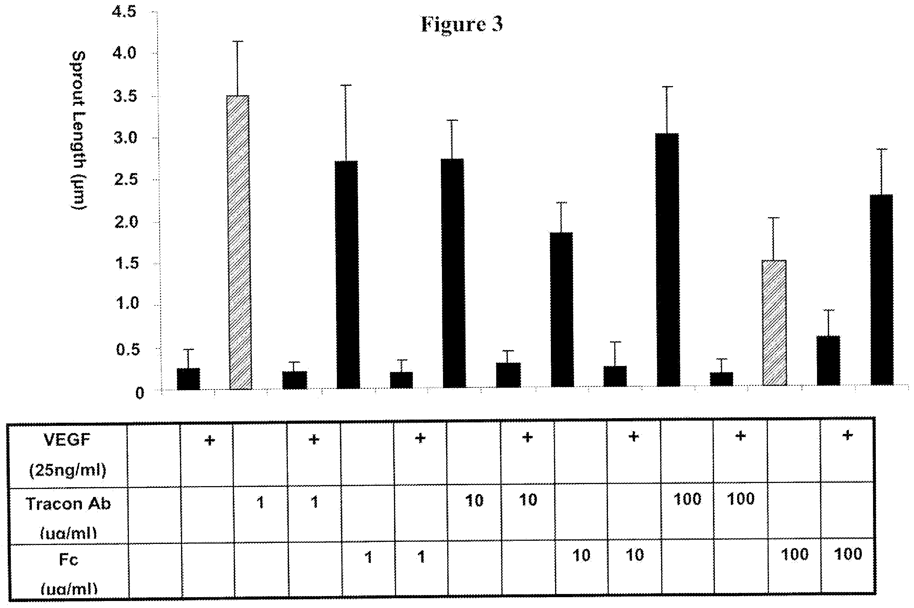

Chimeric TRC105 inhibited VEGF induced sprouting in a dose dependent manner (N=3) using HUVEC spheroid seeded in collagen as illustrated in Figure 3.

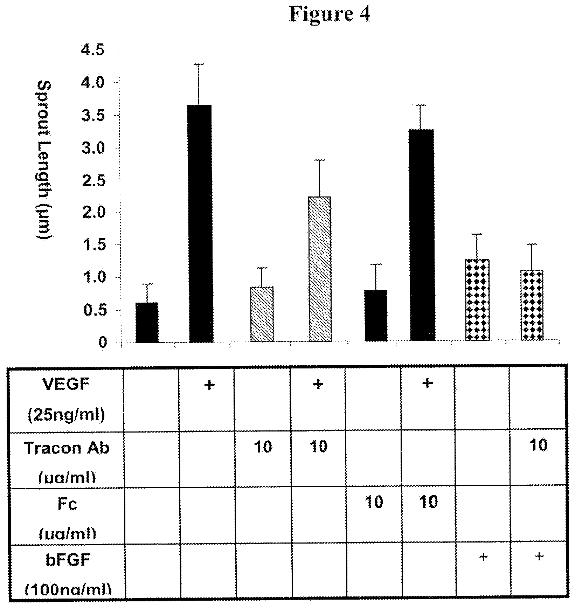

Furthermore, while chimeric TRC105 blocked VEGF induced sprouting (diagonal hatching), it does not inhibit bFGF induced sprouting (diamond hatching) of HUVEC spheroids (N=2) as demonstrated in Figure 4.

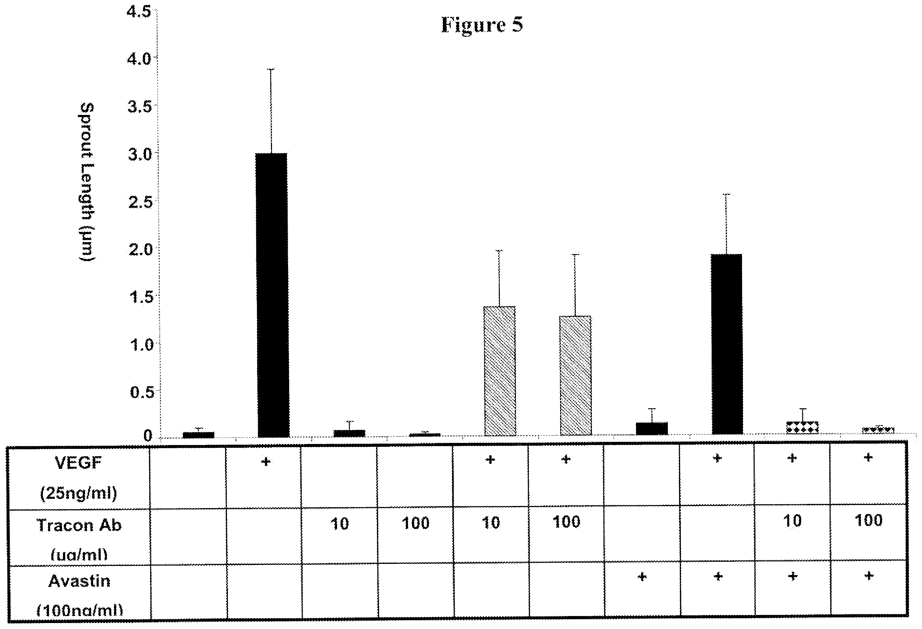

The inhibitory effect of chimeric TRC105 on VEGF induced sprouting (diagonal hatching), was enhanced when combined with the VEGF inhibitor AVASTIN® (diamond hatching) as illustrated in Figure 5.

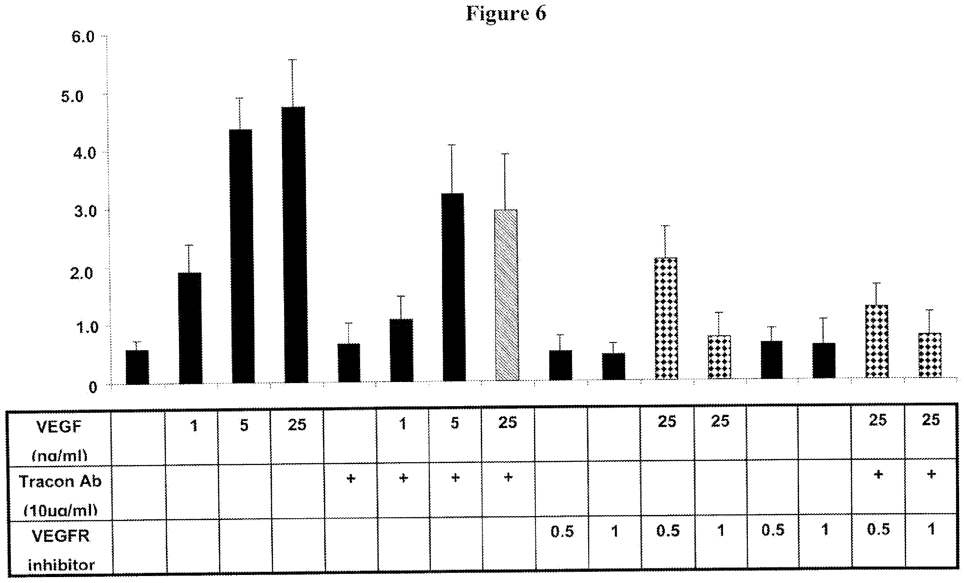

Figure 6 illustrates that the inhibitory effect of chimeric TRC105 on VEGF induced sprouting (diagonal hatching), was not enhanced when combined with the kinase inhibitor PTK787 (diamond hatching).

### EXAMPLE 25

### Immunocytochemistry of Angiogenic Sprouts In Vitro

For endothelial cell (EC) nuclei staining, fibrin gels are washed twice with 1 X PBS and then fixed overnight in 2% paraformaldehyde. After two more washes with 1 X PBS, gels are then stained with 4', 6-diamidino-2-phenylindole (DAPI) (Sigma, St. Louis, MO).

For immunostaining, LF are first removed through a brief treatment of the gels with 10X trypsin. Digestion is stopped with serum as soon as all fibroblasts are removed. Gels are then extensively washed with HBSS, 1X (Cellgro, Herndon, VA). Cultures are then fixed for 10 minutes in 10% formalin and permeabilized with 0.5% Triton X-100 for 5 minutes. Non-specific binding is blocked with a solution of 5% BSA in PBS for 2 hours.

Primary antibodies are used at a 1/100 dilution in blocking buffer and incubated overnight at 4° C. After extensive washing, bound antibody is detected by species-specific Alexa Fluor 488-conjugated or Alexa Fluor 568-conjugated secondary antibodies at a 1/1000 dilution (Molecular Probes, Carlsbad, CA). Isotype-specific non-binding antibodies are used as a control. If high background occurs, the concentration of primary or secondary antibody can be reduced and, if necessary, incubation and/or washing times can be increased. F-actin is stained with TRITC-phalloidin (Sigma, St. Louis, MO) at a concentration of 0.2 µM.

Phase-contrast and fluorescent images are captured on an IX70 Olympus microscope coupled with a digital camera. Fluorescent Z-series image stacks are captured on a two-photon Carl Zeiss MicroImaging LSM 510 Meta microscope and compiled into three-dimensional renderings with Metamorph software (Universal Imaging Corporation, Downingtown PA). Thus, expression of various markers can be readily detected.

Fluorescent optical image stacks along the z-axis of the cultures can be captured to create 3D representations of the vessels. The nuclei are stained by DAPI (green), and vessel walls are stained for vimentin (orange). Wide, hollow lumens are clearly visible, surrounded by a single layer of endothelial cells. These images confirm that the lumens present in the *in vitro* assay are intercellular and not intracellular slits as is often seen in Matrigel assays. Furthermore, it can be confirmed that the HUVECs are polarized, in that they have an apical membrane, facing the lumen, and a basal membrane, apposed to a collagen IV-rich basement membrane and the fibrin gel.

### Example 26

### Suppression of Choroidal Neovascularization

Though animals do not develop age related macular degeneration (AMD) *per se,* choroidal neovascularization resembling that seen in AMD can be produced by using a laser to produce focal disruptions in Bruch's membrane and the overlying retinal pigment epithelium (RPE). This injury stimulates the abnormal growth of underlying choroidal capillaries into the RPE layer and subretinal space. Disruption of Bruch's membrane is common to all forms of choroidal neovascularization (CNV), including that which characterizes the wet form of AMD.

In the laser-induced model of choroidal neovascularization, groups of 9 or 10 mice are treated with subcutaneous (sc) injections of (1) chimeric anti-endoglin antibody alone, (2) anti-VEG antibody alone, (3) chimeric anti-endoglin antibody in combination with anti-VEGF antibody in the same composition or different compositions or (4) control antibody one day prior to laser injury and on days 2, 5, 8, and 11 after laser. At 14 days after laser injury, the mice are injected intravenously with fluorescein-labeled dextran (50 mg), euthanized, and eyes are rapidly dissected for choroidal flat mounts or frozen in optimum cutting temperature embedding compound and sectioned for evaluation of the lesions.

### Example 27

The effect of compositions described herein on laser-induced choroidal neovascularization also is evaluated in adult cynomolgus monkeys.

In this experiment, (1) chimeric anti-endoglin antibody alone, (2) anti-VEGF antibody alone, (3) chimeric anti-endoglin antibody in combination with anti-VEGF antibody in the same composition or different compositions or (4) control antibody is administered by intravenous or intravitreal injection. Each animal receives nine or ten laser burns to each retina, and the development of active choroidal neovascular lesions is assessed by fluorescein angiography, once before the initiation of treatment and 15, 20 and 29 days post-laser treatment. Compositions are administered intravenously once per week, beginning one week before laser injury. Intravitreal injections are made once every two weeks beginning one week before laser, or once, two weeks following laser, at which time active CNV lesions have already formed. Control animals receive weekly intravenous or biweekly intravitreal injections of placebo, beginning one week before laser.

CNV lesions are visualized by fluorescein angiography and graded according to standard procedures.

### Example 28

### Treatment of Age-Related Macular Degeneration

### First Study

Patients manifesting age-related macular degeneration are treated with an intravitreal injection of (1) chimeric anti-endoglin antibody alone, (2) ranibizumab alone, (3) chimeric anti-endoglin antibody in combination with ranibizumab in the same composition or different compositions or (4) control antibody to reduce or prevent the development of neovascularization, macular disease, and retinal damage.

As the first step of treatment, the patients are to receive a full ophthalmic examination to establish a baseline of ocular health. The ophthalmic examination includes indirect ophthalmoscopy, slit-lamp biomicroscopy, peripheral retinal examination, intraocular pressure measurements, visual acuity (unaided and best corrected) symptomatology, fundus photography, fluorescein angiography, optical coherence tomography, electroretinography and A-scan measurements.

Following the preliminary examination, an intravitreal injection as described above is given to a patient's affected eye manifesting AMD. If both eyes are affected, they may be treated separately. The eye to be treated is injected with an ophthalmic solution.

After treatment, the patients' eyes are to be examined on days one (1), two (2), seven (7), fifteen (15), thirty (30) and sixty (60) and every month thereafter for 2 years. Because of the possibility of reoccurrence, the patients should return for periodic examinations on a monthly basis thereafter. On each examination day the patient is monitored for vitreous liquefaction. Additionally, the patients are monitored for posterior vitreous detachments using indirect ophthalmoscopy with scleral depression. Finally, the extent of AMD presented by the patients is continuously monitored through periodic retinal examinations, optical coherence tomography and fluorescein angiograms to monitor for the presence of subretinal fluid, blood, exudates, RPE detachment, cystic retinal changes, or the presence of grayish green subretinal neovascular membrane. Additional treatments may be required if indicia of reoccurring neovascularization are observed. Additional treatments may be given on weekly or monthly basis. In a preferred embodiment, an initial treatment is followed by subsequent treatments between 1-6 months apart.

### Second Study

Purpose: To demonstrate the efficacy of intravitreal chimeric anti-endoglin antibodies and ranibizumab for treatment of neovascular age-related macular degeneration (AMD).

Methods: Fifty to 500 patients (50 to 500 eyes) with subfoveal choroidal neovascularization (CNV) resulting from AMD will participated in the study at an approved site.

The criteria for reinjection are presence of fluid in the macula, increased central retinal thickness (CRT) of at least 100 micron, loss of at least 5 letters of vision associated with increased fluid in the macula, new classic CNV, or new macular hemorrhage. The main outcome measure is the proportion of eyes losing fewer than 15 letters of vision after 12 months. Best-corrected visual acuity measurement and clinical ocular examination are performed at 1 week, 1 month and then monthly for 5-12 months.

Mean visual acuity and mean CRT are measured compared to baseline. Ocular and/or systemic side effects are noted.

### Example 29

### Inhibition of Injury-Induced Corneal Neovascularization

Corneal neovascularization is induced in male C57BL/6 mice by intrastromal placement of 3 nylon sutures, or by chemical injury (NaOH) and mechanical debridement of the corneal epithelium. Multiple experiments are conducted in which (1) chimeric anti-endoglin antibody alone, (2) anti-VEGF antibody alone, (3) chimeric anti-endoglin antibody in combination with anti-VEGF antibody alone in the same composition or different compositions or (4) control antibody is administered intraperitoneally once or at multiple time points immediately before or following injury.

The growth of corneal neovessels is evaluated by slit-lamp microscopy and histological evaluation. The vasculature is labeled with an endothelial cell specific fluorescein-conjugated lectin, and neovascularization is evaluated in corneal flat-mounts, as well as in cross sections using PECAM immunohistochemistry. The presence of corneal edema is evaluated, using slit lamp microscopy, and corneal thickness is measured in cross-sections; increases in corneal thickness reflect the amount of edema. The numbers of polymorphonuclear leukocytes (PMN) and macrophages are determined by staining cross-sections with HEMA-3 or rat anti-mouse F4/80 monoclonal antibody, respectively.

### SEQUENCE LISTING

SEQ ID NO. 1 (TRC105 V_{L}: CDRs are underlined) 
QIVLSQSPAILSASPGEKVTMTCRASSSVSYMHWYQQKPGSSPKPWIYATSNLASGVPVRFSGSGSGTSYSLTIS
RVEAEDAATYYCQQWSSNPLTFGAGTKLELK
SEQ ID NO: 2 (TRC105 C_{L}) 
TVAAPSVFIFPPSDEQLKSGTASVVCLLNNFYPREAKVQWKVDNALQSGNSQESVTEQDSKDSTYSLSSTLTLSK
ADYEKHKVYACEVTHQGLSSPVTKSFNRGEC
SEQ ID NO: 3 (TRC105 V_{H}: CDRs are underlined) 
EVKLEESGGGLVQPGGSMKLSCAASGFTFSDAWMDWVRQSPEKGLEWVAEIRSKASNHATYYAESVKGRFTISRD
DSKSSVYLQMNSLRAEDTGIYYCTRWRRFFDSWGQGTTLTVSS
SEQ ID NO: 4 (TRC105 Cγ1)
SEQ ID NO: 5 (Variable heavy chain of A.4.6.1: murine monoclonal Ab that specifically binds to VEGF) 
EIQLVQSGPELKQPGETVRISCKASGYTFTNYGMNWVKQAPGKGLKWMGWINTYTGEPTYAADFKRRFTFSLETS
ASTAYLQISNLKNDDTATYFCAKYPHYYGSSHWYFDVWGAGTTVTVSS
SEQ ID NO: 6 (Variable heavy chain of humanized F(ab): F(ab)-12)
SEQ ID NO: 7 (Variable heavy chain of humIII: human consensus framework for heavy subgroup III) 
EVQLVESGGGLVQPGGSLRLSCAASGFTFSSYAMSWVRQAPGKGLEWVSVISGDGGSTYYADSVKGRFTISRDNS
KNTLYLQMNSLRAEDTAVYYCARGFDYWGQGTLVTVSS
SEQ ID NO: 8 (Variable light chain of A.4.6.1: murine monoclonal Ab that specifically binds to VEGF) 
DIQMTQTTSSLSASLGDRVIISCSASQDISNYLNWYQQKPDGTVKVLIYFTSSLHSGVPSRFSGSGSGTDYSLTI
SNLEPEDIATYYCQQYSTVPWTFGGGTKLEIKR
SEQ ID NO: 9 (Variable light chain of humanized F(ab): F(ab)-12) 
DIQMTQSPSSLSASVGDRVTITCSASQDISNYLNWYQQKPGKAPKVLIYFTSSLHSGVPSRFSGSGSGTDFTLTI
SSLQPEDFATYYCQQYSTVPWTFGQGTKVEIKR
SEQ ID NO: 10 (Variable light chain of humκI: human consensus framework for kappa subgroup I) 
DIQMTQSPSSLSASVGDRVTITCRASQSISNYLAWYQQKPGKAPKLLIYAASSLESGVPSRFSGSGSGTDFTLTI
SSLQPEDFATYYCQQYNSLPWTFGQGTKVEIKR
SEQ ID NO: 11 (Human Cγ1)

## Claims

1. A method of inhibiting VEGF induced sprouting by contacting cells in vitro with a composition comprising a chimeric anti-endoglin antibody and a composition comprising an antagonistic anti-VEGF antibody; said chimeric anti-endoglin antibody comprising a light chain variable region (V_{L}) having an amino acid sequence set forth as SEQ ID NO: 1; a light chain constant region (C_{L}) having an amino acid sequence set forth as SEQ ID NO: 2; a heavy chain variable region (V_{H}) having an amino acid sequence set forth as SEQ ID NO: 3; and a constant region (Fc) having an amino acid sequence set forth as SEQ ID NO: 4.

2. A composition comprising a chimeric anti-endoglin antibody and a composition comprising an antagonistic anti-VEGF antibody for use in treating an angiogenesis-related disease in a subject; said chimeric anti-endoglin antibody comprising a light chain variable region (V_{L}) having an amino acid sequence set forth as SEQ ID NO: 1; a light chain constant region (C_{L}) having an amino acid sequence set forth as SEQ ID NO: 2; a heavy chain variable region (V_{H}) having an amino acid sequence set forth as SEQ ID NO: 3; and a constant region (Fc) having an amino acid sequence set forth as SEQ ID NO: 4; wherein the treatment in said angiogenesis-related disease is **characterized by** the inhibition of VEGF-induced angiogenesis.

3. The method of claim 1 or the composition for use according to claim 2, wherein the composition further comprises an acceptable carrier or excipient.

4. The composition for use according to claim 2, wherein the angiogenesis-related disease is a cancer, or a metastasis thereof.

5. The composition for use according to claim 2, wherein the composition inhibits cancer growth.

6. The composition for use according to claim 4 or claim 5, wherein the cancer is a solid tumor, preferably, wherein the cancer is an epithelial based tumor.

7. The composition for use according to claim 6, wherein the cancer is selected from a lung cancer, a gynecologic malignancy, a melanoma, a breast cancer, a pancreatic cancer, an ovarian cancer, a uterine cancer, a colorectal cancer, a prostate cancer, a head cancer, a liver cancer (hepatocellular cancer), a neck cancer, a kidney cancer (renal cell cancer), a sarcoma, a myeloma, and a lymphoma.

8. The composition for use according to claim 2, wherein the angiogenesis-related disease is an ocular disease **characterized by** angiogenesis/neovascularization.

9. The composition for use according to claim 8, wherein the ocular disease is macular degeneration or diabetic retinopathy.

10. The composition for use according to claim 9, wherein the macular degeneration is age-related macular degeneration (AMD).

11. The method of claim 1 or the composition for use according to claim 2, wherein the chimeric anti-endoglin antibody is present in the composition in an amount of 0.01 mg/kg, 0.05 mg/kg, 0.1 mg/kg, 0.5 mg/kg, 1 mg/kg, 5 mg/kg, 10 mg/kg, 20 mg/kg or 30 mg/kg.

12. The method of claim 1 or the composition for use according to claim 2, wherein the anti-VEGF antibody is present in the composition in an amount of 2.5 mg/kg, 5 mg/kg, 7.5 mg/kg, 10 mg/kg or 15 mg/kg.

13. The method of claim 1 or the composition for use according to claim 2, wherein the chimeric anti-endoglin antibody and the anti-VEGF antibody are administered sequentially or concurrently.

14. The method of claim 1 or the composition for use according to claim 2, wherein the chimeric anti-endoglin antibody and the anti-VEGF antibody are administered at the same site, or are administered at different sites.

15. The method of claim 1 or the composition for use according to claim 2, wherein the anti-VEGF antibody is bevacizumab.

16. the method of claim 1 or the composition for use according to claim 2, wherein the anti-VEGF antibody is ranibizumab.

## Patentansprüche

1. Verfahren zum Hemmen von VEGF-induziertem Aussprossen durch Inberührungbringen von Zellen *in vitro* mit einer Zusammensetzung, umfassend einen chimären Anti-Endoglin-Antikörper und eine Zusammensetzung, die einen antagonistischen Anti-VEGF-Antikörper umfasst; wobei der chimäre Anti-Endoglin-Antikörper eine leichtkettige variable Region (V_{L}) mit einer als SEQ ID NO: 1 aufgeführten Aminosäuresequenz umfasst; wobei eine leichtkettige konstante Region (C_{L}) eine als SEQ ID NO: 2 aufgeführte Aminosäuresequenz aufweist; wobei eine schwerkettige variable Region (V_{H}) eine als SEQ ID NO: 3 aufgeführte Aminosäuresequenz aufweist; und wobei eine konstante Region (Fc) eine als SEQ ID NO: 4 aufgeführte Aminosäuresequenz aufweist.

2. Zusammensetzung, umfassend einen chimären Anti-Endoglin-Antikörper und eine Zusammensetzung, die einen antagonistischen Anti-VEGF-Antikörper zur Verwendung beim Behandeln einer angiogenesebedingten Erkrankung in einem Subjekt umfasst; wobei der chimäre Anti-Endoglin-Antikörper eine leichtkettige variable Region (V_{L}) mit einer als SEQ ID NO: 1 aufgeführten Aminosäuresequenz umfasst; wobei eine leichtkettige konstante Region (C_{L}) eine als SEQ ID NO: 2 aufgeführte Aminosäuresequenz aufweist; wobei eine schwerkettige variable Region (V_{H}) eine als SEQ ID NO: 3 aufgeführte Aminosäuresequenz aufweist; und wobei eine konstante Region (Fc) eine als SEQ ID NO: 4 aufgeführte Aminosäuresequenz aufweist; wobei das Behandeln in der angiogenesebedingten Erkrankung durch das Hemmen von VEGF-induzierter Angiogenese gekennzeichnet ist.

3. Verfahren nach Anspruch 1 oder Zusammensetzung zur Verwendung nach Anspruch 2, wobei die Zusammensetzung ferner einen unbedenklichen Träger oder Hilfsstoff umfasst.

4. Zusammensetzung zur Verwendung nach Anspruch 2, wobei die angiogenesebedingte Erkrankung ein Krebs oder eine Metastase davon ist.

5. Zusammensetzung zur Verwendung nach Anspruch 2, wobei die Zusammensetzung Krebswachstum hemmt.

6. Zusammensetzung zur Verwendung nach Anspruch 4 oder 5, wobei der Krebs ein solider Tumor ist, wobei der Krebs vorzugsweise ein epithelbasierter Tumor ist.

7. Zusammensetzung zur Verwendung nach Anspruch 6, wobei der Krebs ausgewählt ist aus einem Lungenkrebs, einer gynäkologischen Malignität, einem Melanom, einem Brustkrebs, einem Pankreaskrebs, einem Eierstockkrebs, einem Gebärtmutterkrebs, einem Kolorektalkrebs, einem Prostatakrebs, einem Kopfkrebs, einem Leberkrebs (hepatozellulärer Krebs), einem Halskrebs, einem Nierenkrebs (Nierenzellkrebs), einem Sarkom, einem Myelom und einem Lymphom.

8. Zusammensetzung zur Verwendung nach Anspruch 2, wobei die angiogenesebedingte Erkrankung eine Augenerkrankung ist, **gekennzeichnet durch** Angiogenese/Neovaskularisierung.

9. Zusammensetzung zur Verwendung nach Anspruch 8, wobei die Augenerkrankung Makuladegeneration oder diabetische Retinopathie ist.

10. Zusammensetzung zur Verwendung nach Anspruch 9, wobei die Makuladegeneration altersbedingte Makuladegeneration (AMD) ist.

11. Verfahren nach Anspruch 1 oder Zusammensetzung zur Verwendung nach Anspruch 2, wobei der chimäre Anti-Endoglin-Antikörper in einer Menge von 0,01 mg/kg, 0,05 mg/kg, 0,1 mg/kg, 0,5 mg/kg, 1 mg/kg, 5 mg/kg, 10 mg/kg, 20 mg/kg oder 30 mg/kg in der Zusammensetzung vorliegt.

12. Verfahren nach Anspruch 1 oder Zusammensetzung zur Verwendung nach Anspruch 2, wobei der Anti-VEGF-Antikörper in einer Menge von 2,5 mg/kg, 5 mg/kg, 7,5 mg/kg, 10 mg/kg oder 15 mg/kg in der Zusammensetzung vorliegt.

13. Verfahren nach Anspruch 1 oder Zusammensetzung zur Verwendung nach Anspruch 2, wobei der chimäre Anti-Endoglin-Antikörper und der Anti-VEGF-Antikörper sequenziell oder gleichzeitig verabreicht werden.

14. Verfahren nach Anspruch 1 oder Zusammensetzung zur Verwendung nach Anspruch 2, wobei der chimäre Anti-Endoglin-Antikörper und der Anti-VEGF-Antikörper an der gleichen Stelle verabreicht werden oder an verschiedenen Stellen verabreicht werden.

15. Verfahren nach Anspruch 1 oder Zusammensetzung zur Verwendung nach Anspruch 2, wobei der Anti-VEGF-Antikörper Bevacizumab ist.

16. Verfahren nach Anspruch 1 oder Zusammensetzung zur Verwendung nach Anspruch 2, wobei der Anti-VEGF-Antikörper Ranibizumab ist.

## Revendications

1. Procédé d'inhibition d'un bourgeonnement induit par le facteur de croissance de l'endothélium vasculaire (VEGF) en mettant en contact des cellules *in vitro* avec une composition comprenant un anticorps anti-endogline chimérique et une composition comprenant un anticorps anti-VEGF antagoniste; ledit anticorps anti-endogline chimérique comprenant une région variable de chaîne légère (V_{L}) ayant une séquence d'acides aminés présentée comme SEQ. ID. n° 1 ; une région constante de chaîne légère (C_{L}) ayant une séquence d'acides aminés présentée comme SEQ. ID. n° 2 ; une région variable de chaîne lourde (V_{H}) ayant une séquence d'acides aminés présentée comme SEQ. ID. n° 3 ; et une région constante (F_{C}) ayant une séquence d'acides aminés présentée comme SEQ. ID. n° 4.

2. Composition comprenant un anticorps anti-endogline chimérique et une composition comprenant un anticorps anti-VEGF antagoniste pour une utilisation dans le traitement d'une maladie liée à l'angiogenèse chez un sujet ; ledit anticorps anti-endogline chimérique comprenant une région variable de chaîne légère (V_{L}) ayant une séquence d'acides aminés présentée comme SEQ. ID. n° 1 ; une région constante de chaîne légère (C_{L}) ayant une séquence d'acides aminés présentée comme SEQ. ID. n° 2; une région variable de chaîne lourde (V_{H}) ayant une séquence d'acides aminés présentée comme SEQ. ID. n° 3 ; et une région constante (F_{C}) ayant une séquence d'acides aminés présentée comme SEQ. ID. n° 4 ; dans laquelle le traitement de ladite maladie liée à l'angiogenèse est **caractérisé par** l'inhibition de l'angiogenèse induite par VEGF.

3. Procédé selon la revendication 1 ou composition pour une utilisation selon la revendication 2, dans lesquels la composition comprend en outre un vecteur ou excipient acceptable.

4. Composition pour une utilisation selon la revendication 2, dans laquelle la maladie liée à l'angiogenèse est un cancer ou une métastase de celui-ci.

5. Composition pour une utilisation selon la revendication 2, dans laquelle la composition inhibe la croissance de cancer.

6. Composition pour une utilisation selon la revendication 4 ou la revendication 5, dans laquelle le cancer est une tumeur solide, de préférence, dans laquelle le cancer est une tumeur à base épithéliale.

7. Composition pour une utilisation selon la revendication 6, dans laquelle le cancer est choisi parmi un cancer du poumon, une malignité gynécologique, un mélanome, un cancer du sein, un cancer du pancréas, un cancer de l'ovaire, un cancer de l'utérus, un cancer colorectal, un cancer de la prostate, un cancer de la tête, un cancer du foie (cancer hépatocellulaire), un cancer du cou, un cancer du rein (un cancer des cellules rénales), un sarcome, un myélome et un lymphome.

8. Composition pour une utilisation selon la revendication 2, dans laquelle la maladie liée à l'angiogenèse est une maladie oculaire **caractérisée par** une angiogenèse/néovascularisation.

9. Composition pour une utilisation selon la revendication 8, dans laquelle la maladie oculaire est une dégénérescence maculaire ou une rétinopathie diabétique.

10. Composition pour une utilisation selon la revendication 9, dans laquelle la dégénérescence maculaire est une dégénérescence maculaire liée à l'âge (DMLA).

11. Procédé selon la revendication 1 ou composition pour une utilisation selon la revendication 2, dans lesquels l'anticorps anti-endogline chimérique est présent dans la composition dans une quantité de 0,01 mg/kg, 0,05 mg/kg, 0,1 mg/kg, 0,5 mg/kg, 1 mg/kg, 5 mg/kg, 10 mg/kg, 20 mg/kg ou 30 mg/kg.

12. Procédé selon la revendication 1 ou composition pour une utilisation selon la revendication 2, dans lesquels l'anticorps anti-VEGF est présent dans la composition dans une quantité de 2,5 mg/kg, 5 mg/kg, 7,5 mg/kg, 10 mg/kg ou 15 mg/kg.

13. Procédé selon la revendication 1 ou composition pour une utilisation selon la revendication 2, dans lesquels l'anticorps anti-endogline chimérique et l'anticorps anti-VEGF sont administrés séquentiellement ou simultanément.

14. Procédé selon la revendication 1 ou composition pour une utilisation selon la revendication 2, dans lesquels l'anticorps anti-endogline chimérique et l'anticorps anti-VEGF sont administrés au même site, ou sont administrés à des sites différents.

15. Procédé selon la revendication 1 ou composition pour une utilisation selon la revendication 2, dans lesquels l'anticorps anti-VEGF est le bévacizumab.

16. Procédé selon la revendication 1 ou composition pour une utilisation selon la revendication 2, dans lesquels l'anticorps anti-VEGF est le ranibizumab.
